# FASCICULE DE BREVET EUROPEEN

(11) **EP 3 390 388 B1**
(45) Date de publication et mention de la délivrance du brevet: **03.02.2021**
(21) Numéro de dépôt: 16831494.6
(22) Date de dépôt: 16.12.2016
(51) Int. Cl.: C07D 401/14, C07F 5/00, G01N 23/223, G01N 33/58, C07D 413/14, B01D 9/00

(54) **COMPLEXES DE LANTHANIDE POUR LA CRISTALLISATION DE MACROMOLECULES BIOLOGIQUES ET LA DETERMINATION DE LEUR STRUCTURE CRISTALLOGRAPHIQUE**
LANTHANIDKOMPLEXE ZUM KRISTALLISIEREN BIOLOGISCHER MAKROMOLEKÜLE UND BESTIMMUNG DER KRISTALLOGRAFISCHEN STRUKTUR DAVON
LANTHANIDE COMPLEXES FOR CRYSTALLISING BIOLOGICAL MACROMOLECULES AND DETERMINING THE CRYSTALLOGRAPHIC STRUCTURE THEREOF

(30) Priorité: 18.12.2015 FR 1562880
(43) Date de publication de la demande: 24.10.2018
(73) Titulaire: Ecole Normale Supérieure de Lyon, 69007 Lyon (FR); Centre National de la Recherche Scientifique, 75794 Paris Cedex 16 (FR); Université Claude Bernard Lyon I, 69100 Villeurbanne (FR); Commissariat à l'Énergie Atomique et aux Énergies Alternatives, 75015 Paris (FR)
(72) Inventeur: MAURY, Olivier, 69126 Brindas (FR); GIRARD, Eric, 26100 Romans sur Isère (FR); ENGILBERGE, Sylvain, 74930 Reignier (FR); RIOBE, François, 69007 Lyon (FR)
(74) Mandataire: Cabinet Beau de Loménie
(86) Numéro de dépôt international: PCT/FR2016/053539
(87) Numéro de publication internationale: WO 2017/103545

(56) Documents cités:
- AURORA RODRÍGUEZ-RODRÍGUEZ ET AL: "Lanthanide(III) Complexes with Ligands Derived from a Cyclen Framework Containing Pyridinecarboxylate Pendants. The Effect of Steric Hindrance on the Hydration Number", INORGANIC CHEMISTRY, vol. 51, no. 4, 20 février 2012 (2012-02-20), pages 2509-2521, XP055083216, ISSN: 0020-1669, DOI: 10.1021/ic202436j cité dans la demande
- GUILLAUME POMPIDOR ET AL: "Protein Crystallography through Supramolecular Interactions between a Lanthanide Complex and Arginine", ANGEWANDTE CHEMIE INTERNATIONAL EDITION, vol. 47, no. 18, 21 avril 2008 (2008-04-21), pages 3388-3391, XP055042394, ISSN: 1433-7851, DOI: 10.1002/anie.200704683
- ROMAIN TALON ET AL: "Clicked europium dipicolinate complexes for protein X-ray structure determination", CHEMICAL COMMUNICATIONS - CHEMCOM, vol. 48, no. 97, 1 janvier 2012 (2012-01-01), pages 11886-11888, XP055265553, GB ISSN: 1359-7345, DOI: 10.1039/c2cc36982f
- Eric Girard ET AL: "CCP4 study weekend 1914 Girard et al. High-phasing-power lanthanide complexes Biological Crystallography A new class of lanthanide complexes to obtain high-phasing-power heavy-atom derivatives for macromolecular crystallography", Acta Crystallographica Section D, 1 janvier 2003 (2003-01-01), XP055265430, Extrait de l'Internet: URL:http://journals.iucr.org/d/issues/2003 /11/00/ba5052/ba5052.pdf [extrait le 2013-04-15]
- CORNEILLIE T M ET AL: "Crystal structures of two complexes of the rare-earth-DOTA-binding antibody 2D12.5: ligand generality from a chiral system", JOURNAL OF THE AMERICAN CHEMICAL SOCIETY, AMERICAN CHEMICAL SOCIETY, US, vol. 125, no. 49, 1 janvier 2003 (2003-01-01), pages 15039-15048, XP002984343, ISSN: 0002-7863, DOI: 10.1021/JA037236Y
- INMACULADA PÉREZ-DORADO ET AL: "Crystallization of the pneumococcal autolysin LytC: in-house phasing using novel lanthanide complexes", ACTA CRYSTALLOGRAPHICA SECTION F STRUCTURAL BIOLOGY AND CRYSTALLIZATION COMMUNICATIONS, vol. 64, no. 4, 1 avril 2010 (2010-04-01), pages 112-451, XP055265433, DOI: 10.1107/S1744309110006081

## Description

La présente invention concerne les domaines techniques de la cristallisation et de la cristallographie. Plus précisément, l'invention concerne de nouveaux complexes de lanthanide qui peuvent être utilisés en tant qu'agents phasants pour la détermination de structure cristalline de macromolécules biologiques, mais également en tant qu'aide pour leur cristallisation. L'invention a également pour objet leur utilisation dans ces domaines et les procédés de cristallisation et de détermination de données structurales qui les mettent en œuvre pour une macromolécule biologique choisie parmi les peptides et les protéines.

Le succès retentissant de la détermination complète du génome humain en 2000 a ouvert la voie à un domaine de recherche plus vaste encore : la génomique structurale qui consiste à déterminer la structure des protéines pour comprendre les relations entre leur structure et leurs fonctions. De par le nombre et la variété de protéines existantes, il s'agit d'un chantier énorme dont la portée en termes de retombées scientifiques et médicales est inestimée. Aujourd'hui, les deux outils pour cette résolution structurale sont la cristallographie et la résonance magnétique nucléaire (RMN). Il s'agit de deux techniques complémentaires qui présentent des avantages et des limites : (i) la RMN permet l'analyse en solution de protéines, mais requiert un enrichissement isotopique; (ii) la cristallographie permet une détermination plus rapide de structures mais reste tributaire de la préparation de cristaux de bonne qualité. Actuellement, l'effort de recherche est focalisé sur le développement de très grands instruments au détriment de la recherche de nouvelles méthodologies.

Dans ce domaine, plusieurs difficultés successives doivent être surmontées. La première difficulté relève du domaine de la biologie et concerne la préparation et la purification de protéines d'intérêt et seulement 18% des protéines clonées franchissent cette étape. La seconde difficulté concerne l'étape de cristallisation puisque là encore, seulement 20% des protéines purifiées peuvent être cristallisées et seulement la moitié de ces cristaux permettront la détermination de la structure par diffraction des rayons X. Au bilan, seulement 1,8% des protéines clonées sont structuralement caractérisées et ceci, au prix d'investissements en termes de temps et de moyens humains et financiers considérables.

Un cristal d'une protéine, ou plus généralement d'une macromolécule biologique, est constitué d'un empilement régulier et périodique des molécules qui le composent. L'empilement est maintenu par des contacts au sein du cristal (liaisons hydrogène, ponts salin, contacts hydrophobes). L'obtention d'un cristal d'une macromolécule biologique est une étape clef dans la détermination de sa structure par cristallographie aux rayons X.

Il convient de souligner que les processus de cristallisation des macromolécules biologiques sont encore loin d'être compris et que les approches actuelles sont basées sur une approche empirique, de type essais/erreurs.

Une des notions importantes à considérer dans la cristallogenèse est la notion de solubilité. En solution, une macromolécule est entourée d'une couche de solvatation qui présente un effet répulsif et isolant et permet d'éviter l'auto-agrégation et la précipitation. La cristallisation va contre cet effet, puisqu'il s'agit de s'éloigner des conditions de solubilité de la macromolécule biologique par l'ajout d'un agent précipitant, également nommé agent cristallisant. Dans le cadre de la présente description, « agent précipitant » et « agent cristallisant » sont utilisés indifféremment. On entend par agent cristallisant/précipitant, une molécule ou un mélange de molécules aidant à la formation d'un cristal de macromolécules biologiques, dans certaines conditions.

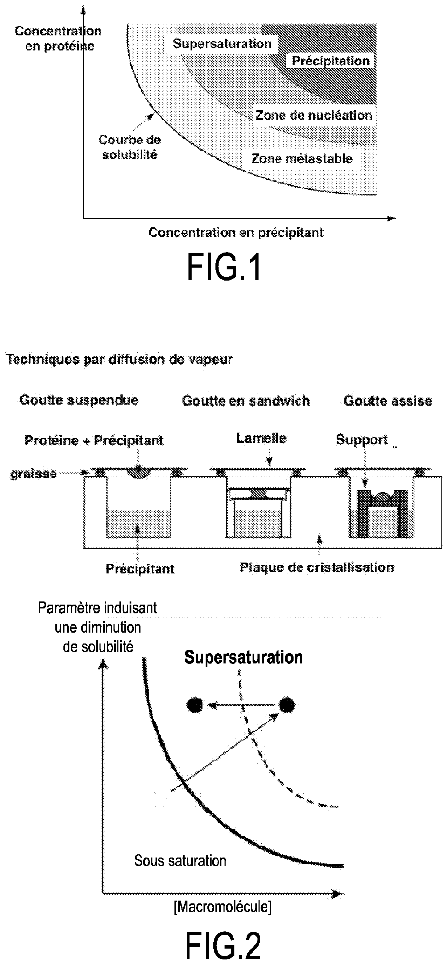

Le principe général de la cristallisation repose sur le diagramme présenté en **Figure 1** qui correspond au schéma réalisé par Mirjam Leunissen et figurant sur http://people.ds.cam.ac.uk/mi527/publications/assets/leunissen-literature-research.pdf.

Les deux étapes principales qui surviennent dans le processus de cristallisation sont : la nucléation et la croissance cristalline.

L'agent cristallisant induit une modification de la solubilité et donc de la position sur le diagramme en fonction du temps. Il existe différentes méthodes de cristallisation mettant en œuvre un agent cristallisant.

Le but lors d'une cristallisation est de jouer sur les concentrations en macromolécule biologique et en agent cristallisant pour parvenir à atteindre la zone de nucléation représentée sur le diagramme de la **Figure 1****.** Lorsque cette zone est atteinte, il peut se former des nucleii (points de nucléation). Ces nucleii sont des agrégats plus ou moins organisés d'une centaine de macromolécules. La formation de ces départs « cristallins » fait diminuer la concentration en macromolécule soluble. C'est le commencement de la croissance cristalline. D'autres macromolécules viennent s'agencer sur ce nucleii et forment des micro-cristaux qui vont grandir avec le temps.

Il existe trois grandes classes majeures d'agents cristallisants :
- les sels,
- les solvants organiques (éthanol, dioxane, MPD pour méthylpentanediol etc...)
- les polymères (PEG (Poly-Ethylène Glycol) de poids moléculaires allant jusqu'à 20000 g/mol, Jeffamine T etc...)

Ces agents ont pour effet :
- d'entrer en compétition avec la macromolécule biologique pour l'eau, ou plus généralement le solvant dans lequel la macromolécule est initialement en solution, et ainsi diminuer la solubilité de la macromolécule, induisant sa cristallisation,
- d'écranter des charges à la surface de la macromolécule permettant le rapprochement de plusieurs macromolécules,
- de modifier la constante diélectrique du solvant et par conséquent les forces entre macromolécules
- de favoriser des phénomènes de type volume exclu (exclusion hydrophobe).

Pour obtenir la cristallisation d'une protéine, ou plus généralement d'une macromolécule biologique, une approche automatisée permettant un criblage de différentes conditions de cristallisations commercialement disponibles (kits de la société Hampton Research, par exemple). Les expériences de cristallisation sont préparées par des robots et la phase de cristallisation est suivie régulièrement par des robots microscopes qui scrutent l'apparition des cristaux. Cette approche à haut débit a fait passer au second plan la recherche de méthodologies alternatives permettant de favoriser la cristallisation, consistant à trouver des agents qui favorisent la cristallisation sans dénaturer la protéine. Quelques travaux existent sur ce sujet dans la littérature, comme l'utilisation de composés naturels : solides minéraux (Falini G., Fermani S., Conforti G., Ripamonti A. (2002) ; Acta Crystallogr D Biol Crystallogr. 58, 1649-1652, McPherson, A. & Shlichta, P. (1988), Science, 239, 385-387), substrat d'origine biologique comme les cheveux, les crins de chevaux, les moustaches de rat ou les algues séchées (D'Arcy A., Mac Sweeney A. & Haber, A. (2003), Acta Crystallogr D Biol Crystallogr. 59, 1343-1346), mais également des produits de synthèse (Bijelic A., Molitor C., Mauracher S., G. Al-Oweini, R., Kortz U. & Rompel, A. (2014), ChemBioChem. 16, 233-241, Pechkova, E. & Nicolini, C. (2002), J. Cell. Biochem. 85, 243-251, Sugahara, M. Asada, Y. Morikawa, Y. Kageyama, Y. & Kunishima, N. (2008), Acta Crystallogr D Biol Crystallogr. 64, 686-695, Matar-Merheb, R. Rhimi, M. Leydier, A. Huché, F. Galián, C. Desuzinges-Mandon, E. Ficheux, D. Flot, D. Aghajari, N. Kahn, R. et al. (2011), PLoS ONE. 6, e18036).

Dans tous ces cas, la croissance cristalline se produit au contact de ces « impuretés ». Les travaux les plus aboutis dans ce domaine sont ceux de l'équipe de Naomi E. Chayen qui propose l'utilisation de matériaux mésoporeux, capable d'induire la nucléation dans les pores après une étape de concentration/agrégation au sein de ces mêmes pores (WO 02/088435). Le même groupe décrit également l'utilisation de MIP « molecular imprinted polymers » basés sur le même principe. Il s'agit de polymères préalablement imprimé par la protéine à cristalliser et qui en contiennent donc la trace. Ajoutés ensuite à la goutte de cristallisation, une interaction de forme peut se produire favorisant la nucléation (Saridakis, PNAS, 2001, 108,11081). Tous ces agents d'aide à la cristallisation sont des solides formant une phase hétérogène dans le milieu de cristallisation, chacun d'eux devant donc être ajouté manuellement dans la goutte de cristallisation, ce qui est inconciliable avec l'utilisation des plateformes de criblage robotisées, à l'exception des MIP.

Par ailleurs, de par la taille gigantesque des macromolécules biologiques, comme les protéines, la détermination de leur structure par diffraction des rayons X implique de résoudre le problème des phases en cristallographie. Cela implique de posséder un cristal natif (protéine pure) et/ou un cristal dérivé contenant un atome lourd facilement repérable et permettant de lever le problème des phases, on parle d'agent de phasage. La méthode la plus classique, basée sur la diffraction anomale à plusieurs longueurs d'onde (MAD) est fondée sur le remplacement du soufre des acides aminées méthionines de la protéine par un atome de sélénium. Utilisée depuis les années 90, cette méthode a révolutionné la cristallographie macromoléculaire, mais elle implique la synthèse, la purification, la cristallisation et la résolution de la structure de protéines séléniées analogues. C'est une méthode très coûteuse en temps. D'autres agents phasants exogènes peuvent être utilisés et sont d'ailleurs commercialisés. On peut citer le brome, les métaux lourds (Pt, Au, Hg). Dans ce contexte, R. Kahn a montré au début des années 2000 que les lanthanides, qui présentent des effets anomaux parmi les plus intenses, peuvent être inclus sous forme de complexes dans des cristaux de protéines, ce qui permet de résoudre rapidement la structure de la macromolécule si le lanthanide est fixé (E. Girard, M. Stelter, P. L. Anelli, J. Vicat, R. Kahn, Acta. Cristallogr. D, 2003, D59, 118; E. Girard, P.L. Anelli, J. Vicat, R. Kahn, Acta. Cristallogr. D, 2003, D59, 1877 et E. Girard, M. Stelter, J. Vicat, R. Kahn, Acta. Cristallogr. D, 2003, D59, 1914-1922). A ce jour, cinq complexes de lanthanide sont commercialisés, en tant qu'agent phasant par la société NatX-ray.

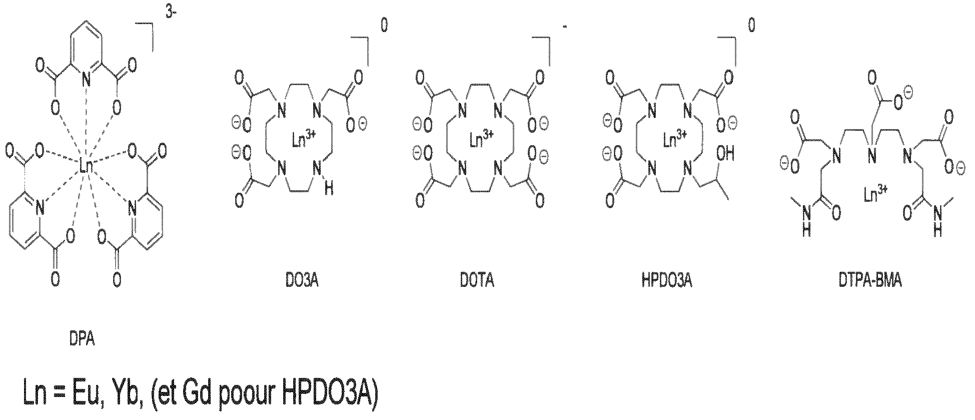

Il existe dans la littérature quelques agents phasants luminescents, comme des dérivés fonctionnels du DPA₃ (FR 2 991 322 et ACIE 2008) ou des complexes de lanthanide directement greffés à la structure des protéines (X. C. Su, H. B. Liang, K. V. Loscha and G. Otting, J. Am. Chem. Soc., 2009, 131, 10352-10353).

Parmi tous ces dérivés, les complexes tri-anioniques [(DPA)₃Ln]³⁻ semblaient très prometteurs car des interactions spécifiques avec des acides aminés cationiques et en particulier, avec l'arginine ont été mises en évidence par certains des inventeurs de la présente demande de brevet (E. Dumont, G. Pompidor, A. D'Aléo, J. Vicat, L. Toupet, R. Kahn, E. Girard, O. Maury, N. Giraud, PhysChemChemPhys 2013, 15, 18235-18242), et confirmées par des mesures de RMN (X. C. Su, H. B. Liang, K. V. Loscha, G. Otting, J. Am. Chem. Soc., 2009, 131, 10352-10353). G. Pompidor et al. dans Angew. Chem. Int. Ed., 2008, 47, 3388-3391 étudient, également, les propriétés du complexe DPA, pouvant expliquer son intérêt dans la cristallographie macromoléculaire. Malheureusement, ce composé s'est avéré instable dans un grand nombre de formulation composant les kits de cristallisation commerciaux : (i) la présence de métaux de transition (Zn(II), Cu(II), Fe(II)) induit la déstructuration du complexe, (ii) la présence de sels alcalino-terreux divalents (Ba(II), Ca(II), Mg(II)) provoque l'auto-cristallisation immédiate du complexe donnant lieu à une détection de faux-positifs (cristaux de complexes et non de cristaux dérivés de protéines) (Thèse de doctorat de R. Talon soutenue à Grenoble le 6 Juin 2012). Les travaux de T. M. Corneillie et al. dans JACS, 2003, 15039-15048 étudient, quant à eux, la structure cristalline d'un anticorps 2D12.5 Fab lié à un complexe d'un anologue du DOTA-yttrium ou gadolinium.

D'autres solutions ont proposé d'introduire des lanthanides dans les cristaux de protéines, pour obtenir un effet phasant. Les travaux de R. Talon et al. dans Chem. Commun. 2012, 48, 11886-11888 portent, également, sur l'utilisation du complexe DPA et de ses analogues CuAAC (cycloaddition azide-alcyne, catalysée au cuivre), en tant qu'agent phasant pour la détermination de structure de protéine par Rayons X. Nagem et al. (Nagem, R. A., Dauter, Z. & Polikarpov I, Acta Crystallogr. D, 2001, D57, 996-1002) ont proposé d'utiliser les sels de lanthanide et la méthode du trempage rapide (Dauter Z., Dauter, M. & Rajashankar, K. R. Acta Crystallogr. D, 2000, D56, 232-237). Cette méthode développée initialement pour les sels d'halogénure (NaCl, NaI, NaBr) consiste à tremper, pendant un temps inférieur à la minute, les cristaux dans une solution saline très concentrée (> 1 mol L⁻¹). Dans le cas du sel de lanthanide, cette méthode a conduit à une dégradation rapide des cristaux. I. Pérez-Dorado et al. dans Acta Crystallization Communications, 2010, F66, 448-451, ont, quant à eux, décrit la cristallisation de la protéine autolysine de pneumocoque par trempage dans une solution avec le complexe HDPO3A du gadolinium.

Purdy et al. (Purdy M. D., Ge P., Chen J., Selvin P. R., & Wiener, M. C. Acta Crystallogr. D, 2002, D58, 1111-1117) ont proposé d'utiliser une liaison covalente reliant des complexes de lanthanide (où le ligand assure une coordination complète du lanthanide) à la protéine. La liaison est constituée par un pont disulfure formé entre des cystéines libres de la protéine et une fonction réactive thiol portée par le complexe. Enfin, Silvaggi et al. (Silvaggi, N. R., Martin, L. J., Schwalbe, H., Imperiali, B., Allen, K. N. J., Am. Chem. Soc., 2007 129, 7114-7120) ont proposé l'utilisation d'un « tag » fixant un ou deux lanthanides (LBT pour Lanthanide-Binding Tag). Le LBT est fondé sur une séquence peptidique dérivée des boucles à calcium qui peut être introduite dans les protéines par les techniques de biologie moléculaire classiques (Allen, K. N. Imperiali B., Current Opinion in Chemical Biology, 2010, 14, 247-254).

Il existe donc un besoin de disposer de nouveaux agents phasants qui ne nécessitent pas de modifier la structure des macromolécules biologiques, dont on souhaite déterminer la structure. De manière additionnelle, l'invention se propose de fournir des agents phasants qui soient suffisamment stables dans la plupart des conditions de cristallisation des macromolécules biologiques et qui permettent également d'élargir les possibilités d'obtention de cristaux permettant d'obtenir des informations structurales sur la macromolécule biologique d'intérêt.

Dans ce contexte, l'invention concerne des complexes cationiques tels que définis à la revendication 1.

Les ligands selon l'invention comprennent au moins 7 sites de coordination pour un ion lanthanide Ln³⁺, voire plus, en fonction de la structure du ligand. Ces sites de coordination se trouvent sur les deux atomes d'azote représentés sur les formule (IA) à (IE), sur les deux pyridines des groupements **R₁**, sur les deux groupes **R₇**, au moins un autre site de coordination se trouvant sur l'une et/ou l'autre des parties du ligand reliant les deux -N(**R₁**)-.

Les complexes selon l'invention, ainsi que les complexes de lanthanide formés avec les ligands de formules (I.1) : 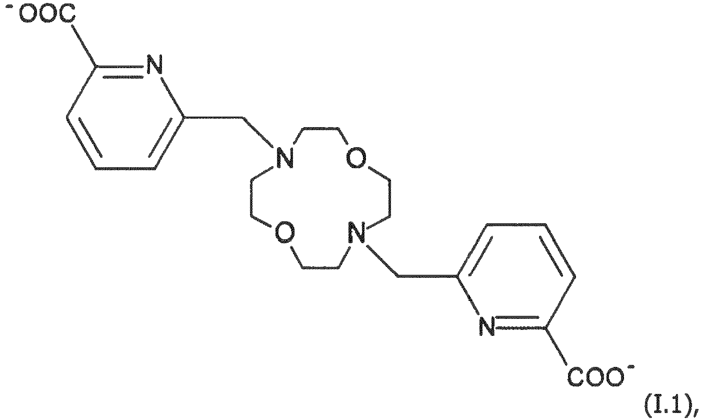 et (I.4) : 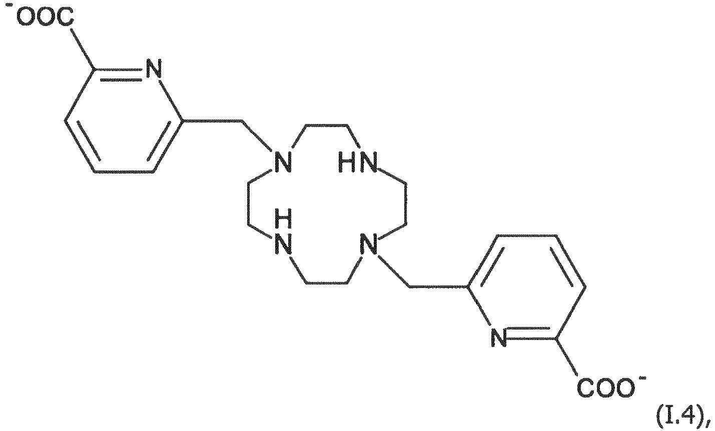 et les ligands de formule (IF) : 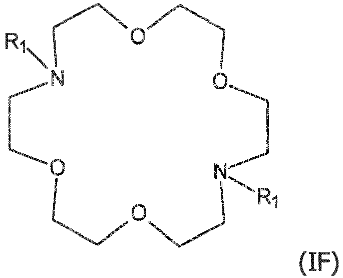 avec R₁ tel que défini à la revendication 1,
sont cationiques et présentent une charge positive supérieure ou égale à 1. Ils comportent un ligand macrocyclique incorporant plusieurs groupements aromatiques formant une sphère de coordination ouverte pour l'ion lanthanide. Ces groupes aromatiques, lorsque l'ion Ln³⁺ est Eu³⁺ ou Tb³⁺, agissent également comme antennes pour sensibiliser la luminescence du lanthanide dans le visible.

Par ailleurs, les complexes selon l'invention, présentent l'avantage d'être solubles dans l'eau et stables dans la plupart des milieux de cristallisation commerciaux. Dans le cadre de l'invention, il a été démontré que ces complexes présentaient un intérêt, non seulement en tant qu'agent phasant, lors de l'obtention de données structurales, mais également en tant qu'aide à la cristallisation. En particulier, ledit complexe est utilisé en tant qu'agent nucléant et/ou en tant qu'agent cristallisant lors de la cristallisation d'une macromolécule biologique. De telles applications n'avaient jamais été décrites ou envisagées pour les complexes de lanthanide formés avec les ligands de formules (I.1) ou (I.4), ou le ligand de formule (I.2) couvert par la formule (IF) : 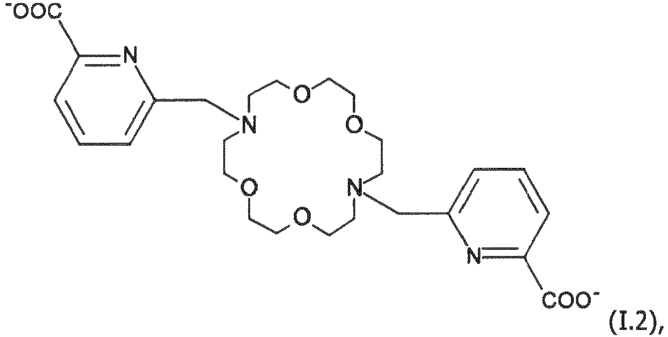 déjà décrits dans la littérature (M. Mato-Iglesias et al., Inorg. Chem. 2008, 47, 7840-7851 ; Z. Palinkas et al., Inorg. Chem. 2009, 48, 8878-8889 ; A. Roca-Sabio et al., Dalton Trans, 2011, 40, 384-392 et M. Regueiro-Figueroa et al., Inorg. Chem. 2015, 54, 4940-4952 pour le complexes du ligand (I.1) et (I.2) et A. Rodrigues-Rodrigues et al., Inorg. Chem. 2012, 51, 2509-2521, pour le complexe du ligands (I.4)). En effet, ces complexes ont été préparés pour des études fondamentales de chimie de coordination des lanthanides (étude de la stabilité des complexes formés avec différents lanthanides) et dans le cas du Gd, seules des applications en tant qu'agent de contraste IRM ont été envisagées.

Les complexes selon l'invention, ainsi que leurs sels avec un anion, leurs solvats et hydrates, sont formés avec un ligand répondant à l'une des formules suivantes : 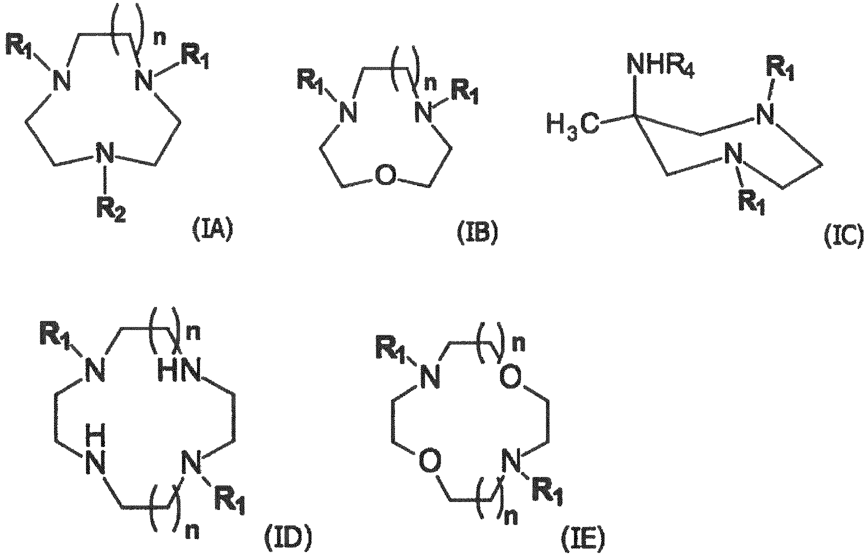 avec **R₁**, **R₂**, **n** et **R₄** qui sont tels que définis à la revendication 1.

A titre d'exemple de sels, des complexes selon l'invention ou des complexes formés avec le ligand (I.1), (I.4) ou (IF) qui trouvent application dans le cadre de l'invention, on peut citer les sels d'un complexe cationique avec un anion (ou plusieurs anions, en fonction de la charge du complexe) choisi parmi : Cl⁻, Br⁻, I⁻, OH⁻, NO₃⁻, triflate, PF₆⁻, SbF₆⁻, B(Ph)₄⁻, BF₄⁻, les sulfates, sulfonates, carbonates, phosphates, phosphonates et carboxylates. Les sulfates et sulfonates pourront correspondre à SO₄²⁻, HSO₃⁻ ou R'SO₃⁻, les carbonates à CO₃²⁻ , HCO₂⁻ ou R'CO₂⁻, les phosphates et phosphonates à R'OPO₃²⁻ et R'PO₃²⁻ et les carboxylates à R'CO₂⁻, avec R' qui peut être, notamment, un groupe alkyle ou aryle, notamment un groupe alkyle comprenant de 1 à 4 atomes de carbone ou un groupe phényle. Sous la forme de sels, les complexes, selon l'invention, pourront également se trouver sous la forme d'hydrate ou solvats, c'est-à-dire avec au moins une molécule d'eau ou de solvant dans la sphère de coordination du lanthanide.

De manière préférée, les complexes selon l'invention, ainsi que leurs sels avec un anion, leurs solvats et hydrates, sont formés avec un ligand répondant à la formule (IA) : 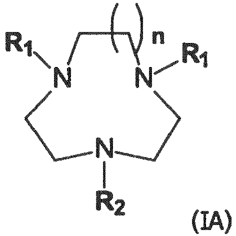 dans laquelle **n**=1 et **R₂**=H, et **R₁** est tel que défini à la revendication 1.

De manière avantageuse, dans les ligands de formule (IA) à (IF), **R₁** représente : 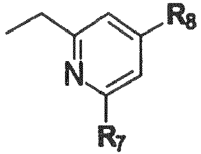 avec :
- **R₇** qui représente -COO⁻ ou -P**R₉**OO⁻ avec **R₉** qui représente un groupe
   méthyle ou éthyle ; ou bien **R₇** qui représente un groupe: 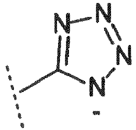
- **R₈** qui représente un atome d'hydrogène, de fluor, chlore, brome ou iode.

Dans le cadre de l'invention, lorsqu'un substituant représente un groupe picolinyle, il représente, de préférence :

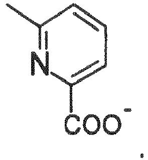

De manière préférée, les complexes selon l'invention sont choisis parmi les complexes de formule : 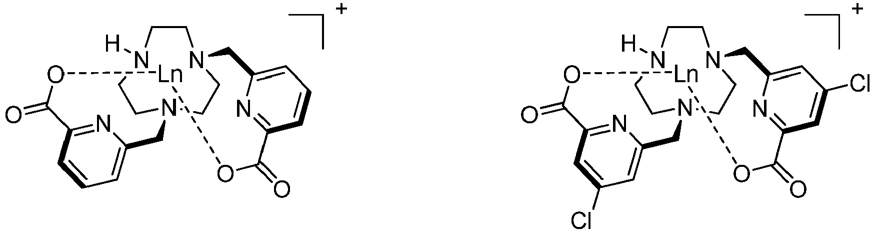 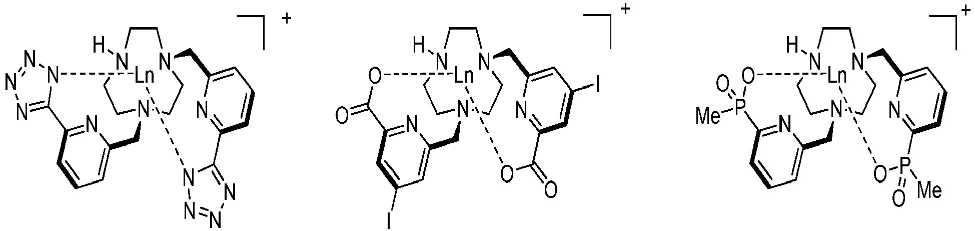 ainsi que leurs sels avec un anion, en particulier, leur sel chlorhydrate, leurs solvats et hydrates.

De manière avantageuse, les complexes selon l'invention ou les complexes formés avec l'un des ligands (I.1), (I.4) ou (IF) qui trouvent application dans le cadre de l'invention, ainsi que leurs sels avec un anion, en particulier leur sel chlorhydrate, leurs solvats et hydrates, sont formés avec un ion lanthanide Ln³⁺, Ln étant Sm, Eu, Gd, Tb, Dy, Ho, Er, Tm, Yb ou Lu, avec Eu, Tb, Yb et Lu qui sont préférés.

L'invention a également pour objet l'utilisation d'un complexe selon l'invention, ou d'un complexe cationique formé d'un ion lanthanide Ln³⁺ et d'un ligand de formule (I.1), (I.4) ou (IF) telles que précédemment définie, ou d'un de leurs sels avec un anion, leurs solvats ou hydrates, en tant qu'aide à la cristallisation d'une macromolécule biologique choisie parmi les peptides et les protéines. En particulier, ledit complexe est utilisé en tant qu'agent nucléant et/ou en tant qu'agent cristallisant lors de la cristallisation d'une macromolécule biologique choisie parmi les peptides et les protéines.

Tous les complexes de lanthanide selon l'invention, ou formés avec un ligand de formule (I.1), (I.4) ou (IF), présentent un intérêt pour leur effet nucléant ou cristallisant. Cependant, seuls les complexes de terbium ou d'europium présenteront des propriétés de luminescence dans le visible. Aussi, si dans le complexe, Ln =Eu ou Tb, le complexe pourra également être utilisé en tant qu'agent luminescent pour la détection de cristaux.

L'invention a également pour objet l'utilisation d'un complexe selon l'invention, ou d'un complexe cationique formé d'un ion lanthanide Ln³⁺ et d'un ligand de formule (I.1), (I.4) ou (IF) telle que précédemment définie, ou d'un de leurs sels avec un anion, leurs solvats ou hydrates, en tant qu'aide à l'obtention de données structurales d'une macromolécule biologique choisie parmi les peptides et les protéines. En particulier, ledit complexe est utilisé en tant qu'agent phasant lors de la détermination structurale par diffraction des rayons X. Là encore, si dans le complexe, Ln =Eu ou Tb, le complexe pourra également être utilisé en tant qu'aide au positionnement du cristal dans un faisceau de rayons X.

En particulier, dans le cadre de la description, par « macromolécule biologique », on entend en particulier les peptides (enchainement de moins de 100 acides aminés), les protéines (enchainement d'au moins 100 acides aminés) et les acides nucléiques, notamment les ADN ou ARN. De telles macromolécules biologiques présenteront notamment une masse moléculaire moyenne supérieure à 1 kDa.

L'invention a également pour objet les cristaux d'une macromolécule biologique choisie parmi les peptides et les protéines, comprenant un complexe selon l'invention ou un complexe cationique formé d'un ion lanthanide Ln³⁺ et d'un ligand de formule (I.1), (I.4) ou (IF) telle que précédemment définie, ou un de ses sels avec un anion, solvat ou hydrate, nommés cristaux dérivés.

Il est possible d'utiliser un complexe selon l'invention, ou un complexe cationique formé d'un ion lanthanide Ln³⁺ et d'un ligand de formule (I.1), (I.4) ou (IF) telle que précédemment définie, dans un procédé de cristallisation d'une macromolécule biologique, de préférence choisie parmi les peptides, les protéines et les acides nucléiques, notamment les ADN ou ARN, comprenant les étapes suivantes :
- disposer d'une solution comprenant ladite macromolécule biologique et un complexe selon l'invention, ou un complexe cationique formé d'un ion lanthanide Ln³⁺ et d'un ligand de formule (I.1), (I.4) ou (IF) telle que précédemment définie, ou un de ses sels avec un anion, solvat ou hydrate,
- obtenir des cristaux de la macromolécule à partir de ladite solution.

La solution utilisée pourra comprendre, en outre, un agent précipitant, autre que le complexe, notamment choisi parmi ceux utilisés dans les kits de cristallisation commerciaux, en particulier parmi ceux présents dans les tableaux présentés en Annexes 2A ou 2B, comme par exemple, les sels tels que les sels d'ammonium, les sulfates (notamment le sulfate d'ammonium), acétates, phosphates, citrates (notamment le citrate de sodium), formiates, tartrates tels que le tartrate de sodium ou de potassium, le tartrate double de sodium et de potassium, les chlorures, iodures et fluorures, par exemple NaCl ; les polymères tels que les polyéthylèneglycols et la Jeffamine T ; l'éthanol, le dioxane, le méthylpentanediol, le glycérol, l'isopropanol, 2-méthyl-2,4-pentanediol.

De manière avantageuse, dans la solution, le complexe est présent à une concentration de 1 à 100 mM, préférentiellement à une concentration de 1 à 25 mM, et de manière encore plus préférée à une concentration de 10 mM ± 10%.

En particulier, l'obtention de cristaux est réalisée par cristallisation par diffusion de vapeur, par dialyse, en batch ou encore dans un procédé de cristallisation en phase cubique de lipides.

Un tel procédé de cristallisation pourra être intégré à un criblage ou à une optimisation de conditions de cristallisation d'une macromolécule biologique. Ledit procédé de cristallisation pourra être mis en oeuvre de manière automatisée.

L'invention trouve également application dans un procédé d'analyse ou de détermination de la structure d'une macromolécule biologique comprenant les étapes suivantes :
a) disposer d'un cristal dérivé de la macromolécule biologique tel que défini dans le cadre de l'invention,
b) analyser la structure cristalline de la macromolécule biologique à partir dudit cristal dérivé.

Le cristal dérivé pourra être obtenu selon un procédé de cristallisation tel que décrit précédemment ou encore par trempage d'un cristal de la macromolécule biologique dans une solution d'un complexe selon l'invention ou d'un complexe cationique formé d'un ion lanthanide Ln³⁺ et d'un ligand de formule (I.1), (I.4) ou (IF) telle que précédemment définie, ou d'un de leurs sels avec un anion, leurs solvats ou hydrates. Le cristal dérivé peut également être obtenu par trempage d'un cristal dérivé de la macromolécule biologique obtenu selon un procédé de cristallisation tel que décrit précédemment, dans une solution d'un complexe selon l'invention ou d'un complexe cationique formé d'un ion lanthanide Ln³⁺ et d'un ligand de formule (I.1), (I.4) ou (IF) telle que précédemment définie, ou d'un de leurs sels avec un anion, leurs solvats ou hydrates.

En particulier, l'analyse de la structure cristalline de la macromolécule biologique à partir dudit cristal dérivé correspond à la détermination de la structure de la macromolécule biologique et est réalisée par diffraction des Rayons X (RX). Il est également possible qu'elle corresponde à l'obtention de données structurales par une méthode haute résolution, telle que la diffraction des Rayons X (RX) ou par une méthode à basse résolution par les méthodes SAXS (Small-Angle X-ray Scattering) ou MASC (Multiwavelength-Anomalous Solvent Contrast).

La description détaillée ci-après permet de mieux comprendre l'invention.

Les complexes cationiques formés d'un ion lanthanide Ln³⁺ et d'un ligand de formule (IA) à (IF) sont des complexes cationiques, et en particulier mono-cationiques. Ils sont donc capables de conduire à la formation de cristaux dérivés d'une macromolécule biologique. De tels cristaux dérivés peuvent être obtenus par trempage ou par co-cristallisation, comme détaillé plus loin. L'effet anomal dû à la présence des lanthanides dans les cristaux dérivés obtenus, conduit à un effet phasant et facilite la détermination de la structure de la macromolécule biologique concernée, notamment par diffraction des rayons X. Les complexes selon l'invention peuvent donc être utilisés en tant qu'aide à la détermination ou à l'analyse de la structure cristalline d'une macromolécule biologique.

Les complexes cationiques formés d'un ion lanthanide Ln³⁺ et d'un ligand de formule (IA) à (IF), ainsi que leurs sels, solvats et hydrates peuvent également être utilisés en tant qu'aide à la cristallisation de macromolécules biologiques. En effet, en fonction de leur structure, et en fonction des conditions de cristallisation mises en œuvre, les complexes selon l'invention présentent au moins l'une des propriétés suivantes :
- effet nucléant : la présence dudit complexe de lanthanide lors de la cristallisation provoque la croissance de cristaux dans des conditions (tampon et/ou additifs et/ou température et/ou concentration et/ou durée...) qui ne permettent pas la formation de cristaux en l'absence du complexe selon l'invention.
- effet cristallisant : la présence dudit complexe de lanthanide lors de tests d'une série de conditions de cristallisation (nombre de cristaux et/ou taille des cristaux et/ou amélioration de la diffraction des cristaux obtenus...) permet d'améliorer la cristallisation obtenue, par rapport à une série de conditions différant par l'absence de complexe. En l'absence de complexe, des cristaux apparaissent mais sont de moins bonne qualité.

En particulier, les complexes de lanthanide formés d'un ion lanthanide Ln³⁺ et d'un ligand de formule (IA) à (IF), ainsi que leurs sels, solvats et hydrates, soit permettent d'augmenter le nombre de conditions dans lesquelles l'obtention de cristaux d'une macromolécule biologique est possible, soit de travailler dans des conditions plus faibles en macromolécule biologique et/ou en autre agent précipitant classiquement utilisé, soit d'améliorer la qualité ou le nombre de cristaux favorisant ainsi l'obtention de données structurales ultérieures, soit d'obtenir plusieurs de ces avantages.

Les complexes d'Europium et de Terbium présentent également l'avantage d'être luminescents sous irradiation UV. Ainsi, leur utilisation permet une mise en évidence aisée des cristaux dérivés lors des essais de cristallisation. Cette propriété peut être utilisée comme méthode de détection rapide pour les plateformes de cristallisation équipées d'imageur automatique sous irradiation UV. De plus, cette propriété peut être utilisée comme une aide au centrage dans un faisceau de rayons X lors d'une caractérisation par diffraction notamment.

Les complexes selon l'invention pourront être préparés selon des techniques adaptées par l'homme du métier, techniques décrites dans les exemples ou dans la demande WO 2014/162105. La synthèse des complexes selon l'invention est généralement réalisée dans l'eau. Les complexes ainsi formés seront donc plutôt sous la forme d'hydrate, mais le complexe peut ensuite être mis dans un solvant autre, du type alcool ou DMSO et donner lieu à un solvat ou encore être déshydraté. Sous forme hydratée, un complexe selon l'invention comportera, notamment jusqu'à 3 molécules d'eau par complexe. En moyenne, le nombre de molécules d'eau par complexe, pourra, par contre, être différent d'un nombre entier, et être par exemple égal à 0,5. Pour les applications envisagées dans le cadre de l'invention, les complexes de lanthanide selon l'invention pourront être utilisés sous forme hydratée, ou déshydratée, solvatée ou non, sous la forme de poudre ou en solution.

La qualité et la pureté d'un agent cristallisant sont essentielles au bon déroulement de la cristallisation d'une macromolécule, lorsque ces derniers sont utilisés en tant qu'agent de cristallisation. Aussi, au final, les complexes de lanthanide selon l'invention seront soumis à une étape de purification adaptée, notamment par chromatographie d'exclusion stérique.

Les complexes cationiques formés d'un ion lanthanide Ln³⁺ et d'un ligand de formule (IA) à (IF), ainsi que leurs sels, solvats et hydrates, pourront être utilisés en tant qu'agent cristallisant dans toute technique adaptée à la cristallisation de macromolécules biologiques.

Pour une description détaillée de telles techniques, on pourra se référer aux ouvrages de référence suivants : « Protein Crystallization, Second Edition (IUL Biotechnology Series) » édité par Therese Bergfors ou « Crystallization of Nucleic Acids and Proteins: A Practical Approach » édité par Arnaud Ducruix et Richard Giegé.

En particulier, les complexes cationiques formés d'un ion lanthanide Ln³⁺ et d'un ligand de formule (IA) à (IF), ainsi que leurs sels, solvats et hydrates pourront être utilisés en tant qu'agent cristallisant dans un procédé de cristallisation de macromolécules biologiques par diffusion de vapeur, par dialyse, en batch ou encore dans un procédé de cristallisation en phase cubique de lipides.

Ces différentes techniques vont chacune être décrites succinctement, l'agent cristallisant s'entendant d'un complexe de lanthanide ou mélange d'agents cristallisants contenant au moins un complexe de lanthanide avec un ligand de formule (IA) à (IF), sous forme de sels, solvats ou hydrates :
- La cristallisation par diffusion de vapeur est la plus utilisée actuellement. C'est la méthode généralement mise en œuvre au niveau des plateformes de cristallisation automatisée. Il existe trois approches différentes schématisées sur la **Figure 2** : les techniques de la goutte assise (« Sitting drop » en anglais), de la goutte suspendue (« Hanging drop » en anglais) et de la goutte en sandwich (« Sandwich drop » en anglais). Ces trois techniques sont représentées schématiquement sur la **Figure 2****.**

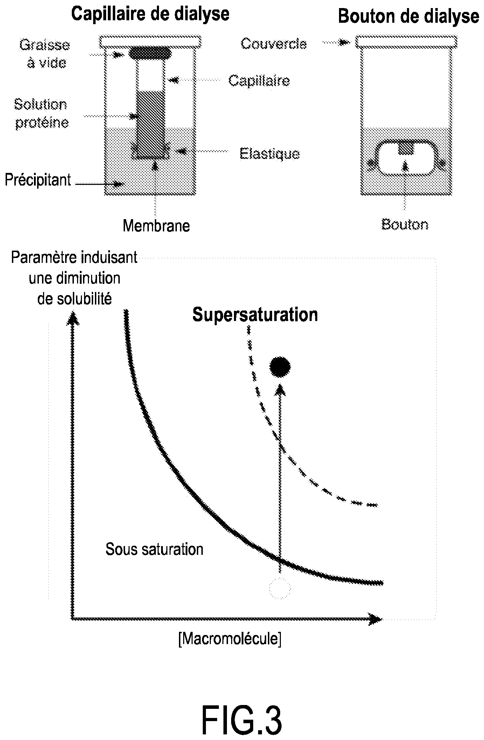

Dans ces trois approches, une solution de l'agent cristallisant est placée dans un puits (solution en gris clair). La solution contenant la macromolécule biologique d'intérêt est mélangée avec l'agent cristallisant et déposée sous forme de goutte (soit suspendue, soit assise ou encore en sandwich). Le puits de cristallisation est fermé hermétiquement par une lamelle de verre ou un film plastique. Au démarrage de la cristallisation, la concentration en agent cristallisant dans la goutte est deux fois plus faible que dans le puits. Le système étant clos, il se met en place une cinétique de diffusion du solvant de la goutte (généralement de l'eau) vers le puits. Le volume de la goutte va donc diminuer (perte de solvant ie. d'eau) et les concentrations en macromolécule biologique et en agent cristallisant vont ainsi augmenter permettant, dans les cas favorables, d'atteindre la phase de nucléation. Dans cette méthode, la concentration en agent cristallisant est extrêmement importante. En effet, un excès d'agent cristallisant pourrait entraîner une cinétique de diffusion du solvant trop rapide et donc entraîner la précipitation de la macromolécule biologique. A l'inverse, une concentration trop faible ne permettra pas d'atteindre la zone de nucléation. La concentration adaptée en agent cristallisant sera donc déterminée, par des tests de routine, par l'homme du métier. A partir de la nucléation, la croissance cristalline va ensuite faire diminuer la concentration en macromolécule soluble jusqu'à atteindre un état stationnaire.
- La dialyse est une autre méthode de cristallisation qui reste cependant moins utilisée que la diffusion de vapeur. Cette technique est représentée schématiquement sur la **Figure 3****.**

Le principe général consiste à faire croitre, de manière lente, la concentration en agent cristallisant. Pour cela, une solution contenant la macromolécule biologique d'intérêt est placée dans un contenant de dialyse, par exemple du type capillaire ou bouton de dialyse fermé par une membrane semi-perméable laissant passer les molécules d'agent cristallisant (mais pas la macromolécule). Ce contenant est alors placé dans une solution d'agent cristallisant. Un équilibre entre la solution contenant la macromolécule biologique d'intérêt (qui ne contient pas d'agent cristallisant) et la solution extérieure contenant l'agent cristallisant va se mettre en place. Ainsi, la concentration en agent cristallisant dans le contenant de dialyse va augmenter et modifier la solubilité de la macromolécule. Cette technique présente l'avantage d'être plus précise que la diffusion de vapeur en ce qui concerne le maintien du pH et des volumes. Cependant, la manipulation des cristaux reste beaucoup plus délicate.
- La cristallisation en batch est la dernière technique utilisée pour cristalliser les macromolécules biologiques. Cette technique, tout comme la dialyse, nécessite un volume important de solution.

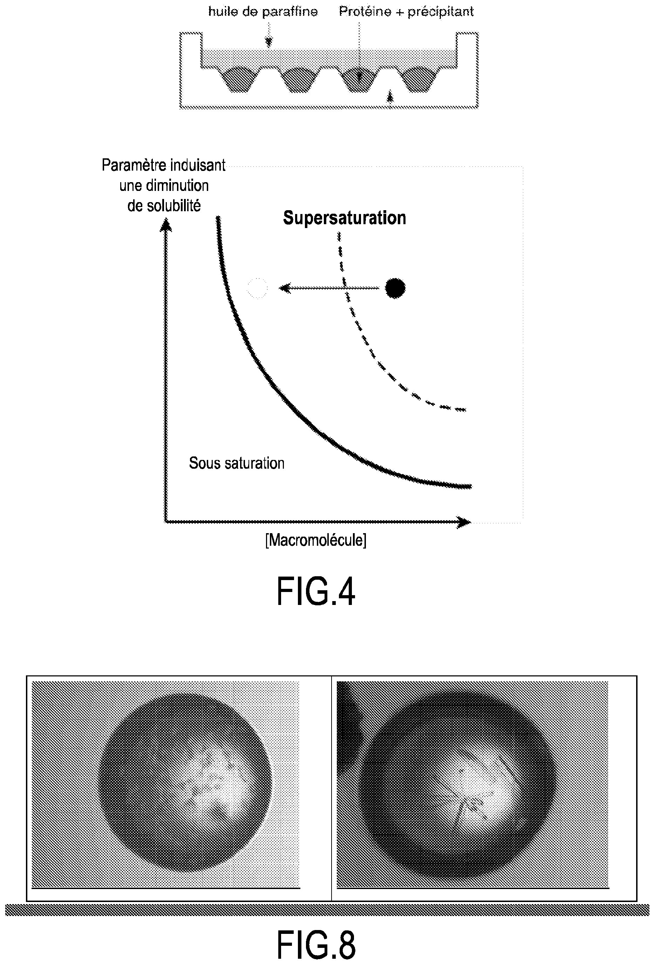

Le principe de cette technique, représenté schématiquement sur la **Figure 4****,** est simple : la macromolécule très concentrée est mélangée à l'agent cristallisant. La solution sursaturée en macromolécule va, avec le temps, induire la formation de nucléii qui va induire la diminution de la concentration de la macromolécule en solution, vers la formation de cristaux.

Les techniques de cristallisation par diffusion de vapeur, de cristallisation par dialyse ou de cristallisation en batch fonctionnent sur tous les types de protéines qu'elles soient solubles dans l'eau ou qu'elles soient membranaires, alors solubles en détergent, ainsi que sur les autres macromolécules biologiques (ADN, ARN, complexes protéiques, complexes protéine-ADN ou ARN...).

Pour la cristallisation des protéines membranaires, il est également possible d'utiliser un complexe de lanthanide, en tant qu'agent cristallisant dans une technique de cristallisation en phase cubique de lipides. La cristallisation en phase cubique de lipides des protéines membranaires est décrite dans les publications suivantes : Landau et al. Lipidic cubic phase: A novel concept for the crystallization of membrane proteins, PNAS Vol. 93 no. 25 14532- 14535 (1996), Caffrey et al. Crystallizing membrane proteins using lipidic mesophases. Nat Protoc 4 (5) 706-31 (2009), Cherezov V., Lipidic cubic phase technologies for membrane protein structural studies. Curr. Opin Struct Biol Vol 21 559-566 (2011).

Les complexes de lanthanide formés d'un ion lanthanide Ln³⁺ et d'un ligand de formule (IA) à (IF), ainsi que leurs sels, solvats et hydrates sont compatibles avec toutes les méthodes de cristallisation qui viennent d'être détaillées.

En particulier, une solution contenant une concentration de 1 à 100 mM, préférentiellement 1 à 25 mM d'un complexe de lanthanide formés d'un ion lanthanide Ln³⁺ et d'un ligand de formule (IA) à (IF), sous forme d'un sel avec un anion, éventuellement sous la forme d'un solvat ou d'un hydrate, et de manière encore plus préférée de 10 mM ± 10% sera utilisée.

Dans les cristallisations, en particulier, en phase vapeur ou en batch, une solution de la macromolécule biologique à cristalliser et d'un complexe de lanthanide formé d'un ion lanthanide Ln³⁺ et d'un ligand de formule (IA) à (IF), sous forme d'un sel avec un anion, éventuellement sous la forme d'un solvat ou d'un hydrate (qui sera, par souci de simplification, dans le reste de la description, nommé complexe de lanthanide), sera préparée, avec de préférence une concentration de 1 à 100 mM, préférentiellement 1 à 25 mM, et de manière encore plus préférée de 10 mM ± 10%, en complexe de lanthanide. Le complexe de lanthanide formé d'un ion lanthanide Ln³⁺ et d'un ligand de formule (IA) à (IF), sous forme d'un sel avec un anion, éventuellement sous la forme d'un solvat ou d'un hydrate, peut être directement solubilisé par la solution contenant la macromolécule biologique pour atteindre une concentration finale en complexe de 1 à 100 mM, de préférence de 1 à 25 mM, et préférentiellement de 10 mM ± 10% qui correspond à la concentration à laquelle l'effet nucléant est prépondérant. Le complexe de lanthanide peut aussi être introduit à partir d'une solution à une concentration préférentielle de 10 à 30 mM, lors de la fabrication d'une goutte de cristallisation : sont alors ajoutés successivement un volume de la solution de macromolécules, un volume identique de la solution de complexe de lanthanide et un volume identique de solution de puits utilisé pour la cristallisation.

Dans ces techniques de cristallisation, le complexe de lanthanide et la macromolécule biologique à cristalliser seront mis en solution généralement dans de l'eau ou dans une solution aqueuse tamponnée (voir exemples dans la description des kits de cristallisation figurant dans les Annexes 2A et 2B), par exemple, dans une solution tampon acétate, cacodylate, citrate, Bis tris propane, TRIS, HEPES, phosphate. Les solutions contenant une macromolécule biologique sont généralement tamponnées. Si une solution du complexe de lanthanide est formée, elle ne sera pas forcément tamponnée. La solution contenant le complexe, la solution contenant la macromolécule biologique et/ou la solution contenant la macromolécule biologique et le complexe, en fonction de la technique de cristallisation utilisée présenteront, de préférence, un pH dans la gamme allant de 3 à 9.

Des additifs tels que des additifs déjà connus pour jouer le rôle d'agent précipitant/cristallisant ou des additifs permettant de favoriser au départ la dissolution de la macromolécule biologique à cristalliser pourront être ajoutés. A titre d'exemples, on peut citer ceux présents dans les tableaux présentés en Annexe 2A ou 2B, comme par exemple, les sels tels que les sels d'ammonium, les sulfates (notamment le sulfate d'ammonium), acétates, phosphates, citrates (notamment le citrate de sodium), formiates, tartrates tels que le tartrate de sodium ou de potassium, le tartrate double de sodium et de potassium, les chlorures, iodures et fluorures, par exemple NaCl ; les polymères tels que les polyéthylèneglycols et la Jeffamine T ; l'éthanol, le dioxane, le méthylpentanediol, le glycérol, l'isopropanol, 2-méthyl-2,4-pentanediol. En particulier, il est possible d'utiliser le complexe de lanthanide dans toute solution de cristallisation déjà commercialisée.

La concentration en macromolécule dans la solution sera en théorie proche de sa limite de solubilité dans ladite solution, mais pourra être plus diluée, en fonction de la solution utilisée.

Les complexes de lanthanide formés d'un ion lanthanide Ln³⁺ et d'un ligand de formule (IA) à (IF), sous forme d'un sel avec un anion, éventuellement sous la forme d'un solvat ou d'un hydrate, pourront également être utilisés pour obtenir des cristaux dérivés de macromolécules biologiques, c'est-à-dire des cristaux contenant ledit complexe de lanthanide. En particulier, ledit complexe de lanthanide sera fixé sur une position spécifique de la macromolécule biologique à étudier permettant la détermination de la structure de ladite macromolécule biologique étudiée.

La détermination des phases peut être faite avec des cristaux dérivés obtenus dans une solution contenant de 1 à 100 mM de complexe, préférentiellement de 10 à 100 mM, et de manière encore plus préférée de 100 mM ± 10% au dit complexe de lanthanide. Cependant, pour améliorer la qualité du phasage, un trempage dans une solution d'un complexe de lanthanide, en particulier, avec une concentration élevée en complexe, peut également être réalisé. Cela permet d'augmenter le taux d'occupation des sites occupés par le complexe.

La co-cristallisation consiste donc à ajouter le complexe de lanthanide pendant le processus de cristallisation. Ainsi, lors de la cristallisation, le complexe de lanthanide peut s'insérer dans des sites cristallins spécifiques conduisant à l'obtention de cristaux dérivés.

Par ailleurs, pour obtenir un cristal dérivé ou augmenter l'occupation par un complexe de lanthanide de sites dans le cristal dérivé, un trempage d'un cristal d'une macromolécule biologique dans une solution de complexe de lanthanide peut être effectué. Il est possible de réaliser un trempage rapide ou un trempage long.

Le trempage rapide consiste à prélever un cristal d'une macromolécule biologique d'intérêt et à le tremper de manière brève (notamment de 45 secondes à 2 minutes) dans une solution du complexe de lanthanide. De manière avantageuse, la solution dans laquelle le cristal est trempé présente une forte concentration en complexe de lanthanide, typiquement une concentration de 50 à 500 mM, de préférence de 100 mM ± 10%.

En particulier, un trempage rapide lors d'une phase de congélation d'un ou plusieurs cristaux peut être réalisé. Un trempage rapide lors d'une phase de congélation se déroule typiquement en trois étapes : des cristaux sont prélevés de leur goutte d'origine et trempés dans une goutte de trempage, correspondant à une solution équivalente à la solution d'origine utilisée pour la formation du(des)dit(s) cristal(aux) à laquelle est ajoutée une concentration de 50 à 500 mM, de préférence de 100 mM ± 10% de complexe de lanthanide, ainsi qu'un cryoprotectant tel qu'utilisé par l'homme de l'art. Après une durée de 45 secondes à 2 minutes, le cristal est trempé dans une solution de cryoprotection sans complexe de lanthanide, afin d'en éliminer l'excès. Enfin, le cristal est plongé dans l'azote liquide et stocké à basse température, par exemple à 100K.

Cette méthode de trempage est très efficace et permet d'augmenter les taux d'occupation et d'améliorer la qualité du phasage. A titre d'exemple, dans le cas de la protéine PhP1 un trempage de 45 secondes dans une solution à 100 mM de complexe 10 a augmenté le taux d'occupation du complexe par 3. Un trempage court permet d'éviter que le cristal ne se dégrade et de préserver les qualités de diffraction de ce dernier.

Le trempage long consiste à prélever un cristal d'une macromolécule biologique d'intérêt et à le tremper de manière prolongée (notamment de 10 minutes à 24 heures) dans une solution du complexe de lanthanide. De manière avantageuse, la solution dans laquelle le cristal est trempé présente une forte concentration en complexe de lanthanide, typiquement une concentration de 50 à 500 mM, de préférence de 100 mM ± 10%.

A l'exception de la durée, le trempage long peut se dérouler selon le même protocole que celui décrit pour le trempage rapide, notamment lors d'une phase de congélation. Cependant, la goutte de trempage est placée en équilibre avec un puits contenant de la solution d'origine utilisée pour la formation du(des)dit(s) cristal(aux), afin d'éviter une déshydratation de la goutte.

Quel que soit le type de trempage, il est possible de tremper aussi bien des cristaux obtenus par co-cristallisation, en présence d'un complexe de lanthanide, que des cristaux obtenus en l'absence d'un tel complexe (nommés cristaux natifs dans le cadre de la présente demande de brevet). Le trempage n'entraîne aucune destruction ou modification de la macromolécule biologique.

Les complexes de lanthanide permettent de former des cristaux dérivés d'une macromolécule biologique permettant l'obtention de données structurales pour ladite macromolécule biologique. En particulier, l'obtention de données structurales sera obtenue à partir d'un cristal dérivé d'une macromolécule biologique intégrant un complexe de lanthanide qui joue le rôle d'agent phasant. Ces données structurales pourront être obtenues par une méthode haute résolution, telle que la diffraction des Rayons X (RX) ou par une méthode basse résolution, telles que les méthodes SAXS et MASC. Les complexes de lanthanide pourront être utilisés en tant qu'agent phasant en diffraction des RX (cristallisation et analyse de structure) ou en diffusion des RX, ou encore en tant qu'agent de contraste en SAXS ou MASC, en microscopie électronique.

L'utilisation des complexes de lanthanide formés d'un ion lanthanide Ln³⁺ et d'un ligand de formule (IA) à (IF), sous forme d'un sel avec un anion, éventuellement sous la forme d'un solvat ou d'un hydrate, en tant qu'agent phasant notamment, présente l'avantage de ne pas nécessiter une modification de la macromolécule biologique par génie génétique, comme dans le cas de l'obtention de protéines séléniés. Aucune liaison covalente n'intervient entre le complexe de lanthanide et la macromolécule biologique d'intérêt.

Les exemples ci-après, en référence aux figures annexées, permettent d'illustrer l'invention mais ne présentent aucun caractère limitatif.
La **Figure 1** représente un diagramme de phase illustrant le processus de cristallisation (Extrait de http://people.ds.cam.ac.uk/ml527/publications/assets/leunissen-literature-research.pdf)
La **Figure 2** représente trois approches différentes : les techniques de la goutte assise (« Sitting drop » en anglais), de la goutte suspendue (« Hanging drop » en anglais) et de la goutte en sandwich (« Sandwich drop » en anglais).
La **Figure 3** représente la technique de dialyse : autre méthode de cristallisation qui reste cependant moins utilisée que la diffusion de vapeur.
La **Figure 4** représente schématiquement une technique de cristallisation en batch utilisée pour cristalliser les macromolécules biologiques.
La **Figure 5** représente des diagrammes de phase de la protéine lysozyme en absence (à droite) et en présence de complexe 10 ou 17 déterminés à différents temps de cristallisation : les conditions, ayant donné des cristaux, sont illustrées par des points noirs, les points blancs étant des gouttes claires.
La **Figure 6** représente des diagrammes de phase du lysozyme de blanc d'œuf de poule en absence et en présence de 10mM de complexe 11 déterminés après 4 jours de cristallisation. Les conditions donnant des cristaux sont illustrées par des points noirs, les points blancs sont des gouttes claires et les triangles des précipités.
La **Figure 7** représente des diagrammes de phase de la protéine PB6 en absence et en présence de 10mM de complexe 10 déterminés après un jour de cristallisation. Les conditions donnant des cristaux sont illustrées par des points noirs, les points blancs sont des gouttes claires et les triangles des précipités.
La **Figure 8** représente des cristaux de pb6 (condition pb6-1) en présence de 10 mM de complexe 10 à droite (13% PEG - 10 mg/ml) et sans complexe 10 à gauche (15 % PEG - 20 mg/ml).
La **Figure 9** représente la mise en évidence de la luminescence induite dans les cristaux par les complexes de lanthanide.
La **Figure 10** représente un spectre de fluorescence X mesuré sur une solution de 10 mM de complexe **10** (à gauche) et traitement par le logiciel CHOOCH (à droite). Les longueurs d'onde pour les enregistrements permettant d'exploiter de manière optimale la diffusion anomale du lanthanide (ici le terbium) sont indiquées par une flèche.
La **Figure 11** représente des exemples de cartes de densité électronique expérimentale.
La **Figure 12** représente des images obtenues au robot de cristallisation HTXlab.

### Exemples de réalisation

### Partie I : Synthèse et caractérisation des complexes

Les abréviations ci-après sont utilisées :
Me = méthyle
Moz = méthoxybenzyloxycarbonyle
Boc = tert-butoxycarbonyle
Ms = mésyle
Et = éthyle
TA = température ambiante
Ac = acétyle
DCM = dichlorométhane
TACN = triazacyclononane
DMF = diméthylformamide
TFA = acide trifluoroacétique
ACN = acétonitrile
CCM = chromatographie sur couche mince

### Produits de départ et caractérisation

Tous les produits de départ, solvants et sels de lanthanide ont été achetés chez Sigma-Aldrich®, Acros Organics® et TCI® avec des puretés supérieures à 98% pour les composés organiques et supérieures à 99,99% pour les sels de lanthanide. Ces produits ont été utilisés directement sans purification supplémentaire.

Les chromatographies ont été effectuées, d'une part, sur alumine neutre activité III obtenue par hydratation d'alumine Acros Organics® d'activité I (60Â), et d'autre part, sur gel de silice Acros Organics® (60Â). Les complexes formés ont tous été purifiés par colonne d'exclusion stérique LH20 de chez Sephadex®.

Les spectres RMN (¹H, ¹³C) ont été enregistrés sur deux appareils Bruker® Avance opérant respectivement à 500.10 MHz et 125.75 MHz pour ¹H et ¹³C pour l'un, et à 300 MHz pour ¹H pour le second. Les déplacements chimiques sont reportés en partie par million (ppm) relativement au signal du tetraméthylsilane (¹H, ¹³C), les pics résiduels des solvants étant utilisés comme référence interne.

Les mesures de masses exactes ont été effectuées au Centre Commun de spectrométrie de Masse (Villeurbanne, France).

### A) Préparation d'une première série de ligands/complexes à base de triazacyclononane (TACN)

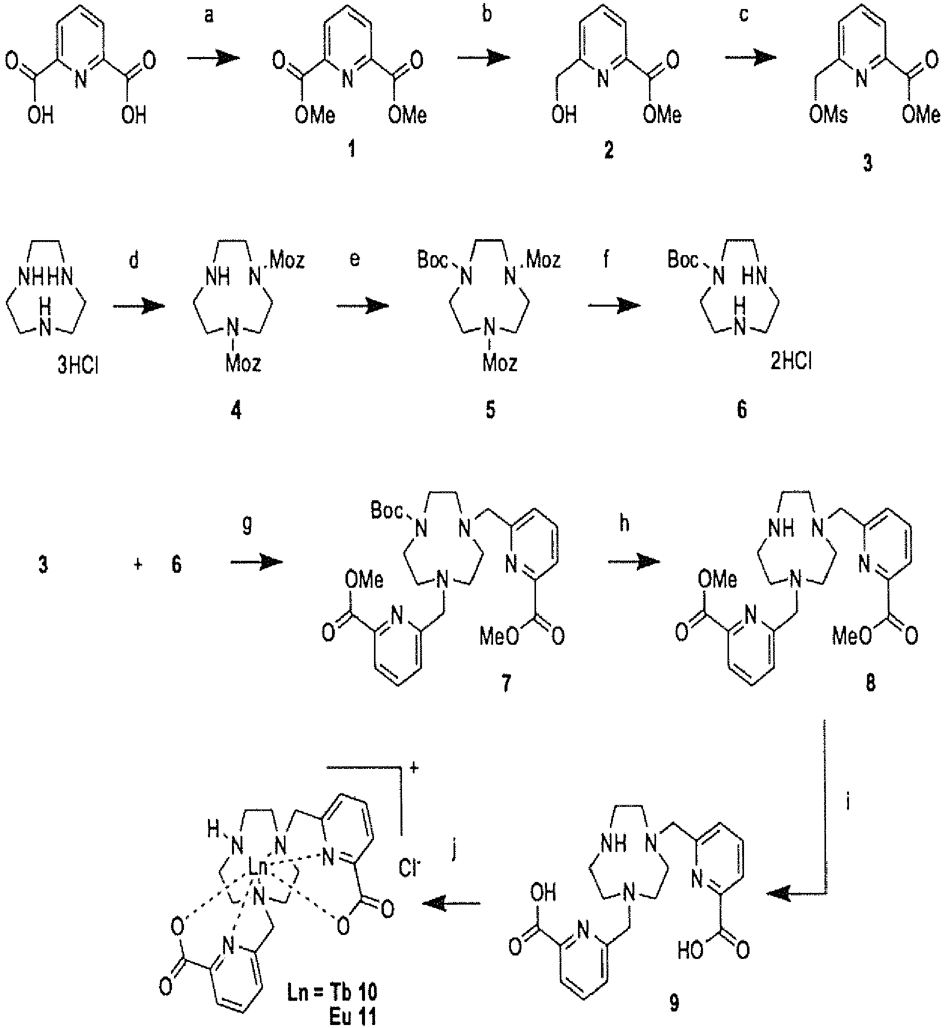

a) HCl (cat.) dans MeOH b) NaBH₄ dans DCM/MeOH c) MsCl, Et₃N dans DCM d) Moz-on, Et3N dans ACN e) Boc-O-succinimide dans DCM f) H₂, Pd/C dans MeOH g) Na₂CO₃ dans MeOH/H₂O h) TFA dans DCM i) Na₂CO₃ dans ACN j) Na₂CO₃ dans H₂O puis LnCl₃,6(H₂O) avec Ln=Tb ou Eu

Le composé **6** a été préparé selon la procédure précédemment décrite dans la demande de brevet WO2013/011236A1.

### A1) Préparation du composé 1

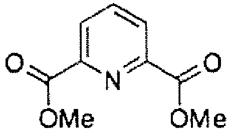

A une suspension de 20 g d'acide dipicolinique (0,12 mol, 1éq.) dans 120 mL de méthanol, on ajoute 1 mL d'acide sulfurique concentré. Le mélange est porté à reflux pendant 24h. Après refroidissement, le produit cristallisé est filtré et rincé au méthanol froid pour donner, après séchage, 18 g de poudre cristalline blanche du composé **1** (R = 78%).
¹H-NMR (300 MHz, CDCl₃) δ (ppm) = 8.26 (d, J = 8 Hz, 2H), 7.99 (t, J = 8 Hz, 1H), 3.98 (s, 6H).

### A2) Préparation du composé 2

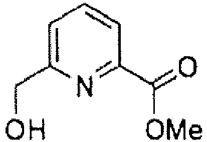

18 g de composé **1** (92 mmol, 1 éq.) sont dissouts dans 450 ml de méthanol et refroidis à 0°C. 3,8 g de NaBH₄ (101,2 mmol, 1,1 éq.) sont alors ajoutés. Le mélange est ensuite agité 30 min à 0°C et 30 min supplémentaires en laissant la température remontée lentement jusqu'à TA. La réaction est stoppée par ajout de 50 mL de HCl (1M dans H₂O), puis, la phase organique est extraite avec 100 mL de dichlorométhane. Après, lavage de la phase organique avec de la saumure (jusqu'à pH = 7), séchage avec Na₂SO₄ et évaporation, le produit obtenu est purifié par chromatographie sur gel de silice (éluant: DCM/AcOEt 9/1 v/v jusqu'à AcOEt pur). On obtient 6 g d'un solide blanc du composé **2** (R = 40%).
¹H-NMR (300 MHz, CDCl₃) δ (ppm) = 8.01 (d, J = 8 Hz, 1H), 7.84 (t, J = 8 Hz, 1H), 7.53 (d, J = 8 Hz, 1H), 4.85 (d, J = 3 Hz, 2H), 3.98 (s, 3H), 3.69 (bs, 1H).

### A3) Préparation du composé 3

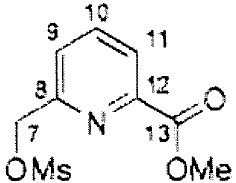

A une solution de 1,2 g de l'alcool **2** dans 50 mL de dichlorométhane à 0°C, on ajoute 3mL de Et₃N (22 mmol, 3,2 éq.) puis, rapidement, 0,79 mL de chlorure de mésyle (10,2 mmol, 1,5éq.). On laisse ensuite le mélange revenir à température ambiante avant de le chauffer pendant 1h à 50°C. On ajoute ensuite 40 mL d'eau et on extrait le mélange au dichlorométhane (3x20 mL). Le résidu huileux obtenu, après séchage et évaporation des phases organiques, est finalement purifié sur gel de silice (éiuant : CH₂Cl₂) pour obtenir une huile incolore du composé **3** qui cristallise au congélateur.
¹H NMR (500 MHz, CDCl₃) δ 8.12 (d, J = 7.7 Hz, 1H), 7.93 (t, J = 7.8 Hz, 1H, H10), 7.70 (d, J = 7.8 Hz, 1H), 5.44 (s, 2H, H7), 4.01 (s, 3H, OMe), 3.15 (s, 3H, OMs). ¹³C NMR (126 MHz, CDCl₃) δ 165.33 (C13), 154.59 (s), 147.92 (s), 138.45 (C10), 125.41 (s), 125.13 (s), 71.11 (C7), 53.23 (OMe), 38.24 (OMs).

### A4) Préparation du composé 6

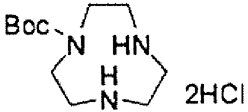

Le composé **6** est préparé par la méthode précédemment décrite. (a) WO2013011236A1 ; b) 1.S. J. Butler, B. K. McMahon, R. Pal, D. Parker and J. W. Walton, Chem. Eur. J., 2013, 19, 9511-9517.)

### A5) Préparation du composé 7

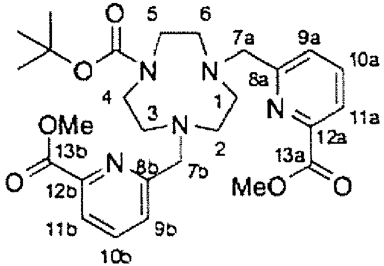

360 mg de composé **6** (1,19 mmol) et 760 mg de carbonate de sodium (7,15 mmol, 6 éq.) sont séchés sous pression réduite dans un schlenk avant d'ajouter 100 mL d'acétonitrile. Sous argon, 710 mg de composé **3** (2,74 mmol, 2,3 éq.) sont ajoutés à la suspension avant chauffage 12h à 70°C. Après retour à la température ambiante, le mélange est filtré sur fritté (porosité 4) et concentré sous pression réduite. Le produit est purifié par chromatographie sur alumine (activité III, éluant DCM puis DCM/MeOH 96/4 v/v) pour finalement obtenir une huile jaune du composé 7 (532 mg, Rendement : 80%).
¹H NMR (500 MHz, CDCl₃) δ 7.99 (dd, J = 6 Hz, 2H, H11), 7.86 - 7.65 (m, 4H, H9), 3.98 (s, 10H, H7+OMe), 3.39 (d, J = 30 Hz, 4H, H4H5), 3.11 (s, 2H, H3H6), 2.95 (s, 2H, H3'H6'), 2.66 (d, J = 31 Hz, 4H, H1H2), 1.44 (s, 9H, boc).
¹³C NMR (126 MHz, CDCl₃) δ 166.01, 166.09 (C13), 161.43, 161.60 (C8), 155.88(CO(boc)) 147.45(C12), 137.42 (d,C10), (s), 126.19,126.46 (C9), 123.70 (C11), 79.48 (C(boc)), 63.70 (d, C7), 57.21 (C1C2), 55.37 (s), 55.11 (s), 54.67 (s), 53.04 (OMe), 50.54, 49.95 (C4C5), 28.72 (CH₃(boc)).

### A6) Préparation du composé 8

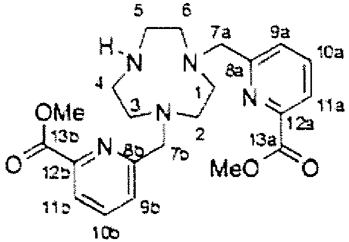

Dans 100 mL de dichlorométhane, 5 mL d'acide trifluoroacétique sont ajoutés sur une solution de 427 mg du composé **7.** Après 5h d'agitation à la température ambiante, le mélange est évaporé en éliminant le TFA par entrainement avec plusieurs ajouts de toluène. Le résidu obtenu est purifié par chromatographie sur alumine (activité III, éluant DCM/MeOH (gradient 1% jusqu'à 7%)). Le solide visqueux jaunâtre obtenu du composé **8** est conservé sous argon au congélateur (masse : 315 mg, rendement : 90%).
¹H NMR (500 MHz, CDCl₃) δ 7.96 (dd, J = 7.7, 0.5 Hz, 2H, H11), 7.71 (t, J = 8 Hz, 2H, H10), 7.42 (dd, J = 7.9, 0.5 Hz, 2H), 3.98 (d, 10H, H7+OMe), 3.40 (t, J = 5 Hz, 4H, H4H5), 3.03 (t, J = 5 Hz, 4H, H3H6), 2.71 (s, 4H, H1H2).
¹³C NMR (126 MHz, CDCl₃) δ 165.37 (C13), 159.43 (C8), 147.59 (C12), 137.87 (C10), 126.10 (C9), 124.02 (C11), 60.95 (C7), 54.60 (C1C2), 53.31 (OMe), 51.66 (C3C6), 46.52 (C4C5).
NB : la synthèse de ce composé a été précédemment décrite dans la référence suivante : A. Nonat, C. Gateau, P. H. Fries, M. Mazzanti, Chem. Eur. J., 2006, 12, 7133.

### A7) Préparation du composé 9

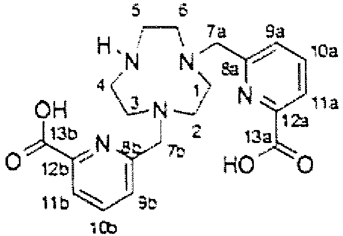

Le ligand **9** est généré in situ par saponification du diester **8** en présence de carbonate de sodium Na₂CO₃ (2 éq.) dans un mélange MeOH/H₂O (1/1, v/v) agité 3h à 50°C.
¹H NMR (500 MHz, D₂O) δ 7.77 (d, J = 7.1 Hz, 2H, H9), 7.69 (t, J = 7.7 Hz, 2H, H10), 7.29 (d, J = 7.2 Hz, 2H, H11), 3.86 (s, 4H, H7), 3.08 (t, J = 5.9 Hz, 4H, H4H5), 2.91 (t, J = 5.9 Hz, 4H, H3H6), 2.65 (s, 4H, H1H2).
¹³C NMR (126 MHz, D₂O) δ 173.22 (C13), 158.44 (C8), 153.01 (C12), 138.23 (C10), 125.17 (C11), 122.40 (C9), 60.27 (C7), 50.53 (C1,C2), 47.12 (C3,C6), 44.10 (C4,C5).

### A8) Préparation du complexe 10

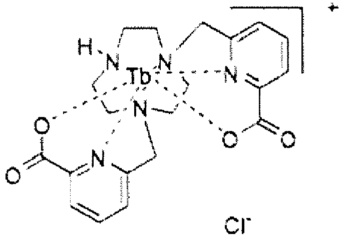

Après réaction pendant 3h à 50°C de 110 mg de diester **8** (0,26 mmol) avec 83 mg de Na₂CO₃ (0,78 mmol, 3,0 éq.) et neutralisation par HCl (1M), la disparition de l'ester est vérifiée par RMN. 96 mg de chlorure de terbium (0,26 mmol, 1 éq.) sont ajoutés au composé **9,** formé *in situ,* avant d'agiter le mélange pendant 12h à 50°C. Solubilisé dans un minimum de méthanol, le complexe **10** est ensuite séparé des différents sels par centrifugation. Les dernières traces de sels sont retirées par passage sur une colonne d'exclusion stérique (LH20, Sephadex®, éluant : eau).
MS (ESI-TOF) calculé M+ : 556.1003 ; mesuré : 556.0990

### A9) Préparation du complexe 11

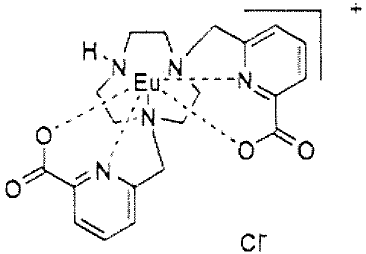

Un protocole identique à celui mis en œuvre pour la préparation du complexe **10** est utilisé avec 170 mg de diester **8** (0,4 mmol) et 219 mg de EuCl₃,6H₂O (0,6 mmol, 1,5 éq.).
MS (ESI-TOF) calculé M+ : 550.0962 ; mesuré : 550.0957.

### B) Préparation d'une deuxième série de ligands à base de triazacyclononane (TACN)

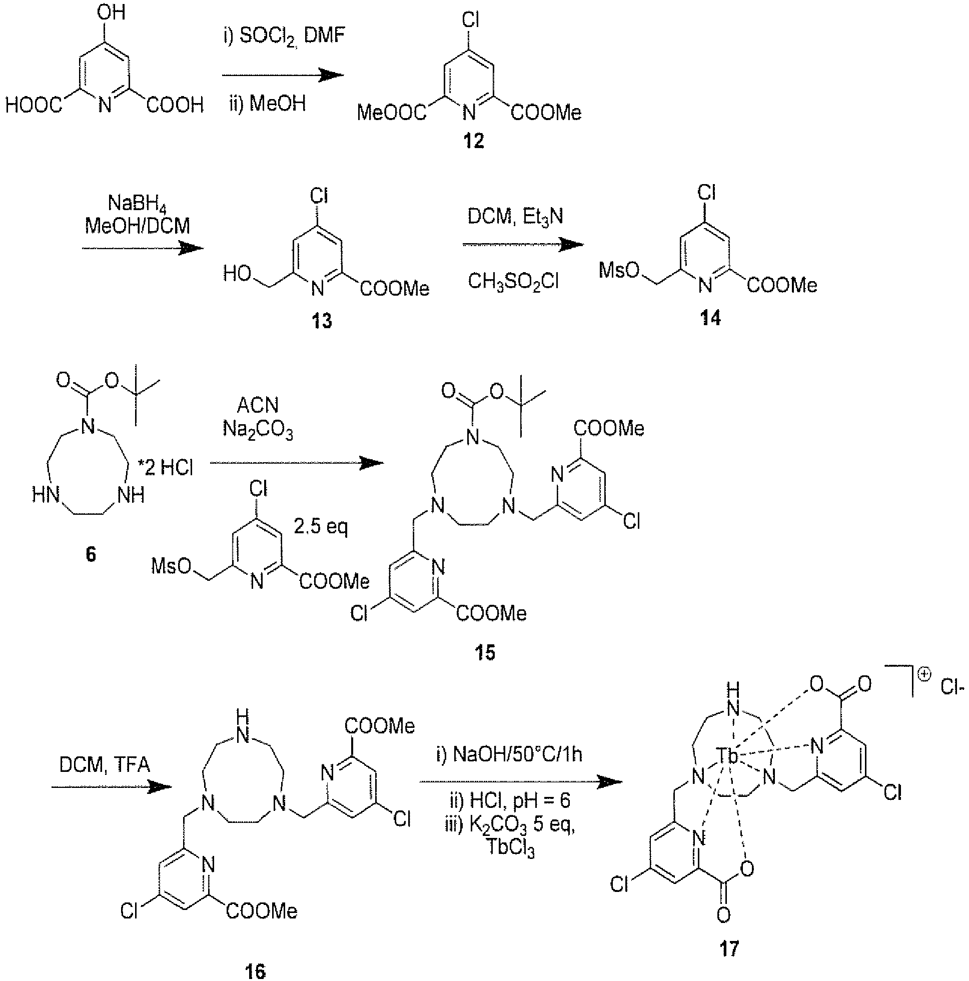

### B1) Préparation du composé 12

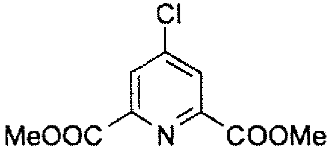

15 g d'acide chélidamique monohydrate (0,075mol, 1éq.) sont dissouts dans 120 mL de chlorure de thionyle sous argon. La suspension est refroidie à 0°C et 3 mL de DMF sont ajoutés. Le mélange réactionnel est ensuite agité pendant 12h à reflux. Les volatiles sont évaporés après plusieurs ajouts de toluène pour enlever les dernières traces de SOCl₂. Le solide jaunâtre obtenu est ensuite dissout dans du méthanol et le mélange est porté à reflux pendant 12h pour achever la réaction. Après, évaporation du solvant, le résidu est repris par du dichlorométhane et lavé successivement par une solution saturée de NaHCO₃, d'eau et de saumure. La phase organique est ensuite séchée sur Na₂SO₄, filtrée et évaporée. Le composé **12** pur est obtenu par recristallisation dans le méthanol pour obtenir 8,3g de poudre cristalline blanche. (R = 44%).
¹H-NMR (300 MHz, CDCl₃) δ (ppm) = 8.29 (s, 2H), 4.03 (s, 6H).

### B2) Préparation du composé 13

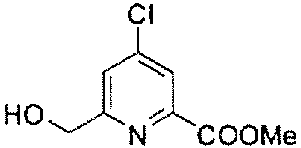

6 g de composé **12** (0,026 mol, 1éq.) sont dissouts dans 250 mL d'un mélange DCM/ méthanol (150/100, v/v) et la solution est refroidie à 0°C. Puis, 1,09g de NaBH₄ (0,029 mol, 1,1 éq.) sont ajoutés en une fois avant agitation du mélange pendant 30 min à 0°C et 30 minute à TA. La réaction est stoppée par l'ajout de 50 mL d'acide chlorhydrique (1M) et de 100 mL d'eau. La phase organique est ensuite lavée à l'eau jusqu'à atteindre un pH = 7, puis lavée à la saumure, séchée sur Na₂SO₄ et évaporée. Le brut réactionnel est ensuite purifié sur gel de silice (éluant : DCM/ AcOEt 1/1 v/v) pour obtenir après évaporation 1,5g d'une poudre blanche composé **13** (R = 30%).
¹H-NMR (300 MHz, CDCl₃) δ (ppm) = 8.00 (bd, 1H), 7.60 (bd, 1H), 4.85 (d, J = 7 Hz, 2H), 3.99 (s, 3H) 3.48 (t, J = 7 Hz, 1H).

### B3) Préparation du composé 14

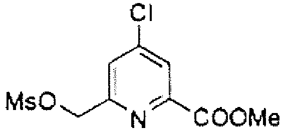

1,5 g de composé **13** (7,4 mmol, 1 éq.) sont dissouts dans 200 mL de dichlorométhane et 3 mL de triéthylamine sont ajoutés (22,2 mmol, 3 éq.). Puis, 0,87 mL de chlorure de mésyle sont ajoutés (11,1 mmol, 1,5 éq.) lentement et une coloration jaune progressive du mélange est observée. L'avancement de la réaction est suivie par CCM et stoppée après 30 min. 100 mL d'une solution saturée de NaHCO₄ sont ajoutés puis la phase organique est lavée à l'eau (jusqu'à pH = 7) et avec de la saumure. La solution organique est ensuite séchée sur Na₂SO₄ et évaporée pour donner 2 g d'une huile jaune du composé **14** utilisée sans purification supplémentaire (rendement quantitatif).
¹H-NMR (300 MHz, CDCl₃) δ (ppm) = 8.1 (s, 1H), 7.68 (s, 1H), 5.40 (s, 2H), 4.01 (s, 3H), 3.17 (s, 3H).

### B4) Préparation du composé 15

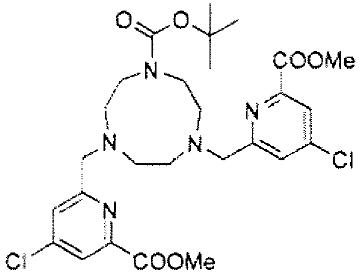

200 mg of triazacyclononane mono-boc (0,7 mmol, 1éq.) sont mis en suspension dans 100 mL d'acétonitrile sec avec 420 mg de carbonate de sodium anhydre. 463 mg de composé **13** (1,75 mmol, 2,5 éq.) sont ensuite ajoutés. Après refroidissement, le mélange est passé sur fritté pour enlever le carbonate avant évaporation du solvant. Le résidu est repris dans du dichlorométhane et purifié par chromatographie sur colonne d'alumine (activité III, éluant: dichlorométhane puis acétate d'éthyle). 250 mg d'un solide jaune du compose **15** (R = 63%).
¹H-NMR (300 MHz, CDCl₃) δ (ppm) = 8.00 ppm (d, J = 1 Hz, 1H), 7.99 (d, J = 1 Hz, 1H), 7.89 (d, J = 1 Hz, 1H), 7.72 (d, J = 1 Hz, 1H), 3.99 (m, 10 H), 3.39 (m, 4H), 3.03 (m, 4H), 2.67 (m, 4H), 1.48 (s, 9H). LC-MS: [M+H]⁺ = 596.2 m/z.

### B5) Préparation du composé 16

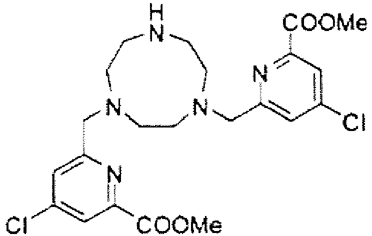

A une solution de 110 mg de composé **15** dans 60 mL de dichlométhane, on ajoute 3 mL d'acide trifluoroacétique. Le mélange est ensuite agité pendant 12h à température ambiante. Le solvant est ensuite évaporé ainsi que le TFA par plusieurs ajouts d'un mélange méthanol/toluène. Le résidu est ensuite repris par 50 mL de dichlorométhane et lavé à l'eau jusqu'à pH = 7. La phase organique est ensuite séchée sur Na₂SO₂ et évaporée pour obtenir 100 mg de poudre blanche du composé **16.** (R = 95%).
¹H-NMR (300 MHz, CDCl₃) δ (ppm) = 7.97 (d, J = 1 Hz, 2H), 7.54 (d, J = 1 Hz, 2H), 4.04 (s, 6H), 4.03 (s, 4H), 3.35 (m, 4H), 3.00 (m, 4H), 2.76 (s, 4H).
¹³C-NMR (75 MHz, CDCl₃) δ (ppm) = 163.91, 160 (q, J = 40 Hz), 147.58, 146.99, 126.49, 125.22, 60.37, 53.80, 53.56, 53.28, 45.39.
HR-MS: [M+H]⁺ = 496.1501 m/z, théor. pour C₂₂H₂₈Cl₂N₄O₄ is 496.1513 m/z.

### B6) Préparation du complexe 17

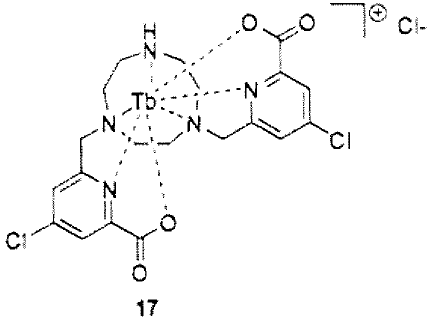

100 mg de composé **16** (0,2 mmol, 1 eq) sont mis en suspension dans 30 mL d'eau et 32 mg d'hydroxyde de sodium (0,81 mmol, 4 eq) sont ajoutés. Le mélange est ensuite agité à 60°C pendant 1h. L'hydrolyse complète des fonctions ester est confirmée par LC-MS. La solution est ensuite refroidie et son pH est ajusté à 5 avec un ajout progressif d'une solution d'acide chlorhydrique (1M). 5 mL de méthanol sont ensuite ajoutés avant d'introduire 42 mg de carbonate de sodium à la solution. 91 mg de TbCl₃, 6H₂O sont ajoutés avant d'agiter le mélange à 50°C pendant 12h. Après retour à température ambiante, les sels insolubles sont filtrés sur verre fritté avant évaporation des solvants pour obtenir 300 mg de produit brut. Le complexe est purifié par chromatographie d'exclusion stérique (Sephadex® LH20, éluant : eau) pour finalement obtenir 55 mg d'un produit cristallin incolore (rendement = 44%).
¹H-NMR (300 MHz, D₂O) δ (ppm) = 121.1, 83.90, 69.15, 52.71, 46.23, 29.58, 26.06, -0.96, -11.39, -20.2, -33.57, -36.26, -44.20, -70.35, -91.19, -92.47, -120.84. HR-MS: M⁺ = 624.0213 m/z, théor. pour C₂₀H₂₁Cl₂N₅O₄Tb 624.0219 m/z.

### C) Préparation d'une troisième série de ligands à base de triazacyclononane (TACN)

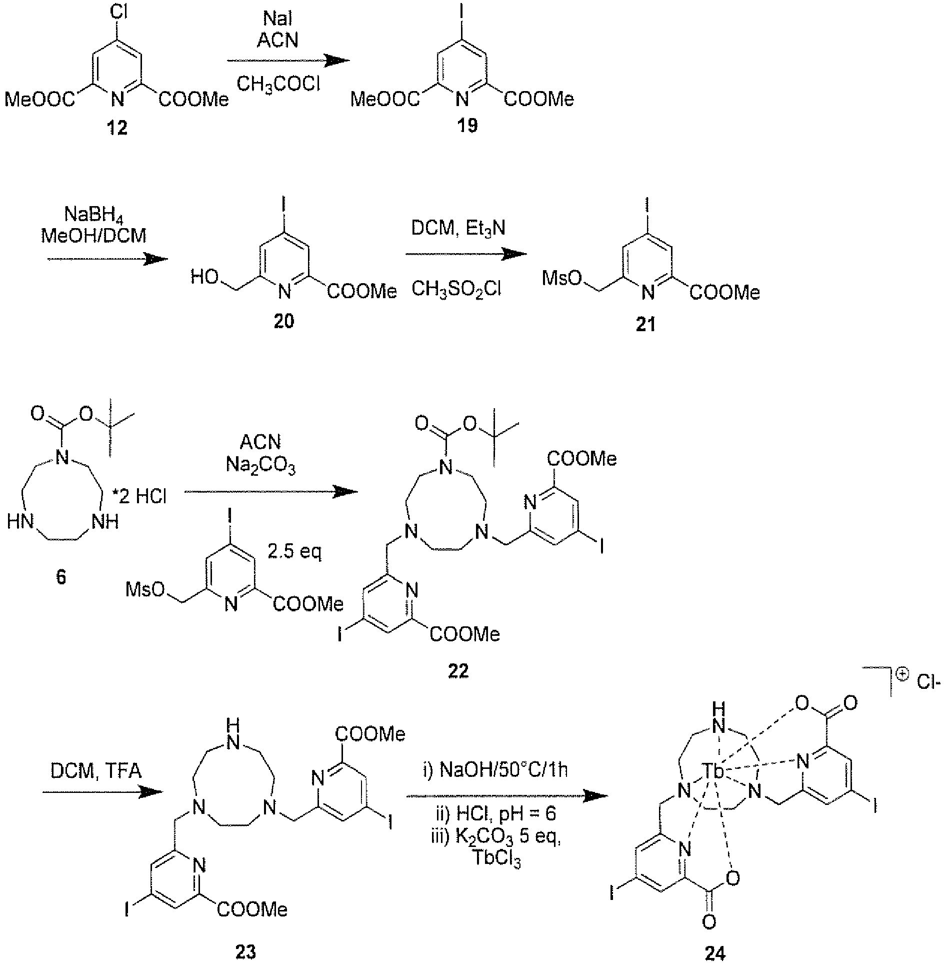

### C1) Préparation du composé 19

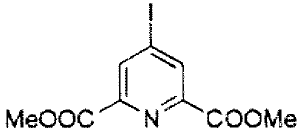

8,3 g de composé **12** (36 mmol, 1 éq.) sont dissouts dans 200 mL d'acétonitrile et 54 g d'iodure de sodium sont ajoutés (0,36 mol, 10 éq.) ainsi que 10 mL de chlorure d'acétyle (0,108 mol, 3 éq.). La suspension est ensuite placée dans un bain à ultrasons pendant 3h. Puis, 200 mL de dichlorométhane sont ajoutés et la phase organique est lavée avec une solution saturée d'hydrogénocarbonate de sodium. La phase organique est ensuite lavée à l'eau jusqu'à pH = 7, séchée sur sulfate de sodium et évaporée pour obtenir 10,7 g de solide blanc cassé du composé **19,** utilisé sans autre purification (R = 92 %).
¹H-NMR (300 MHz, CDCl₃) δ (ppm) = 8.65 (s, 2H), 4.02 (s, 6H).

### C2) Préparation du composé 20

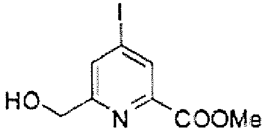

A une solution de 10,7 g (33,3 mmol, 1 éq.) de composé **19** dans 200 ml d'un mélange méthanol/dichlorométhane (140/60) et refroidie à 0°C, 1,39 g de borohydrure de sodium (36,6 mol, 1,1 éq.) est ajouté. Le mélange est ensuite agité pendant 30 min à 0°C et 30 min à la température ambiante. La réaction est ensuite stoppée par ajout de 50 mL d'une solution d'acide chlorhydrique (1M). La phase organique est ensuite lavée à l'eau jusqu'à pH = 7, séchée sur Na₂SO₄ et évaporée. Le produit brut est ensuite purifié sur gel de silice (éluant : dichlorométhane avec ajout progressif de méthanol (0 à 10%)) pour obtenir 5 g d'une poudre blanche du composé **20.** (R = 51%).
¹H-NMR (300 MHz, CDCl₃) δ (ppm) = 8.39 (bd, 1H), 7.97 (bd, 1H), 4.82 (d, J = 5 Hz, 2H), 3.99 (s, 3H) 3.04 (t, J = 7 Hz, 1H).

### C3) Préparation du composé 21

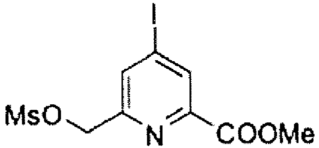

A une solution de 1 g de composé **20** (3,4 mmol, 1 éq.) et de 1,4 mL de triéthylamine (10,2 mmol, 3 éq.) dans 120 mL de dichlorométhane sont ajoutés 0,4 mL de chlorure de mésyle (5,1 mmol, 1,5 éq.). Après 30 minutes d'agitation (réaction suivie par CCM), 100 mL d'une solution saturée de NaHCO₃ sont ajoutés. La phase organique est ensuite lavée à l'eau, séchée sur Na₂SO₄ et évaporée. 1,1 g d'huile jaune du composé **21** est obtenue et utilisée sans autre purification (R = 95%).
¹H-NMR (300 MHz, CDCl₃) δ (ppm) = 8.46 (s, 1H), 8.04 (s, 1H), 5.36 (s, 2H), 4.00 (s, 3H), 3.16 (s, 3H).

### C4) Préparation du composé 22

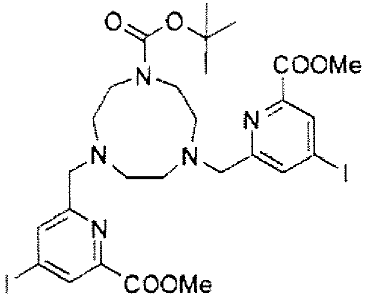

60 mg de triazacyclononane mono-boc **6** (0,2 mmol, 1 éq.) sont mis en suspension dans 50 mL d'acétonitrile sec sous argon avec 140 mg de Na₂CO₃ anhydre (1,2 mmol, 6 éq.). Une solution de 184 mg de composé **21** (0,5 mmol, 2,5 éq.) dans de l'acétonitrile sec est ensuite ajoutée et le mélange est agité pendant 12h à 60°C sous argon. Après refroidissement, le mélange est filtré sur fritté et évaporé. Le résidu est repris par du dichlorométhane et purifié sur colonne d'alumine (activité III ; éluant : dichlorométhane puis acétate d'éthyle). On obtient 110 mg de composé pur **22** sous forme de solide blanc pâteux (R = 71 %).
¹H-NMR (300 MHz, CDCl₃) δ (ppm) = 8.34 (s, 2H), 8.22 (s, 1H), 8.11 (s, 1H), 3.97 (s, 6H), 3.94 (s, 4H), 3.4-2.58 (m, 12 H), 1.47 (s, 9H).
¹³C-NMR (75 MHz, CDCl₃) δ (ppm) = 164.6, 162 (d, J = 14 Hz), 155.61, 147.6 (d, J = 14 Hz), 135.3 (d, J = 14 Hz), 132.7, 106.6, 79.5, 63.1, 56.6, 54.9, 54.4, 53.1, 50.8, 50.2, 28.7. LC-MS: [M+H]⁺ = 780.0 m/z.

### C5) Préparation du composé 23

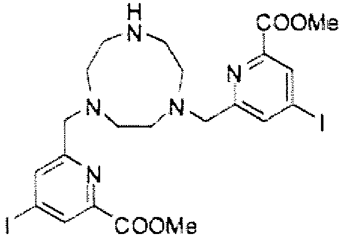

110 mg de composé **22** (0,14 mmol, 1 éq.) sont dissouts dans 50 mL de dichlorométhane, puis un excès d'acide trifluoroacétique est ajouté (3 mL). Le mélange est ensuite agité à température ambiante pendant 12 h. Le solvant est ensuite évaporé et un mélange de 20 mL de dichlorométhane et de 10 mL d'eau est ajouté. La phase aqueuse est neutralisée par ajout d'une solution saturée de NaHCO₃ et extraite avec du dichlorométhane. L'ensemble des phases organiques est séché sur sulfate de sodium et évaporé. On obtient 100 mg de solide blanc (rendement quantitatif) du composé **23** qui est utilisé sans autre purification.
¹H-NMR (300 MHz, CDCl₃) δ (ppm) = 8.23 (s, 2H), 7.83 (s, 2H), 3.92 (s, 10H), 3.27 (s, 4H), 2.92 (s, 4H), 2.67 (s, 4H). ¹³C-NMR (75 MHz, CDCl₃) δ (ppm) = 164, 159.98, 147.81, 134.86, 133.14, 107.08, 59.85, 53.36, 52.65, 49.87, 45.33. HR-MS: [M+H]⁺ = 680 m/z, théor. pour C₂₂I₂H₂₈N₂O₅ 680.0225 m/z.

### C6) Préparation du complexe 24

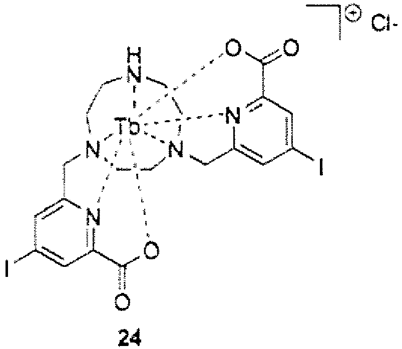

100 mg de composé **23** (0,15 mmol, 1 eq) sont mis en suspension dans 30 mL d'eau et 24 mg d'hydroxyde de sodium (0,81 mmol, 4 eq) sont ajoutés. Le mélange est ensuite agité à 60°C pendant 1h. L'hydrolyse complète des fonctions ester est confirmée par LC-MS. La solution est ensuite refroidie et son pH est ajusté à 5 avec un ajout progressif d'une solution d'acide chlorhydrique (1M). 5 mL de méthanol sont ensuite ajoutés avant d'introduire 42 mg de carbonate de sodium à la solution. 66 mg de TbCl₃, 6H₂O (0,18 mmol, 1,2 eq) sont ajoutés avant d'agiter le mélange à 50°C pendant 12h. Après retour à température ambiante, les sels insolubles sont filtrés sur verre fritté avant évaporation des solvants pour obtenir 200 mg de produit brut. Le complexe est purifié par chromatographie d'exclusion stérique (Sephadex® LH20, éluant : eau) pour finalement obtenir 36 mg d'un produit cristallin incolore (rendement = 30%).
¹H-NMR (300 MHz, D₂O) δ (ppm) = 122.8, 84.95, 82.17, 70.62, 49.22, 25.03, 20.8,-0.89, -8.54, -19.41, -33.05, -41.19, -67.6, -88.49, -90.57, -125.1.
HR-MS: [M+H]⁺ = 807.8921 m/z, théor. pour C₂₀H₂₁I₂N₅O₄Tb 807.8931 m/z.

### D) Préparation d'une quatrième série de ligands à base de triazacyclononane (TACN)

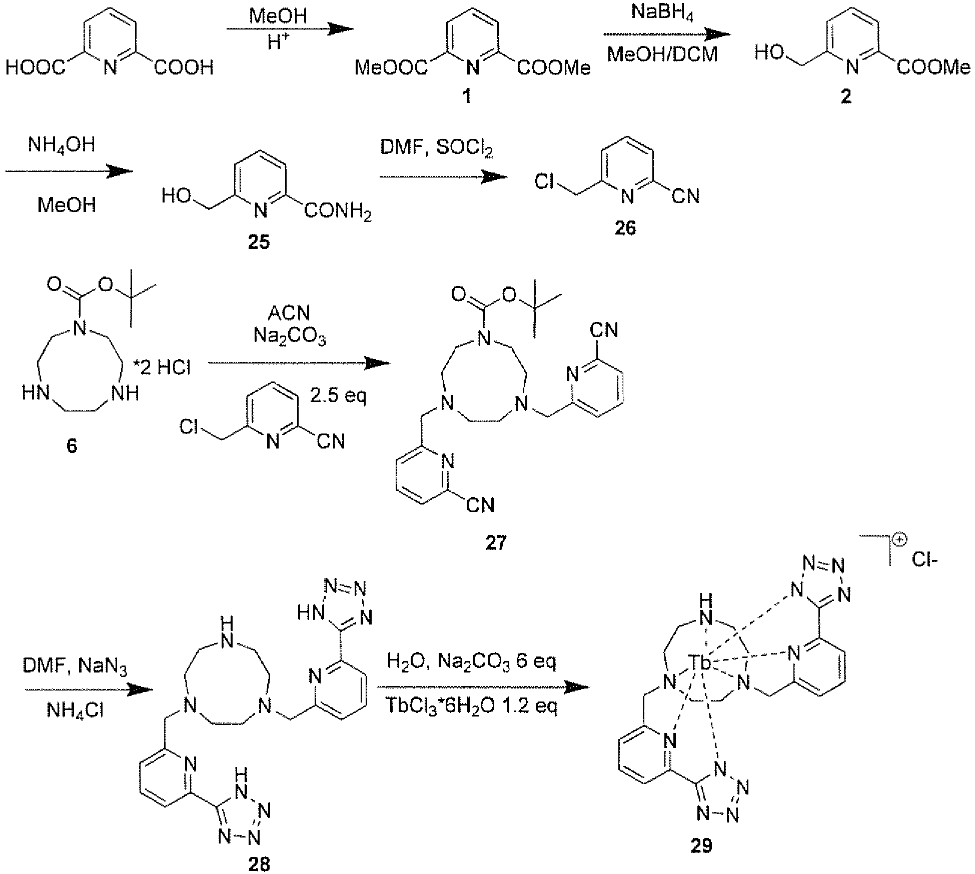

### D1) Préparation du composé 25

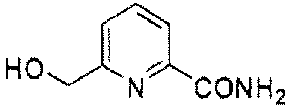

1 g de composé **2** (6 mmol, 1 éq.) est dissout dans 100 mL de méthanol et 8 mL d'un solution aqueuse d'ammoniaque à 30% (60 mmol, 10 éq.). Ce mélange est agité à température ambiante pendant 12 h. Les solvants sont ensuite évaporés, jusqu'à obtenir 900 mg d'un solide blanc du composé **25** pur (R = quantitative).
¹H-NMR (300 MHz, C₂D₆SO) δ (ppm) = 8.14 (bs, 1H), 7.96 (t, J = 8 Hz, 1H), 7.88 (d, J = 8 Hz, 1H), 7.61 (bd, J = 8 Hz, 2H), 4.64 (s, 2H).
LC-MS: [M+H]⁺ = 153.2 m/z.

### D2) Préparation du composé 26

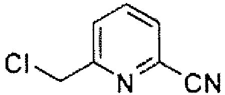

A 1 g de composé **25** (6,6 mmol, 1 éq.) dans 20 mL de DMF à 0°C, 4 mL de chlorure de thionyle (53 mmol, 8 éq.) sont ajoutés. Le mélange est agité pendant 2h, avant de le laissé revenir à température ambiante en 15 min. 150 mL d'eau sont ensuite ajoutés, avant d'extraire le produit avec 30 mL de dichlorométhane. La phase organique est ensuite lavée à l'eau jusqu'à atteindre un pH = 7, puis lavée à la saumure, séchée sur Na₂SO₄ et évaporée. Le résidu obtenu est purifié par chromatographie sur gel de silice (éluant : dichlorométhane) et conduit à l'obtention de 720 mg d'un solide blanc du composé **26** (R = 55%).
¹H-NMR (300 MHz, CDCl₃) δ (ppm) = 7.89 (t, J = 8 Hz, 1H), 7.75 (d, J = 8 Hz, 1H), 7.65 (d, J = 8 Hz, 1H), 4.69 (s, 2H).
LC-MS: [M+H]⁺ = 153.3.

### D3) Préparation du composé 27

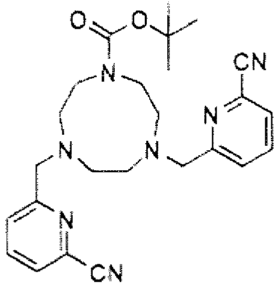

120 mg de triazayclononane protégé par un mono-Boc (0,4 mmol, 1 éq.) sont dissouts dans 50 mL d'acétonitrile sec sous argon avec 252 mg de Na₂CO₃ anhydre (2,4 mmol, 6 éq.). Une solution de 151 mg de composé **26** (1 mmol, 2,5 éq.) dans de l'acétonitrile sec est ensuite ajoutée et le mélange est agité pendant 12h à 60°C sous argon. Après refroidissement, le mélange est filtré sur fritté et évaporé. Le résidu est repris par du dichlorométhane et purifié sur colonne d'alumine (activité III ; éluant : dichlorométhane puis acétate d'éthyle). On obtient 160 mg de composé **27** sous forme de solide blanc pâteux (R = 83 %).
¹H-NMR (300 MHz, CDCl₃) δ (ppm) = 7.79 (dt, ³J = 8 Hz, ⁴J = 1 Hz, 2H), 7.70 (bd, J = 8 Hz, 2H), 7.57 (dt, ³J = 8 Hz, ⁴J = 1 Hz, 2H), 3.91 (s, 4H), 3.36 (m, 4H), 3.03 (m, 4H), 2.66 (m, 4H), 1.47 (s, 9H).
LC-MS: [M+H]⁺ = 462.2 m/z.

### D4) Préparation du composé 28

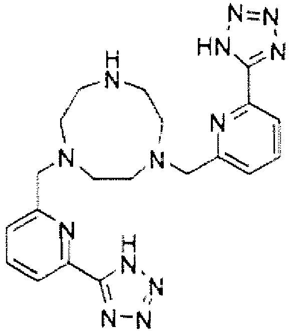

A une solution de 160 mg du composé **27** (0,35 mmol, 1 éq.) dans 40 mL de DMF sec sous argon, 225 mg de NaN₃ (3,5 mmol, 10 éq.) et 185 mg de NH₄Cl sec sont ajoutés. Le mélange est agité à reflux à 120°C pendant 12 h. Après refroidissement, filtration sur fritté et évaporation des solvants, un résidu est obtenu, qui est repris par 50 mL d'acide chlorhydrique (1M) et mis dans un bain à ultrasons pendant 2h.
HR-MS [M+H+] : 604.1439 th 604.1447

### D5) Préparation du composé 29

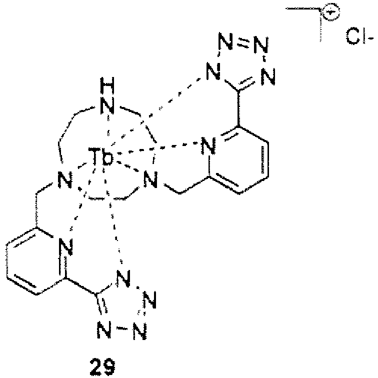

155 mg de composé **28** (0.35 mmol, 1 eq) sont mis en suspension dans 10mL d'eau avec 220 mg de Na₂CO₃ (2.08 mmol, 6 eq). Après 10 min d'agitation, 155 mg de TbCl₃^{∗}6H₂O (0.42 mmol, 1.2 eq) sont ajoutés. La solution est ensuite agitée 12h à température ambiante. Après évaporation des solvants, le produit brut est purifié sur une colonne d'exclusion stérique. (Sephadex ® LH20 dans l'eau) pour finalement obtenir 40 mg de solide blanc cristallin (rendement = 20 %).
HR-MS: [M+H]⁺ = 604.1439 m/z, théor pour C₂₀H₂₃N₁₃Tb 604.1427 m/z.

### E) Préparation d'une première série de ligands à base de 1,7-dioxa-4,10-diazacyclododecane

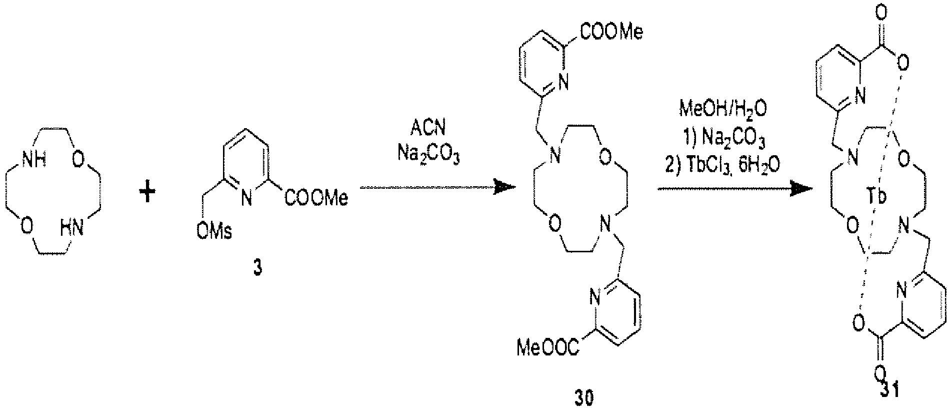

Le composé **31** est préparé selon la méthode précédemment reportée (M. Mato-Iglesias, Adriàn Roca-Sabio, Z. Pélinkas, D. Esteban-Gómez, C. Platas-Iglesias, E. Tóth, A. de Blas, T. Rodríguez-Blas, Inorg. Chem., 2008, 47, 7840).

### Partie II : Etudes cristallographiques

### A) Protéines modèles étudiées

5 protéines, dont trois protéines commerciales ont été sélectionnées. La structure de ces cinq protéines était connue. Ces cinq protéines ont été choisies en raison de leurs différences physico-chimiques. En effet, elles appartiennent à des organismes variés, présentent des propriétés thermiques larges et un état oligomérique allant du monomère à l'hexamère. Des tests, avec des protéines dont la structure est inconnue, ont également été réalisés.

### a. Protéines commerciales

Les trois protéines commerciales sélectionnées sont le lysozyme de blanc d'œuf de poule (HEWL), la Thaumatine de Thaumatococcus danielli et la Protéinase K de Tritirachium album. Ces trois protéines sont des protéines modèles en matière de cristallogenèse. Elles sont achetées sous forme lyophilisée. Elles ont été solubilisées dans de l'eau milliQ juste avant les expériences de cristallogenèse à la concentration souhaitée. Elles sont présentées dans le **Tableau 1** ci-après.

**Tableau 1 : Protéines commerciales utilisées et fournisseur associé**

| **Protéines** | **Nombre d'acides aminés** | **Référence produit Fournisseur** | **Séquence** |
|---|---|---|---|
| **HEWL** | **129** | 10 837 059 001 Roche | **SEQ ID N°1** |
| **Thaumatine** | **207** | T7638 Sigma | **SEQ ID N°2** |
| **Protéinase K** | **384** | 03 115 879 001 Roche | **SEQ ID N°3** |

Les conditions de cristallisation native (c'est-à-dire sans ajout de complexe selon l'invention) de ces protéines sont connues et décrites dans le **Tableau 2** ci-après. Ces conditions ont été reprises pour caractériser l'effet des complexes de lanthanide selon l'invention, sur la cristallogenèse de ces protéines commerciales.

**Tableau 2 : Conditions de cristallisation usuelles des protéines commerciales**

| **Protéines** | **Tampon** | **Sel** |
|---|---|---|
| **HEWL** | 100 mM acétate de sodium pH 4,6 | 0,5 à 2 M NaCl |
| **Thaumatine** | 100 mM Bis tris propane pH 6,5 | 0,9 à 1,4 M Tartrate double de Na+ et de K+ |
| **Protéinase K** | 100 mM Cacodylate de sodium pH 6,5 | 0,9 à 1,5 M sulfate d'ammonium |

### a. Protéines non-commerciales

Les deux protéines non-commerciales (structures connues) sont la Protéase 1 de Pyrococcus horikoshii et la Glyoxylate hydroxypyuvate réductase de Pyrococcus furiosus. Ces deux protéines ont été purifiées selon les protocoles décrits dans les références ci-dessous :
- Protocole de purification de la protéine Protéase 1 de P. horikoshii : Xinlin Du et al. Crystal structure of an intracellular protéase from Pyrococcus horikoshii at 2-A résolution. 2000. Vol97. N° 26. PNAS.
- Protocole de purification de la protéine Glyoxylate réductase de P. furiosus : Thèse de Louise Lassalle, soutenue le 19 Décembre 2014 à Grenoble : Bases moléculaires de l'adaptation piézophile : études structurales et biochimiques d'enzymes clés du métabolisme provenant d'archées et de bactéries isolées dans les fonds marins. Lien : http://www.theses.fr/s98711.

Les détails techniques concernant ces protéines sont donnés dans le **Tableau 3** ci-après :

**Tableau 3 : Caractéristiques des protéines non commerciales utilisées**

| Nom | Organisme | Vecteur | Taille (Acides Aminés) | Caractéristique Physico-chimique | Lien vers séquence | Unité biologique |
|---|---|---|---|---|---|---|
| GRHPR | P. furiosus | pET41 | 336 | Hyperthermophile | 1 | dimère |
| Protéase 1 | P. horikoshii | pET41c | 166 | Thermophile | 2 | hexamère |

| | | | | | | |
|---|---|---|---|---|---|---|
| Séquence de la GRHPR : **SEQ ID N°4** Séquence de la Protéase 1 : **SEQ ID N°5** | | | | | | |

Les conditions de cristallisation de ces deux protéines sont décrites dans le **Tableau 4** ci-après. Ces conditions permettent de cristalliser les protéines sous leur forme native et sont extraites des mêmes références que les protocoles de purification.

**Tableau 4 : Conditions de cristallisation des protéines non commerciales utilisées**

| **Protéines** | **Tampon** | **Sel** | **Précipitant** |
|---|---|---|---|
| GRHPR | 100 mM acétate de sodium pH 5,2 | 100 mM NaCl | 14 à 24 % |
| | | | PEG 400 |
| Protease 1 | Tris sodium citrate dihydrate pH 5,6 | 200 mM K+ tartrate tétrahydrate | 1,9 à 2,4 M |
| | | | Sulfate d'ammonium |

Les cinq protéines décrites ci-dessus sont les protéines de référence pour caractériser les effets des complexes de lanthanide.

### c. Protéines inconnues

Les spécificités des trois protéines dont la structure est inconnue sont décrites dans le **Tableau 5** ci-après. La structure de la protéine MDH-ANC80 a été déterminée grâce au complexe de lanthanide **17.** Les différentes étapes seront décrites de manière individuelle dans un paragraphe dédié.

**Tableau 5 : Caractéristiques des protéines de structure inconnue sur lesquelles les complexes de lanthanide selon l'invention ont été testés**

| Nom | Organisme | Vecteur | Taille AA | caractéristiques | Lien vers séquence | Unité biologique |
|---|---|---|---|---|---|---|
| **pb6** | Phage T5 | pET41 | 464 | Protéine virale | 1 | monomère |
| **MDH-**ANC80 | La séquence a été générée bioinformatiquement | pET41c | 309 | Halophile | 2 | Tétramère |

| | | | | | | |
|---|---|---|---|---|---|---|
| Séquence de la Pb6 : **SEQ ID N°6** Séquence de la MDH : **SEQ ID N°7** Aucune condition de cristallisation n'était publiée concernant ces deux protéines. | | | | | | |

Les séquences SEQ IN N°1 à 7 sont présentées en ANNEXE1.

Le protocole de purification de la malate déshydrogénase (MDH) de l'ANC80 est décrit dans la littérature (Madern et al. 1995 230(3):1088-95 Eur. J. Biochem).

Le protocole de purification de pb6 a été établi par l'équipe de Cécile Breyton du groupe M&P de l'Institut de Biologie Structurale et est le suivant :

### Protocole de purification de pb6 :

Système d'expression = E. coli BL21(DE3) transformé avec LIM1 (Kan R) His tag avec site de clivage « Tobacco Etch Virus ».
   - Préculture milieu LB classique avec 50 µg/ml de kanamicyne
   - Culture en milieu classique LB : Ensemencement avec une densité optique de 0.1 et 50 µg/ml de Kanamicyne.
   - Après 6 heures de culture, centrifugation 30 minutes 4000rpm
   - Congélation des bactéries a -80 °C
   - Cassage
   - Reprise dans du tampon de lyse 50mM Tris pH 8, 150mM NaCl, 2mM MgCl2 en précence d'anti-protéase et DNase
   - Lyse des bactéries au micro-fluidizer 6x 12000psi
   - Centrifugation : 20 minutes à 14 000 rpm
   - Colonne d'affinité nickel
      Tampon d'équilibration = 20 mM Tris pH 8, 250 mM NaCl, 15 mM Imidazole
      Tampon d'élution = 20 mM Tris pH 8, 200 mM Imidazole
      Débit = 1ml/min
   - Dilution des fractions d'élutions au cinquième avec de l'eau distillée pour diminuer la conductimétrie.
   - Colonne échangeuse d'ions (HT Q 1ml)
      Tampon d'équilibration = 20 mM Tris pH 8
      Tampon d'élution = 20 mM Tris pH 8 1M NaCl
      Elution par gradient linéaire
      Débit = 1ml/min
   - Concentration sur concentrateur ayant une membrane de 30kDa
   - Dessalage par gel filtration dans un tampon tris pH 8 20 Mm

### B) Cristallisation automatisée/Stabilité des complexes de lanthanide

### a. La plateforme HTXlab.

Afin de déterminer les conditions de cristallisation d'une protéine, la plateforme HTXlab permettant de cribler un large panel de conditions de cristallisation a été utilisée. Ces conditions sont toutes décrites dans les Tableaux figurant en Annexe 2A et 2B et issus de https://embl.fr/htxlab/index.php?option=com_content&view=article&id=38&Itemid= 172.

Un criblage classique est constitué de 6 plaques de cristallisation de 96 puits chacune, soit 576 conditions.

Les études réalisées sur les protéines MDH-ANC80 et Pb6 ont utilisé les conditions décrites en Annexe 2A.

Les études réalisées sur les autres protéines ont été réalisées avec les conditions décrites en Annexe 2B, du fait d'un changement dû au fournisseur.

### Stabilité des complexes

Les études de stabilité ont été réalisées avec les conditions décrites en Annexe 2A. Il a été montré que les complexes de lanthanide basés sur le tris-dipicolinate (DPA) [Ln(DPA)₃]³⁻ présentaient une « auto-cristallisation » pour certaines conditions de cristallisation. En particulier, la présence de cations divalents, de fortes concentrations en sels, la présence de MPD provoquait l'auto-cristallisation de ce type de complexe (Thèse de doctorat de R. Talon soutenue à Grenoble le 6 juin 2012). Deux concentrations de Ln(DPA)₃³⁻ ont été évaluées (25 et 100 mM). Ainsi, sur 576 conditions, plus d'un quart des conditions conduisent à une auto-cristallisation/formation de précipités du Ln(DPA)₃³⁻ à 100 mM et de l'ordre de 8% lorsqu'il est utilisé à 25 mM.

De manière équivalente, la stabilité du complexe **10** a été évaluée à 3 concentrations différentes (10, 50 et 100 mM). Le complexe **10,** même à une concentration de 100 mM, présente une stabilité accrue. En effet, aucun cristal du complexe n'a été observé, comme détaillé ci-dessous où seule la présence de précipités a été détectée.

Récapitulatif du criblage des 6 plaques classique pour 100 mM de complexe **10** :
**Plaque Hampton 3**
   Légères précipitations observées en présence de NaHPO₄ ou KHPO₄.
**Plaque Hampton 5**
   Majoritairement des PEGs dans ce cas 100 % des gouttes sont claires.
**Plaque Hampton 4**
   Majoritairement des sels. Quelques conditions sont redondantes avec la plaque Hampton 3. Effet similaire en présence de HPO₄⁻ avec de légères précipitations observées.
**Plaque Qiagen 1**
   Mélange PEGs, sels et métaux. Un léger précipité est observé en présence d'acétate de cadmium.
**Plaque Hampton 6**
   Léger précipité observé en présence d'un mélange de métaux (cadmium, zinc, cobalt).
**Plaque Hampton 2**
   Léger précipité en présence de NaF et de (NH₄)₂PO₄

Ci-dessous le détail des conditions qui conduisent à ces précipités :
Avec 100 mM de complexe **10** ajoutés aux conditions classiques :
Plaque Hampton 4 : 21 conditions précipitées n° A7, B7, C7, A8, B8, ABCD 9, 10, 11 et 12 (majoritairement phosphate d'ammonium)
Plaque Hampton 5 : Aucun précipité
Plaque Quiagen 1 : 6 conditions précipitées n° D6, B9, F9, F9, D10, E10
Plaque Hampton 6: 4 conditions précipitées n° A3, B3, C3, H8
Plaque Hampton 2 : 7 conditions précipitées n° C2, C5, B6, A7, C7, A12, B12
Plaque Hampton 3 : 4 conditions précipitées n° H3, E4, A6, E5
Soit un total de 42 conditions présentant des précipités. Sur un criblage de 576 conditions cela équivaut à 7,3 % ;

Aucun faux positif comme avec le DPA.

Avec 10 mM de complexe **10** ajoutés aux conditions classiques:
Plaque Hampton 4 : Aucun précipité marqué comme observé avec 100 mM quelque trace de précipité pour ABCD ligne 12
Plaque Qiagen 1 : E10 une condition légèrement précipitée
Plaque wizard I et II rigaku 5 conditions précipitées n° E6, C7, B9, C10, H11
Plaque JCSG : 3 conditions légèrement précipitées n° D2 G5, A6
Plaque PACT Qiagen : 5 conditions précipitées n° E1, A5, A6, E11, C12
Plaque PEGs Qiagen : Aucune condition précipitées

Soit un total de 18 conditions présentant de légères traces de précipités. (3,2% du criblage) sachant que des gouttes avec quelques traces de précipité ont été qualifiées de « précipité ». La quantité et le nombre d'essais entraînant des précipités n'ont rien à voir avec les précipités observés à 100 mM de complexe **10,** ni à 25 mM de Ln(DPA)₃³⁻.

Il est à noter que la présence de ces précipités n'est pas incompatible avec l'apparition de cristaux de protéine. En effet, des essais de cristallisations manuelles de la protéine MDH de C. aurantiacus en présence de cadmium ont permis d'obtenir des cristaux en présence de 50 mM de complexe **10**.

### C) Criblage de nouvelles conditions de cristallisation

Pour réaliser un criblage de six plaques sur la plateforme HTXlab, 100 µl de solution de protéine sont nécessaires. Afin de disposer d'une comparaison directe, la protéine native (sans complexe de lanthanide) et la protéine en présence de 10 mM de complexe **10** ont été criblées en parallèle dans les mêmes plaques de cristallisation.

### a. Protocole de préparation des échantillons protéiques contenant le complexe de lanthanide.

Les complexes de lanthanide ont été stockés sous forme de poudre à 4°C. La masse correspondant à 10 mM pour 100 µl de solution de protéine a été pesée sur une balance de précision. La poudre a été centrifugée pour former un culot. Ce culot a été repris par 100 µl de la solution de protéine. Une centrifugation de deux minutes a été réalisée pour éliminer les éventuels agrégats. La solution a ensuite été changée de tube. Ce protocole a été appliqué pour l'ensemble des protéines envoyées au robot.

### b. Détermination de nouvelles condition pour la protéine inconnue pb6

Les conditions les plus intéressantes en terme de cristallogenèse et permettant la cristallisation de pb6 sont répertoriés dans le **tableau 6.** Les conditions ayant permis la formation de cristaux uniquement en présence de 10 mM de complexe **10** sont mentionnées en gras (il s'agit de pb6-1 et pb6-4).

**Tableau 6 : Conditions de cristallisation pour la protéine pb6 obtenues au robot HTX en absence ou en présence de complexe 10**

| **Référence condition** | **Native / complexe 10** | **Tampon** | **Sel** | **Précipitant** |
|---|---|---|---|---|
| **pb6-1** | 10 mM complexe 10 | 0,1M HEPES pH7 | 0 | 10% PEG 6K |
| pb6-2 | Native | 0,1M Acétate de sodium pH 4,6 | 0 | 8% PEG 4K |
| pb6-3 | Native | 0,1M MES pH 6 | 0 | 15% PEG 5KMME |
| **pb6-4** | 10mM complexe 10 | 0,1 M Bis tris pH 6,5 | 0,2 M acétate d'ammonium | 45% MPD |

La condition pb6-1 a été reproduite manuellement en laboratoire et a également donné des cristaux. L'utilisation des complexes selon l'invention permet de doubler le nombre de conditions ayant conduit à une cristallisation.

### c. Détermination de nouvelles condition pour la protéine inconnue MDH-ANC80

Les conditions les plus intéressantes en terme de cristallogenèse et permettant la cristallisation de la protéine ANC80 sont répertoriés dans le **tableau 7**, Les conditions ayant permis la genèse de cristaux uniquement en présence de 10 mM de complexe **10** sont mentionnées en gras (il s'agit de ANC-1 et ANC-2).

**Tableau 7 : Conditions de cristallisation pour la protéine ANC80 obtenues au robot HTX en absence ou en présence de complexe 10**

| **Référence condition** | **Native / FR39** | **Tampon** | **Sel** | **Précipitant** |
|---|---|---|---|---|
| **ANC-1** | 10 mM complexe 10 | 0,1 M MES pH 6 | 0 | 65% MPD |
| **ANC-2** | 10 mM complexe 10 | 0,1M HEPES pH 7.5 | 0,2M MgCl₂ | 30% PEG 400 |
| ANC-3 | Native | 0,1M MES pH 6.5 | 0,01M Zinc sulfate | 25% PEG 550MME |
| ANC-4 | **Native et 10 mM complexe 10** | 0,1 M MES pH 6 | Chlorure de lithium 1M | PEG 6 K 30% |

La condition ANC-1 a été reproduite au laboratoire et a permis la croissance de cristaux (Paragraphe D.a). L'utilisation des complexes selon l'invention permet de tripier le nombre de conditions ayant conduit à une cristallisation.

### d. Statistiques d'obtention des cristaux des différentes protéines étudiées sur la plateforme HTXlab

Dans le **Tableau 8** ci-après, est présenté le nombre de conditions ayant conduit à des cristaux après un criblage classique (de 576 conditions commerciales pour le complexe 10 et de 480 pour le complexe 31) sur la plateforme HTXlab des différentes protéines étudiées. Les valeurs indiquées correspondent à une observation des plaques de cristallisation après 85 jours. La colonne « conditions uniques » correspond au nombre de conditions qui entraînent une cristallisation en présence de complexe, mais aucune cristallisation dans les mêmes conditions en l'absence de complexe (dites natives).

**Tableau 8**

| Protéine Organisme Commerciale (oui/non) | Concentration en protéine (mg/ml) | Complexe en ( | Concentration en complexe (mM) | Conditions totales | Hits natifs nides* | Hits anthanides* | Conditions uniques |
|---|---|---|---|---|---|---|---|
| Lysozyme (HEWL) *Gallus* oui | 20 | 10 | 10 | 576 | 17 | 110 | 102 |
| Protéinase K *T. Album* oui | 20 | 10 | 10 | 576 | 22 | 24 | 21 |
| Pho Protéase 1 *P. Horikoshii* non | 11,5 | 10 | 10 | 576 | 82 | 113 | 47 |
| Pho Protéase 1 *P. Horikoshii* non | 11,5 | 17 | 10 | 576 | 82 | 101 | 54 |
| Glyoxylate hydroxy-pyruvate réductase *P*. *Furiosus* Non | 10 | 10 | 10 | 576 | 38 | 24 | 6 |
| Glyoxylate hydroxy-pyruvate réductase *P*. *Furiosus* Non | 10 | 17 | 10 | 576 | 38 | 15 | 5 |
| Thaumatin *T. Daniellii* oui | 20 | 10 | 10 | 576 | 4 | 5 | 2 |
| Lysozyme (HEWL) *Gallus* oui | 20 | 31 | 10 | 480 | 9 | 91 | 89 |
| Protéinase K *T. Album* oui | 20 | 31 | 10 | 480 | 16 | 4 | 4 |

| | | | | | | | |
|---|---|---|---|---|---|---|---|
| * Condition ayant conduit à cristallisation en l'absence de complexe (conditions natives) ** Condition ayant conduit à cristallisation avec ajout de complexe | | | | | | | |

Les conditions conduisant à une cristallisation correspondent à des conditions conduisant à l'apparition de cristaux potentiellement exploitables pour des expériences de diffraction. Ainsi, le complexe **10** permet l'augmentation significative du nombre de conditions de cristallisation pour les protéines suivantes : HEWL et Pho protéase1. Si son effet peut paraître moins efficace dans le cas des protéines Proteinase K et Thaumatine, il n'en est rien. En effet, l'introduction du complexe **10** ne conduit pas à une augmentation importante du nombre de hits lanthanide, mais les conditions obtenues sont en grande partie différentes des conditions natives (21 pour Proteinase K et 2 pour Thaumatine). On augmente donc le nombre de conditions potentielles permettant d'obtenir des cristaux de la protéine d'intérêt.

Il est à noter que dans le cas de la protéine Protéase1 avec le complexe **10,** l'obtention de 113 conditions couvre une large gamme de conditions de cristallisation différentes. Cela prouve à nouveau que le complexe **10** est compatible avec l'ensemble des conditions physico-chimiques que l'on peut rencontrer dans les kits de cristallisation commerciaux.

Le complexe **10,** comme les complexes **17** et **31,** possèdent tous trois un caractère nucléant. Cependant, les complexes **17** et **31** sont moins efficaces que le complexe **10**. A titre d'exemple, le complexe **31** semble moins efficace que le complexe **10** pour la protéinase K. Cependant, les 4 conditions ayant conduit à cristallisation, en présence du complexe **31,** sont différentes de celles ayant conduit à des cristaux natifs. Ces cristaux sont potentiellement de meilleure qualité. Le fait d'obtenir de nouvelles conditions conduisant à une cristallisation est donc une avancée.

### D) Cristallisations réalisées manuellement en laboratoire

Les protéines commerciales (Paragraphe A.a), ont été cristallisées manuellement selon les conditions de cristallisation usuelles connues **(Tableau 2).** Les conditions de cristallisation décrites dans le **Tableau 2** ont donc été reproduites en présence de complexes **10** ou **17** à une concentration de 10 mM. Pour réaliser ces gammes de cristallisation manuelles, les gouttes de cristallisation ont été préparées selon le schéma suivant : 1,5µl de solution de protéine + 1,5µl de solution de complexe à une concentration de 10 mM + 1,5µl de solution de précipitant. Afin de mettre en avant un potentiel effet des complexes de lanthanide sur la cristallisation, la gamme en agent précipitant a été ajustée de manière à se trouver en limite de la zone de cristallisation, puis, éventuellement étendue au-delà lorsqu'un effet était observé.

Ainsi, dans le cas de la protéine lysozyme, un effet nucléant marqué a été observé. L'effet nucléant doit ici être entendu par la croissance de cristaux en présence du complexe de lanthanide à des concentrations en précipitant faible, concentrations qui ne permettent pas la formation de cristaux natifs. Afin de mettre clairement en évidence ce phénomène de nucléation induit par les complexes selon l'invention, des diagrammes de phase ont été réalisés en déterminant les gammes de concentration en agents précipitant et en protéine qui permettent l'obtention de cristaux.

### a. Diagrammes de phase pour le lysozyme de blanc d'œuf de poule natif et en présence de 10 mM de complexe 10 ou de complexe 17

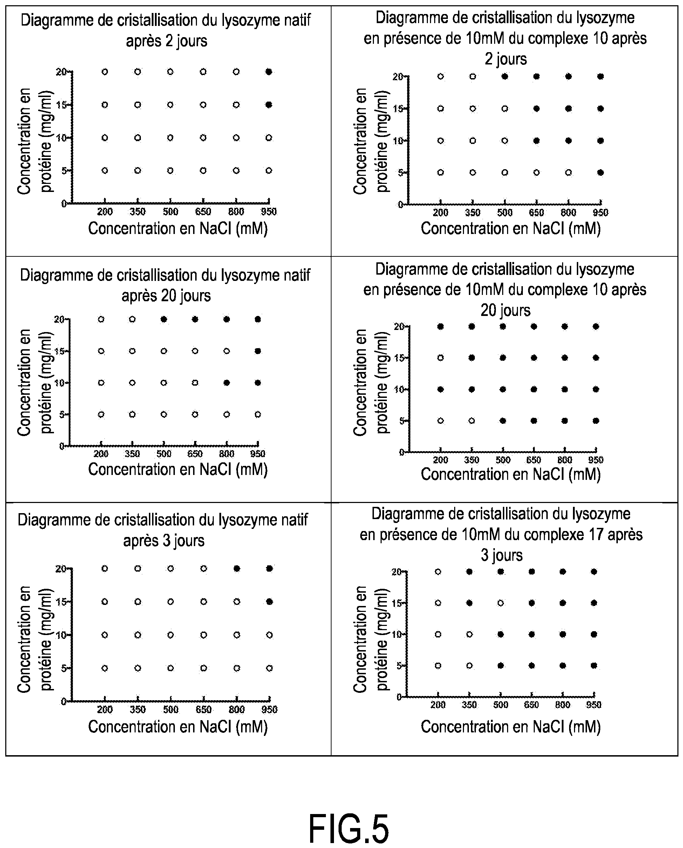

Les diagrammes de phase de la protéine lysozyme obtenus après 2 jours et 15 jours de croissance sont représentés **Figure 5** :

Chaque condition de cristallisation a été réalisée en triplicata. Après seulement deux jours de croissance cristalline, une nette différence a été observée. En présence du complexe **10** ou **17,** des cristaux ont été obtenus à de faibles concentrations en protéine (5 mg/ml) et en précipitant.

Après 20 jours de croissance cristalline en présence de complexe **10** ou **17** à 10 mM, des cristaux ont été obtenus sur l'ensemble de la gamme évaluée. En particulier, des cristaux sont apparus pour 5mg/ml de lysozyme et pour une concentration en précipitant de 500 mM. Par comparaison, l'absence de complexe de lanthanide permet seulement la formation de cristaux jusqu'à 500 mM de NaCl et pour des concentrations de 20 mg/ml de protéine.

A titre de comparaison :
Les complexes de type IRM, qui n'induisent pas d'effets nucléants, sont classiquement utilisés à des concentrations de l'ordre de 50-300 mM.
- Le tris-dipicolinate de lanthanide a produit une nouvelle forme cristalline du lysozyme seulement lorsqu'il a été utilisé à des concentrations supérieures à 50 mM.
- Les MIPs proposées par Naomi E. Chayen (Saridakis, E., Khurshid, S., Govada, L., Phan, Q., Hawkins, D., Crichlow, G. V., Lolis, E., Reddy, S. M., & Chayen, N. E. (2011). Proceedings of the National Academy of Sciences. 108, 11081-11086) conduisent à des décalages de la zone de cristallisation de seulement une à deux unités de concentration en agents précipitant. Les effets nucléants associés ne sont obtenus que pour des concentrations conventionnelles de lysozyme de l'ordre de 30 mg/ml (Khurshid et al., Automating the application of smart materials for protein crystallization, (2014) Acta cryst D). Selon Saridakis et al. (Saridakis, E., Khurshid, S., Govada, L., Phan, Q., Hawkins, D., Crichlow, G. V., Lolis, E., Reddy, S. M., & Chayen, N. E. (2011). Proceedings of the National Academy of Sciences. 108, 11081-11086.), la gamme de cristallisation du lysozyme en présence de leurs agents nucléants débute à partir de 480 mM de NaCl et ce, pour une concentration en protéine de 20 mg/mL. Aucun cristal de lysozyme n'a été observé en dessous de 460 mM NaCl. Concernant la protéine thaumatine (30 mg/ml) aucun cristal n'est observé en dessous de 0,2 M tartrate. En présence de leurs agents nucléants, ils obtiennent des cristaux pour 0,3 et 0,4 M tartrate (idem complexe **10**) et des cristaux natifs pour 0,5 M tartrate.
- Les complexes de type POM ne présentent d'effet nucléant qu'avec des concentrations élevées de lysozyme (de l'ordre de 100 mg/ml), ce qui pose des problèmes de solubilité pour la plupart des protéines. (A. Bijelic et al Hen Egg-White Lysozyme Crystallisation: Protein Stacking and Structure Stability Enhanced by a Tellurium(VI)-Centred Polyoxotungstate ChemBioChem 2015, 16, 233 - 241).

### b. Diagramme de phase pour le lysozyme de blanc d'œuf de poule natif et en présence de 10 mM de complexe 11 (Eu)

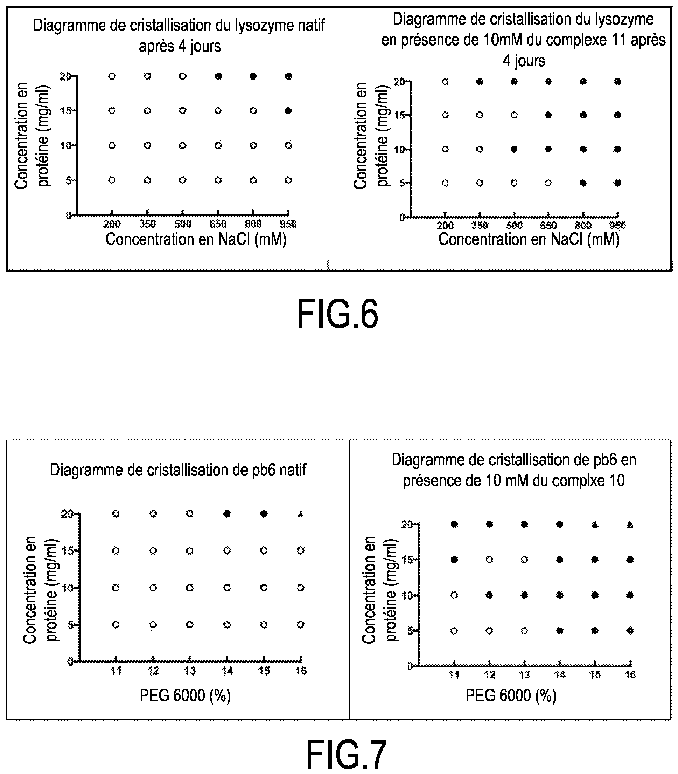

Le diagramme de phase obtenu en présence du complexe **11 (****Figure 6****)** est équivalent à celui obtenu en présence du complexe **10.** La nature du lanthanide présent au sein du complexe n'influence pas l'effet nucléant observé.

### c. Diagrammes de phase pour la protéine de structure inconnue pb6

La protéine pb6 a été purifiée selon le protocole décrit précédemment. La condition pb6-1 (Paragraphe Cb) obtenue au robot HTXlab a été reproduite manuellement. Des diagrammes de phases ont été déterminés. Ils sont représentés sur la **Figure 7****.** Comme pour le lysozyme, l'effet nucléant est très important. Les cristaux obtenus en présence du complexe de lanthanide selon l'invention sont très prometteurs (voir **Figure 8**) pour une étude cristallographique.

Les cristaux obtenus en présence de 10 mM de complexe **10** sont parfaitement exploitables pour des expériences de diffraction. Les cristaux natifs sont trop petits, trop fins et mal organisés. Ainsi dans le cas de la protéine pb6, on observe à la fois un effet nucléant et cristallisant induit par le composé **10**.

### d. Effet cristallisant des complexes de lanthanide 10

Si une amélioration de la cristallisation (nombre de cristaux, taille des cristaux, amélioration de la diffraction) est observée par l'ajout de complexe de lanthanide, on parle alors d'effet cristallisant.

Dans le cas de la cristallisation de la protéine Protease 1 de P. horikoshii un effet cristallisant a également été observé lié à l'ajout du complexe **10.** En effet, les cristaux obtenus en présence de 10 mM de complexe **10** sont apparus en moyenne 2,5 fois plus gros que les cristaux obtenus dans les mêmes conditions, mais sans complexe (taille moyenne évaluée sur 10 cristaux présents dans une goutte photographiée).

Cet effet cristallisant présente donc un intérêt certain pour la cristallographie aux rayons X puisque l'intensité diffractée est proportionnelle au volume de l'échantillon irradiée. Cela peut ainsi permettre d'obtenir une résolution supérieure pour les données de diffraction.

### E) Propriétés de luminescence et applications

Les complexes de coordinations du terbium et de l'europium (III) sont connus pour présenter des propriétés de luminescence très particulières, dues aux transitions f-f qui se traduisent par des raies d'émission fines et caractéristiques de chaque élément et des durées de vie longues (µs-ms). Il est difficile d'induire cette luminescence par irradiation directe de l'ion métallique, car ces transitions f-f sont interdites et présentent donc de très faibles coefficients d'absorption molaire. En revanche, il est possible de sensibiliser cette luminescence par un processus indirect, appelé effet d'antenne, et qui consiste à exciter un ligand organique contenant un chromophore (typiquement un groupement aromatique) et de transférer cette énergie sur l'ion métallique (Luminescence of Lanthanide Ions in Coordination Compounds and Nanomaterials, Ed. A. De Bettencourt-Dias, Wiley 2014).

Ces propriétés de luminescence des complexes de lanthanide proposés peuvent être mises à profit, lors de deux étapes de la détermination de la structure d'une protéine : a) la détection des cristaux lors des cristallisations et b) durant le centrage des cristaux lors de l'expérience de diffraction.

### a. Détection des cristaux lors des cristallisations

La détection des cristaux peut parfois être compliquée, par exemple lorsque les cristaux sont de petites tailles, sont noyés dans du précipité ou encore obtenus en bord de goutte de cristallisation. Dans le cas particulier des protéines membranaires, on peut aussi citer le problème de la détection des cristaux obtenus par la technique de cristallisation en phase cubique de lipides.

Pour améliorer la détection des cristaux, de nombreux fournisseurs proposent des microscopes avec une source UV, permettant d'utiliser la fluorescence intrinsèque des acides aminés aromatiques, en particulier du tryptophane. A titre d'exemple, on peut citer la source UV proposée par Molecular Dimension (http://www.moleculardimensions.com/applications/upload/Xtalight_100.pdf) ou le microscope UVEX (http://www.moleculardimensions.com/shopdisplayproducts.asp?id=299&cat=UVEX+ UV+Fluorescence+Imaging+systems) proposé par la même société.

L'utilisation de la luminescence des complexes de lanthanide peut contribuer à résoudre une grande partie des problèmes cités auparavant.

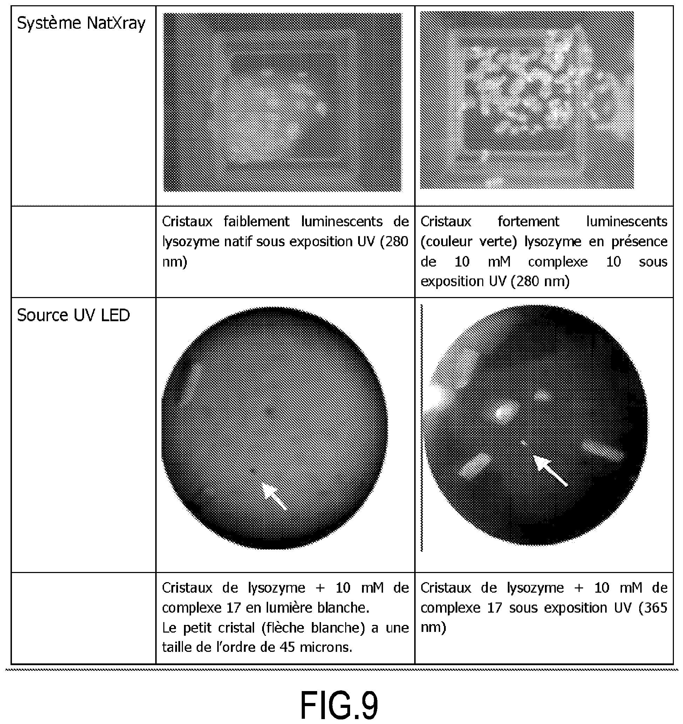

La luminescence des complexes de lanthanide selon l'invention a donc été étudiée, en utilisant une source UV en voie de commercialisation par la société NatXray sur un microscope classique et une source UV LED externe de la société OceanOptics **(****Figure 9** ; Longueur d'onde d'excitation 365 nm ; Modèle LLS-365 ; http://oceanoptics.com/product/lls-family/) (cristaux de lysozyme obtenus en présence de 10mM du complexe **10** ou cristaux de la protéine MDH ANC80 obtenus en présence de 50mM de complexe **17**).

A l'aide du système de la société NatX-ray, les cristaux obtenus en absence du complexe **10** apparaissent de couleur bleue (à gauche). A l'inverse ceux obtenus avec ce complexe apparaissent de couleur verte (à droite) qui se traduit par une augmentation du contraste entre les cristaux et la solution qui les entoure. Cela est également observable avec le système utilisant une source UV LED, puisque les cristaux, obtenus en présence du complexe **17** et sous illumination UV sont plus facilement identifiables que lorsqu'ils sont observés en lumière blanche. A titre d'exemple, un cristal de petite taille est indiqué par une flèche blanche **(****Figure 9****).**

On remarquera en outre que la luminescence est observable pour deux longueurs d'onde d'excitation (280 nm et 365 nm).

### b. Aide au centrage des cristaux

Cette partie a été évaluée à l'aide de l'instrument CRYOBENCH de l'IBS-ESRF (http://www.isbg.fr/analyses-structurales/cryobench/) et sur la ligne de lumière FIP-BM30A de l'ESRF.

Les cristaux utilisés (cristaux de lysozyme obtenus en présence de 10mM du complexe **10** ou cristaux de la protéine MDH ANC80 obtenus en présence de 50mM de complexe **17**) ont été congelés de manière classique à 100 K sur des boucles de nylon.

Pour prendre des photos, la même source UV LED externe de la société OceanOptics a été utilisée. La qualité des photos obtenues permet d'envisager différents moyens de faciliter le centrage :
- un centrage direct grâce à la luminescence,
- l'utilisation d'un spectrophotomètre pour mesurer précisément la luminescence et rechercher les zones présentant les intensités les plus fortes qui correspondraient à la présence d'un cristal. L'idée serait donc de scanner la boucle avec un faisceau UV assez fin.
- Effectuer un pré-positionnement grâce à la luminescence, complété par un centrage précis par la technique dite de Raster scanning (Aishima et al. Acta D (2010), D66, 1032-1035), notamment dans le cas des cristaux de petite taille ou de type aiguille fine.

### F) Potentiel phasant des complexes de lanthanide selon l'invention a. Méthodologie utilisée

L'évaluation du potentiel phasant des complexes de lanthanide selon l'invention a été effectuée de manière conventionnelle, en utilisant différentes méthodes de novo de détermination des phases, représentant un panel des techniques couramment utilisées pour la détermination des structures de macromolécules biologiques. Cela montre que l'utilisation des complexes selon l'invention permet une utilisation habituelle des méthodes de phasage.

Les méthodes testées sont :
- la méthode SAD, qui présente l'avantage de nécessiter un seul cristal et d'effectuer un seul enregistrement de diffraction,
- la méthode MAD, qui présente l'avantage de nécessiter un seul cristal, qui suppose un enregistrement à plusieurs longueurs d'onde et qui produit théoriquement des phases de meilleure qualité que la méthode SAD,
- la méthode SIRAS, qui suppose un enregistrement sur un cristal de la protéine en absence de complexe et un enregistrement sur un cristal de la protéine en présence de complexe. Cette méthode suppose un excellent isomorphisme des deux cristaux et donc nécessite qu'ils présentent la même forme cristalline.

Les données de diffraction ont été intégrées et mises à l'échelle avec les programmes XDS, SCALA et TRUNCATE.

Le programme AutoSharp (https://www.globalphasing.com/sharp/) a été utilisé. Ce programme effectue, de manière automatique, la recherche de la position des atomes lourds, leur affinement, la détermination initiale des phases, ainsi que leur amélioration. Le programme a été utilisé avec les paramètres par défaut. Le résultat du phasage est évalué sur la base des figures de mérite (FOM pour « Figure of Merit »), avant et après amélioration des phases.

Dans un second temps, la qualité du phasage a été évaluée en effectuant une reconstruction automatique du modèle de la protéine considérée. On dispose, alors, du nombre de résidus modélisés sur le nombre de résidus attendus. Le programme Buccaneer (http://www.ccp4.ac.uk/dist/html/cbuccaneer.html) a été utilisé avec les paramètres par défaut et avec 10 cycles de reconstruction.

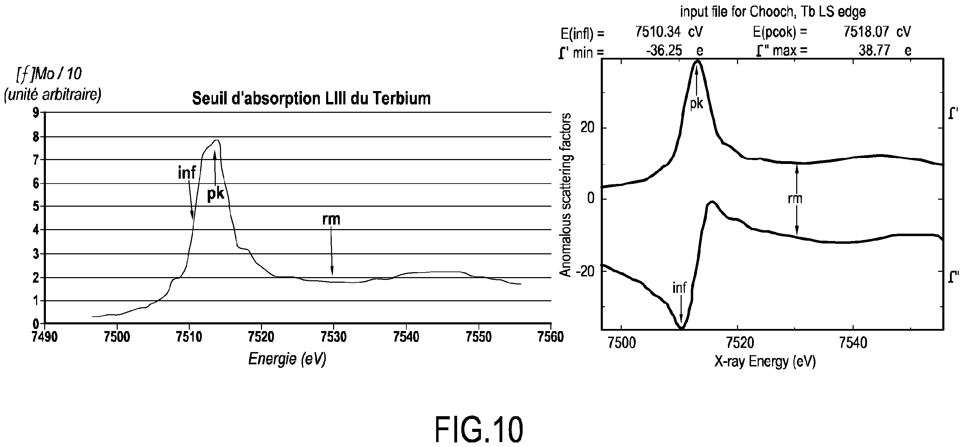

En ce qui concerne l'obtention des données de diffraction, ces dernières ont été enregistrées de manière conventionnelle sur ligne de lumière synchrotron. Afin de déterminer de manière précise le seuil d'absorption L_{III} du lanthanide utilisé, une mesure de fluorescence a été effectuée et traitée à l'aide du programme Chooch (http://www.gwyndafevans.co.uk/chooch.html). Les longueurs d'onde d'enregistrement sont ainsi obtenues, afin d'exploiter de manière optimum le signal anomal du lanthanide (méthodes SAD et MAD). Dans le cas de la méthode SIRAS et en plus de l'enregistrement au seuil L_{III} du lanthanide sur le cristal dérivé, un enregistrement sur un cristal natif a été effectué à la longueur d'onde de 0,9798 Å. Les résultats sont présentés **Figure 10**.

Pour chacune des méthodes de phasage évaluées, les enregistrements ont été effectués aux longueurs d'onde indiquées dans le **Tableau 9** :

**Tableau 9: Méthodes de phasage utilisées et énergies d'enregistrement associées.**

| Méthode de phasage | Enregistrements effectués aux énergies correspondant à : |
|---|---|
| SAD | pk |
| MAD | pk, inf, rm |
| SIRAS | pk + native à 0,9798 Å |

### b. Test de diffraction de protéines cristallisées en présence de 10 mM de complexe 10 ou 17

Les cristaux des protéines co-cristallisées en présence de 10 mM de complexe **10** ou **17** (Condition d'effet nucléant du complexe de lanthanide) ont été évalués en termes de diffraction. Les résultats de cette évaluation sont résumés dans le **Tableau 10** :

**Tableau 10 : Résultats des tests de diffraction obtenus sur différentes protéines cristallisées en présence des complexes de lanthanide**

| **Protéine** | **Résolution (Å)** |
|---|---|
| HEWL 10m Complexe **10** | >1,5 |
| Thaumatine 10mM Complexe **10** | >1,5 |
| Protéinase K 10mM Complexe **10** | >1,5 |
| Protéase1 10mM Complexe **10** | 1,7 |
| PfuGR 10mM Complexe **10** | 2,0 |
| pb6-1 10mM Complexe **10** | 2,6 |
| ANC80-1 10 mM Complexe **17** | 1,7 |

Dans le cas de la protéine Protéase 1, l'effet cristallisant se manifeste aussi bien par une augmentation de la taille moyenne des cristaux, comme indiqué précédemment, que par la résolution obtenue pour la diffraction. En effet, les données de diffraction enregistrées sur un cristal obtenu en présence de 10 mM de complexe **10** présentent une résolution de 1,7 Å. Le meilleur modèle actuellement disponible dans la Protein Data Bank est à 2,0 Å (Code PDB : 1G2I Référence publication : idem protocole partie 1b).

### c. Phasage de novo des protéines modèle

Les structures des différentes protéines modèles ont été déterminées selon les différentes méthodes explicitées dans le paragraphe F.a.

En outre, les tentatives de phasage ont été effectuées sur des cristaux obtenus en conditions nucléantes du complexe (c'est-à-dire à 10 mM), mais la possibilité d'effectuer un trempage d'un cristal obtenu en présence de 10 mM de complexe **10** ou **17** dans une solution similaire à la condition de cristallisation et contenant 100 mM de complexe de lanthanide a également été étudiée. Ceci avait pour but d'augmenter éventuellement le taux de marquage de la protéine et de faciliter ainsi la détermination de la structure. Le temps de trempage était de l'ordre de la minute. Il est à noter que cette technique de trempage peut aussi être appliquée à des cristaux natifs (obtenus en absence de complexe de lanthanide).

En résumé, les trois méthodes de préparation des cristaux suivantes ont été évaluées pour un phasage de novo en utilisant les complexes de lanthanide selon l'invention :
- cristal co-cristallisé en présence de 10 mM de complexe (condition nucléante),
- cristal co-cristallisé en présence de 10 mM de complexe et trempé dans une solution contenant 100 mM du même complexe,
- cristal natif trempé dans une solution de 100 mM du complexe de lanthanide.

L'ensemble des données de diffraction a été obtenu en accord avec la méthodologie explicitée au paragraphe F.a. Les résultats sont présentés dans le **Tableau 11** ci-après.

**Tableau 11 : Résultats des phasage de novo pour les protéines modèles**

| Protéine | Complexe | Concentration | Méthode | Résolution (Å) | FOM après SHARP | FOM après SOLO-MON | Buccanneer % de modèle reconstruit |
|---|---|---|---|---|---|---|---|
| Hewl | **10** | 10 mM | SAD | 1,8 | 0,428 | 0,881 | 79,1 |
| Hewl | **17** | 10 mM | SAD | 1,8 | 0,314 | 0,839 | 46,5 |
| Proteas e1 | **17** | Trempage * | SAD | 2,0 | 0,389 | 0,955 | 95 |
| PfuGR | **17** | Trempage*su r cristal natif | SIRAS | 2,5 | 0,043 | 0,943 | 68 |
| PfuGR | 10 | Trempage * | SAD | 2,5 | 0,184 | 0,923 | 84,5 |

| | | | | | | | |
|---|---|---|---|---|---|---|---|
| * Trempage : Dans une solution cryoprotectante contenant 100 mM de complexe | | | | | | | |

Le haut pouvoir phasant des complexes objets de la présente invention se reflète dans le pourcentage de modèle reconstruit dans intervention manuelle. Plus la détermination des phases est de qualité (ce qui dépend de la méthode de phasage utilisée, de l'occupation des sites de fixation des complexes, de la qualité des données...) plus la carte de densité électronique expérimentale sera interprétable par les programmes de reconstruction automatique (tel que le programme Buccaneer). Dans les cas évalués, des modèles reconstruits automatiquement de l'ordre de 50% à pratiquement 100% du modèle final ont été obtenus.

### d. Exemples de densité électroniques obtenues après phasage et aplatissement de solvant

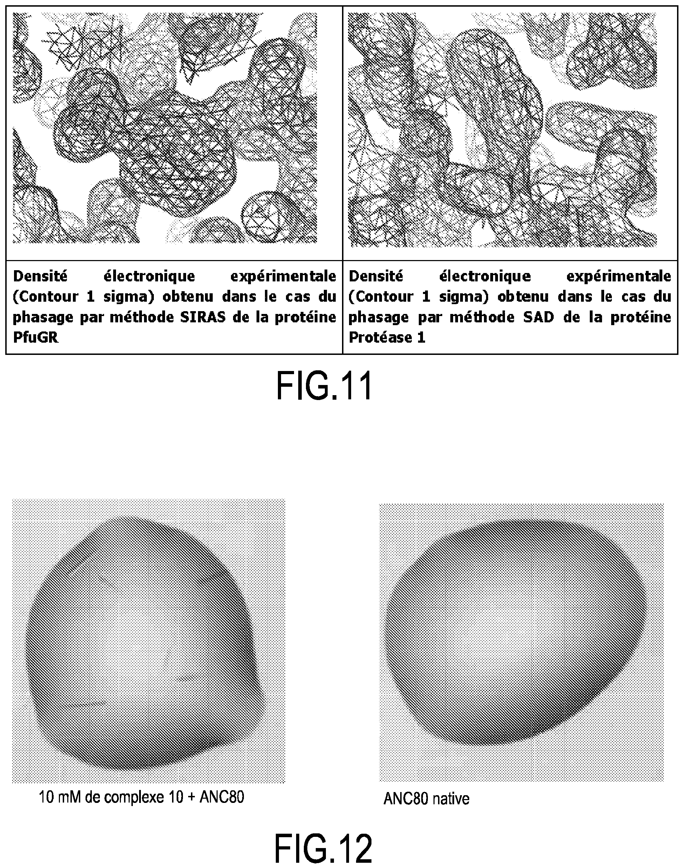

Un exemple de densité électronique expérimentale obtenue selon les méthodes décrites précédemment pour la protéine glyoxylate réductase et pour la protéine Protéase 1 sont illustrées **Figure 11****.** Les cristaux de glyoxylate réductase ont été obtenus selon les conditions décrites dans le **tableau 4** en présence de 10 mM de complexe **10.** Les cristaux de la protéine Protéase 1 ont été obtenus selon les conditions décrites dans le **tableau 4** en présence de 10 mM de complexe **10** puis trempés dans une solution cryoprotectante contenant 100 mM de complexe **10**.

Dans les deux cas, compte tenu de la qualité de la détermination des phases, on obtient des cartes de densité électronique expérimentale aisément interprétables, où l'on distingue les chaînes latérales des acides aminés. Une tyrosine pour la carte de la glyoxylate réductase et un tryptophane pour la carte de de la protéine Protéase 1. Les images ont été produites à l'aide du logiciel coot.

### G) Application de la technologie à la détermination de la structure de la protéine MDH ANC80

### a. Effet nucléant et cristallisant : le cas de la MDH ANC80

La protéine MDH de l'ANC80 concentrée à 10 mg/ml a été envoyée au robot de cristallisation pour un criblage classique des 576 conditions. La condition de cristallisation la plus prometteuse est la condition ANC-1 du **Tableau 7.** Les photos obtenues au robot de cristallisation pour cette condition sont présentées **Figure 12****.**

Cette condition a été reproduite manuellement au laboratoire. Des cristaux sont apparus en présence de 10 mM de complexe **17** et 10 mM de complexe **10** en 7 jours. La même condition en condition native (sans complexe de lanthanide) a également été réalisée. Après environ 3 semaines, des cristaux de formes différentes sont apparus (non représentés).

Les cristaux natifs ont été testés en diffraction. Celle-ci présente une résolution de l'ordre de 2,5 Â. Ces cristaux présentent une symétrie différente de celle obtenue pour les cristaux obtenus en présence de complexe **10** ou **17** (Groupe d'espace F222) avec des paramètres de maille de l'ordre de 81, 140 et 395 Â respectivement pour a, b, et c. Ceci est à comparer au groupe d'espace obtenu pour les cristaux en présence de complexe et à la résolution obtenue pour les données de diffraction. En effet, les cristaux en présence de 10 mM de complexe de lanthanide diffractent à 1,7 Angström. Ainsi, les effets nucléants et cristallisants sont bien observés dans le cas de cette protéine.

La détermination de la structure de la MDH de l'ANC80 a été réalisée selon la méthode MAD. Trois jeux de données ont été enregistrés sur le même cristal au seuil d'absorption L_{III} du terbium. Un jeu de données supplémentaire a été mesuré au seuil d'absorption K du sélénium pour obtenir la meilleure résolution possible. Les statistiques, après intégration sur l'ensemble des données collectées, sont présentées dans le **Tableau 12** ci-après.

**Tableau 12 Collecte des données, phasage et statistiques d'affinements**

| | ANC80 | | ANC80 | |
|---|---|---|---|---|
| | 10mM complexe **17** | | 10mM complexe **17** | |
| **Collection des données** | Seuil K du Se Ligne id23-2 (ESRF) | | Seuil LIII Tb Ligne BM30A (ESRF) | |
| Groupe d'espace | R3 | | R3 | |
| Paramètres de maille | | | | |
| a, b, c (Å) | a = b = 217,5 | | a = b = 217,5 | |
| | c = 86,3 | | c = 86,3 | |
| | | **pk*** | **inf** | **rm** |
| Longueur d'onde | 0,8726 | 1,650237 | 1,65087 | 1,64766 |
| Résolution (Å) | 1,85 | 2 | 2 | 2 |
| R_{merge} | 0,093 (0,850) | 0,126 (0,617) | 0,117 (1,035) | 0,116 (1,111) |
| I / sigma(I) | 7,3 (1,3) | 6,8 (2,1) | 7 (1,5) | 7,6 (1,3) |
| Complétude (%) | 98,9 (97,6) | 99,5 (96,8) | 99,6 (97,0) | 99,5 (96,5) |
| Multiplicité | 3,9 (3,9) | 5,5 (5,2) | 5,5 (5,1) | 5,5 (5,0) |
| **Reconstruction automatique (Buccaneer)** | | 99,2 % du modèle reconstruit à 2,0 Angström | | |

| | | | | |
|---|---|---|---|---|
| * selon la **Figure 8** | | | | |

L'affinement du modèle conduit à des facteurs de qualité très bons avec un facteur R et Rfree de 19,2 et 21,2 %.

Les complexes luminescents suivants ont été également testés à 10 mM et ne présentent pas d'effet sur la cristallisation :

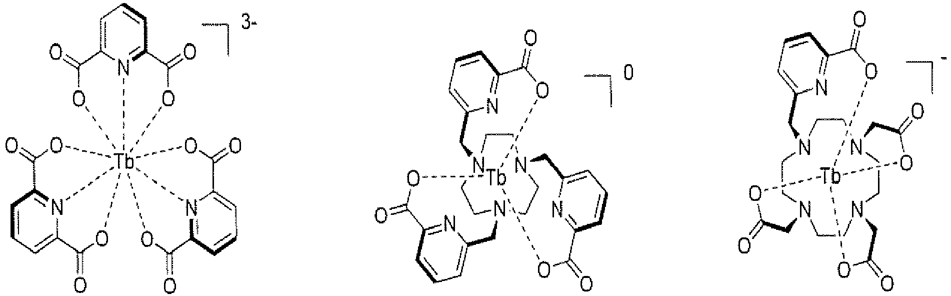

### ANNEXE 1 : Séquence en acides aminés des différentes protéines testées

**Lysozyme (Gallus gallus) HEWL : SEQ ID N°1**
**Thaumatine (T. danielli) : SEQ ID N°2** 
ATFEIVNRCSYTVWAAASKGDAALDAGGRQLNSGESWTINVEPGTNGGKIWARTDCYFDDS
GSGICKTGDCGGLLRCKRFGRPPTTLAEFSLNQYGKDYIDISNIKGFNVPMNFSPTTRGCRGVR
CAADIVGQCPAKLKAPGGGCNDACTVFQTSEYCCTTGKCGPTEYSRFFKRLCPDAFSYVLDKP
TTVTCPGSSNYRVTFCPTA
**Protéinase K (Tritirachium Album) : SEQ ID N°3** 
AAQTNAPWGLARISSTSPGTSTYYYDESAGQGSCVYVIDTGIEASHPEFEGRAQMVKTYYYSS
RDGNGHGTHCAGTVGSRTYGVAKKTQLFGVKVLDDNGSGQYSTIIAGMDFVASDKNNRNCP
KGVVASLSLGGGYSSSVNSAAARLQSSGVMVAVAAGNNNADARNYSPASEPSVCTVGASDRY
DRRSSFSNYGSVLDIFGPGTDILSTWIGGSTRSISGTSMATPHVAGLAAYLMTLGKTTAASACR
YIADTANKGDLSNIPFGTVNLLAYNNYQA
**Glyoxylate hydroxtypyruvate réductase (P. furiosus) : SEQ ID N°4** 
MKPKVFITRAIPENGINMLEEEFEVEVWEEEREIPREKLLEKVKDVDALVTMLSERIDQEVFENA
PRLRIVANYAVGYDNIDVEEATRRGIYVTNTPDVLTNATADHAFALLLATARHVVKGDKFVRS
GEWKRKGIAWHPKWFLGYELYGKTIGIVGFGRIGQAIARRAKGFNMRILYYSRTRKSQAEKEL
GAEYRPLEEVLKESDFVILAVPLTKETMYMINEERLKLMKPTAILVNIARGKVVDTKALIKALKE
GWIAGAGLDVFEEEPYYNEELFSLDNVVLTPHIGSATFEAREAMAELVARNLIAFKRGEIPPTLV
NKEVIKIRKPGFNEQ
**Protéase 1 (P. horikoshii OT3) : SEQ ID N°5** 
MKVLFLTANEFEDVELIYPYHRLKEEGHEVYIASFERGTITGKHGYSVKVDLTFDKVNPEEFDAL
VLPGGRAPERVRLNEKAVSIARKMFSEGKPVASICHGPQILISAGVLRGRKGTSYPGIKDDMIN
AGVEWVDAEVVVDGNWVSSRVPADLYAWMREFVKLLK
**Pb6 protéine majoritaire de la queue du phage T5 : SEQ ID N°6** 
MSLQLLRNTRIFVSTVKTGHNKTNTQEILVQDDISWGQDSNSTDITVNEAGPRPTRGSKRFN
DSLNAAEWSFSTYILPYKDKNTSKQIVPDYMLWHALSSGRAINLEGTTGAHNNATNFMVNFK
DNSYHELAMLHIYILTDKTWSYIDSCQINQAEVNVDIEDIGRVTWSGNGNQLIPLDEQPFDPD
QIGIDDETYMTIQGSYIKNKLTILKIKDMDTNKSYDIPITGGTFTINNNITYLTPNVMSRVTIPIG
 
SFTGAFELTGSLTAYLNDKSLGSMELYKDLIKTLKVVNRFEIALVLGGEYDDERPAAILVAKQAH
VNIPTIETDDVLGTSVEFKAIPSDLDAGDEGYLGFSSKYTRTTINNLIVNGDGATDAVTAITVKS
AGNVTTLNRSATLQMSVEVTPSSARNKEVTWAITAGDAATINATGLLRADASKTGAVTVEATA
KDGSGVKGTKVITVTAGG
**ANC80 Malate Déhydrogénase (protéine synthétique) : SEQ ID N°7**

MTKVSVVGAAGTVGAAAGYNLALRDIADELVFVDIPDQEDVTIGQAADTNHGVAYDSNTTVR
QGGYEDTAGSDVVVITAGIPRQPGQTRIDLAGDNAPIMEDIGSSLAEHNDDFVTITTSNPVDL
LNRHLYETGDRAREKVIGFGGRLDSARFRYVLSQRFDAPVQNVEATILGEHGDAQVPVFSKVR
VDGTDPEFSADEKEEILGDLQESAMDVIERKGATQWGPATGVAHMVEAVLHDTGEVLPGSVV
LDGEFGHEDTAFGVPVKLGSNGVEEVVEWDLDDYEQDLMDDAAEKLSDQYDKIA

| ANNEXE 2A | | | | | | | | | | | | | |
|---|---|---|---|---|---|---|---|---|---|---|---|---|---|
| Annexe plaque « Quiagen » | | | | | | | | | | | | | |
| Puits | C | U | Sel | C | U | Tampon pH | | C | U | Précipitant | C | U | Additif |
| B09 | 0,2 | M | phosphate d'ammonium | 0,1 | M | TRIS | 8,5 | 50 | %(v/v) | MPD | | | |
| B05 | 0,2 | M | acétate d'ammonium | 0,1 | M | citrate trisodique | 5,6 | 30 | %(v/v) | MPD | | | |
| B08 | 0,5 | M | sulfate d'ammonium | 0,1 | M | HEPES | 7,5 | 30 | %(v/v) | MPD | | | |
| B06 | 0,2 | M | acétate de magnésium | 0,1 | M | cacodylate de sodium | 6,5 | 30 | %(v/v) | MPD | | | |
| G08 | | | | | | | | 10 | %(w/v) | PEG 1000 | 10 | %(w/v) | PEG 8000 |
| H11 | | | | 0,1 | M | HEPES | 7,5 | 20 | %(w/v) | PEG 10000 | 8 | %(v/v) | éthylène glycol |
| G09 | | | | | | | | 30 | %(w/v) | PEG 1500 | | | |
| G11 | 0,2 | M | sulfate d'ammonium | 0,1 | M | acétate de sodium | 4,6 | 30 | %(w/v) | PEG 2000 MME | | | |
| G10 | 0,01 | M | chlorure de nickel | 0,1 | M | TRIS | 8,5 | 20 | %(w/v) | PEG 2000 MME | | | |
| H12 | | | | 0,1 | M | MES | 6,5 | 12 | %(w/v) | PEG 20000 | | | |
| G02 | 0,2 | M | chlorure de calcium | 0,1 | M | Sel de sodium d'HEPES | 7,5 | 28 | %(v/v) | PEG 400 | | | |
| G04 | 0,2 | M | chlorure de magnesium | 0,1 | M | Sel de sodium d'HEPES | 7,5 | 30 | %(v/v) | PEG 400 | | | |
| G05 | 0,2 | M | citrate trisodique | 0,1 | M | TRIS HCl | 8,5 | 30 | %(v/v) | PEG 400 | | | |
| G01 | 2 | %(v/v) | PEG 400 | 0,1 | M | Sel de sodium d'HEPES | 7,5 | 2 | M | Sulfate d'ammonium | | | |
| G03 | 0,1 | M | chlorure de cadmium | 0,1 | M | acétate de sodium | 4,6 | 30 | %(v/v) | PEG 400 | | | |
| H02 | 0,2 | M | acétate d'ammonium | 0,1 | M | acétate de sodium | 4,6 | 30 | %(w/v) | PEG 4000 | | | |
| H01 | 0,2 | M | sulfate d'ammonium | 0,1 | M | acétate de sodium | 4,6 | 25 | %(w/v) | PEG 4000 | | | |
| H05 | 0,2 | M | sulfate de lithium | 0,1 | M | TRIS HCl | 8,5 | 30 | %(w/v) | PEG 4000 | | | |
| H06 | 0,2 | M | acétate de sodium | 0,1 | M | TRIS HCl | 8,5 | 30 | %(w/v) | PEG 4000 | | | |
| H03 | 0,2 | M | acétate d'ammonium | 0,1 | M | citrate trisodique | 5,6 | 30 | %(w/v) | PEG 4000 | | | |
| H07 | 0,2 | M | sulfate d'ammonium | | | | | 30 | %(w/v) | PEG 4000 | | | |
| G12 | | | | 0,1 | M | acétate de sodium | 4,6 | 8 | %(w/v) | PEG 4000 | | | |
| H04 | 0,2 | M | chlorure de magnesium | 0,1 | M | TRIS HCl | 8,5 | 30 | %(w/v) | PEG 4000 | | | |
| H08 | 0,2 | M | sulfate d'ammonium | 0,1 | M | MES | 6,5 | 30 | %(w/v) | PEG 5000 MME | | | |
| G06 | 0,1 | M | chlorure de sodium | 0,1 | M | bicine | 9 | 20 | %(w/v) | PEG 550 MME | | | |
| G07 | 0,01 | M | sulfate de zinc | 0,1 | M | MES | 6,5 | 25 | %(w/v) | PEG 550 MME | | | |
| H09 | | | | 0,1 | M | HEPES | 7,5 | 10 | %(w/v) | PEG 6000 | 5 | %(v/v) | MPD |
| H10 | 10 | %(w/v) | PEG 6000 | | | | | 2 | M | chlorure de sodium | | | |
| F04 | | | | 0,1 | M | HEPES | 7,5 | 10 | %(w/v) | PEG 8000 | | | |
| F06 | 0,2 | M | acétate de zinc | 0,1 | M | cacodylate de sodium | 6,5 | 18 | %(w/v) | PEG 8000 | | | |
| F10 | 0,2 | M | sulfate d'ammonium | 0,1 | M | cacodylate de sodium | 6,5 | 30 | %(w/v) | PEG 8000 | | | |
| F07 | 0,2 | M | acétate de calcium | 0,1 | M | cacodylate de sodium | 6,5 | 18 | %(w/v) | PEG 8000 | | | |
| F08 | 0,2 | M | acétate de magnésium | 0,1 | M | cacodylate de sodium | 6,5 | 20 | %(w/v) | PEG 8000 | | | |
| F11 | 0,2 | M | acétate de sodium | 0,1 | M | cacodylate de sodium | 6,5 | 30 | %(w/v) | PEG 8000 | | | |
| F12 | 0,2 | M | sulfate d'ammonium | | | | | 30 | %(w/v) | PEG 8000 | | | |
| F05 | 0,5 | M | sulfate de lithium | | | | | 15 | %(w/v) | PEG 8000 | | | |
| F09 | 0,05 | M | phosphate de potassium | | | | | 20 | %(w/v) | PEG 8000 | | | |
| F03 | | | | 0,1 | M | TRIS HCl | 8,5 | 8 | %(w/v) | PEG 8000 | | | |
| D03 | 0,05 | M | sulfate de cadmium | 0,1 | M | HEPES | 7,5 | 1 | M | acétate de sodium | | | |
| D02 | | | | 0,1 | M | imidazole | 6,5 | 1 | M | acétate de sodium | | | |
| D04 | | | | 0,1 | M | cacodylate de sodium | 6,5 | 1,4 | M | acétate de sodium | | | |
| D07 | | | | 0,1 | M | HEPES | 7,5 | 4,3 | M | chlorure de sodium | | | |
| D06 | 0,1 | M | phosphate de sodium | 0,1 | M | MES | 6,5 | 2 | M | chlorure de sodium | 0,1 | M | phosphate de potassium |
| D05 | | | | 0,1 | M | acétate de sodium | 4,6 | 2 | M | chlorure de sodium | | | |
| D11 | | | | 0,1 | M | acétate de sodium | 4,6 | 2 | M | formiate de sodium | | | |
| D12 | | | | | | | | 4 | M | formiate de sodium | | | |
| D10 | | | | 0,1 | M | Sel de sodium d'HEPES | 7,5 | 0,8 | M | phosphate de sodium | 0,8 | M | phosphate de potassium |
| B11 | | | | 0,1 | M | TRIS | 8,5 | 25 | %(v/v) | tert-butanol | | | |
| B12 | | | | 0,1 | M | citrate trisodique | 5,6 | 35 | %(v/v) | tert-butanol | | | |
| D08 | | | | 0,1 | M | Sel de sodium d'HEPES | 7,5 | 1,4 | M | citrate trisodique | | | |
| D09 | | | | | | | | 1,6 | M | citrate trisodiquepH 6.5 | | | |
| Puits | C | U | Sel | C | U | Tampon | pH | C | U | Précipitant | C | U | Additif |
| A03 | 0,2 | M | chlorure de maqnesium | 0,1 | M | TRIS | 8,5 | 3,4 | M | 1.6-hexanediol | | | |
| A02 | | | | 0,1 | M | citrate trisodique | 5,6 | 2,5 | M | 1.6-hexanediol | | | |
| A01 | 0,01 | M | chlorure de cobalt | 0,1 | M | acétate de sodium | 4,6 | 1 | M | 1.6-hexanediol | | | |
| C04 | | | | 0,1 | M | HEPES | 7,5 | 2 | M | ammonium formate | | | |
| C03 | | | | 0,1 | M | TRIS HCl | 8,5 | 2 | M | phosphate d'ammonium | | | |
| C02 | | | | 0,1 | M | citrate trisodique | 5,6 | 1 | M | phosphate d'ammonium | | | |
| C01 | | | | | | | | 0,4 | M | phosphate d'ammonium | | | |
| C08 | 0,1 | M | chlorure de sodium | 0,1 | M | HEPES | 7,5 | 1,6 | M | sulfate d'ammonium | | | |
| C09 | 0,01 | M | chlorure de cobalt | 0,1 | M | MES | 6,5 | 1,8 | M | sulfate d'ammonium | | | |
| C05 | | | | 0,1 | M | acétate de sodium | 4,6 | 2 | M | sulfate d'ammonium | | | |
| C06 | | | | 0,1 | M | TRIS HCl | 8,5 | 2 | M | sulfate d'ammonium | | | |
| C10 | 0,2 | M | tartrate de sodium /potassium | 0,1 | M | citrate trisodique | 5,6 | 2 | M | sulfate d'ammonium | | | |
| C07 | | | | | | | | 2 | M | sulfate d'ammonium | | | |
| E06 | 0,5 | M | chlorure de sodium | | | | | 0,01 | M | CTAB | 0,01 | M | chlorure de magnesium |
| E02 | 10 | %(v/v) | dioxane | 0,1 | M | MES | 6,5 | 1,6 | M | sulfate d'ammonium | | | |
| E03 | | | | | | | | 35 | %(v/v) | dioxane | | | |
| E01 | 2 | %(v/v) | dioxane | 0,1 | M | bicine | 9 | 10 | %(w/v) | PEG 20000 | | | |
| A12 | 10 | %(v/v) | éthanol | | | | | 1,5 | M | chlorure de sodium | | | |
| B01 | | | | 0,1 | M | TRIS | 8,5 | 20 | %(v/v) | éthanol | | | |
| B02 | | | | | | | | 25 | %(v/v) | éthylène glycol | | | |
| E04 | 0,5 | M | chlorure de sodium | 0,1 | M | citrate trisodique | 5,6 | 2 | %(v/v) | ethylene imine polymer | | | |
| E05 | 12 | %(v/v) | glycérol | 0,1 | M | TRIS | 8,5 | 1,5 | M | sulfate d'ammonium | | | |
| C11 | | | | | | | | 1 | M | imidazole pH 7.0 | | | |
| A10 | 0,2 | M | chlorure de maqnesium | 0,1 | M | Sel de sodium d'HEPES | 7,5 | 30 | %(v/v) | isopropanol | | | |
| A08 | 0,2 | M | citrate trisodique | 0,1 | M | Sel de sodium d'HEPES | 7,5 | 20 | %(v/v) | isopropanol | | | |
| A05 | 10 | %(v/v) | isopropanol | 0,1 | M | Sel de sodium d'HEPES | 7,5 | 20 | %(w/v) | PEG 4000 | | | |
| A06 | 0,2 | M | chlorure de calcium | 0,1 | M | acétate de sodium | 4,6 | 20 | %(v/v) | isopropanol | | | |
| A09 | 0,2 | M | citrate trisodique | 0,1 | M | cacodylate de sodium | 6,5 | 30 | %(v/v) | isopropanol | | | |
| A11 | 0,2 | M | acétate d'ammonium | 0,1 | M | TRIS HCl | 8,5 | 30 | %(v/v) | isopropanol | | | |
| A07 | 20 | %(v/v) | isopropanol | 0,1 | M | citrate trisodique | 5,6 | 20 | %(w/v) | PEG 4000 | | | |
| A04 | 5 | %(v/v) | isopropanol | | | | | 2 | M | sulfate d'ammonium | | | |
| E08 | | | | 0,1 | M | HEPES | 7,5 | 20 | %(v/v) | jeffamine M-600 | | | |
| E07 | 0,01 | M | chlorure ferrique | 0,1 | M | citrate trisodique | 5,6 | 10 | % (v/v) | jeffamine M-600 | | | |
| D01 | | | | 0,1 | M | Sel de sodium d'HEPES | 7,5 | 0,8 | M | tartrate de potassium / sodium | | | |
| C12 | | | | | | | | 0,4 | M | tartrate de potassium / sodium | | | |
| E11 | | | | 0,1 | M | Sel de sodium d'HEPES | 7,5 | 1,5 | M | sulfate de lithium | | | |
| E10 | 0,01 | M | chlorure de nickel | 0,1 | M | TRIS | 8,5 | 1 | M | sulfate de lithium | | | |
| E09 | 0,5 | M | sulfate d'ammonium | 0,1 | M | citrate trisodique | 5,6 | 1 | M | sulfate de lithium | | | |
| E12 | | | | 0,1 | M | bicine | 9 | 2 | M | chlorure de magnesium | | | |
| F01 | | | | | | | | 0,2 | M | formiate de magnésium | | | |
| F02 | | | | 0,1 | M | MES | 6,5 | 1,6 | M | sulfate de magnesium | | | |
| B10 | | | | 0,1 | M | HEPES | 7,5 | 70 | %(v/v) | MPD | | | |
| B07 | 0,2 | M | citrate trisodique | 0,1 | M | Sel de sodium d'HEPES | 7,5 | 30 | %(v/v) | MPD | | | |
| B03 | 0,02 | M | chlorure de calcium | 0,1 | M | acétate de sodium | 4,6 | 30 | %(v/v) | MPD | | | |
| B04 | 0,2 | M | chlorure de sodium | 0,1 | M | acétate de sodium | 4,6 | 30 | %(v/v) | MPD | | | |

| Annexe plaque « Hampton 2 » | | | | | | | | | | | | | |
|---|---|---|---|---|---|---|---|---|---|---|---|---|---|
| Puits | C | U | Sel | C | U | Tampon | pH | C | U | Précipitant | C | U | Additif |
| A01 | 0,02 | M | chlorure de calcium dihydraté | 0,1 | M | acétate de sodium trihydraté | 4,6 | 15 | %(v/v) | MPD | | | |
| A02 | 0,2 | M | acétate d'ammonium | 0,1 | M | citrate trisodique dihydraté | 5,6 | 15 | %(w/v) | PEG 4000 | | | |
| A03 | 0,2 | M | lithium monohydrate de sulfate | 0,1 | M | TRIS HCl | 8,5 | 15 | %(w/v) | PEG 4000 | | | |
| A04 | | | | 0,1 | M | imidazole | 6,5 | 0,5 | M | acétate de sodium trihydraté | | | |
| A05 | | | | | | | | 2 | M | formiate de sodium | | | |
| A06 | 5 | %(v/v) | iso-propanol | 0,1 | M | Sel de sodium d'HEPES | 7,5 | 10 | %(w/v) | PEG 4000 | | | |
| A07 | 0,2 | M | fluorure de sodium | | | | 7,1 | 20 | %(w/v) | PEG 3350 | | | |
| A08 | 0,2 | M | chlorure d'ammonium | | | | 6,3 | 20 | %(w/v) | PEG 3350 | | | |
| A09 | 0,2 | M | nitrate de sodium | | | | 6,8 | 20 | %(w/v) | PEG 3350 | | | |
| A10 | 0,2 | M | tétrahydrate d'acétate de magnésium | | | | 7,7 | 20 | %(w/v) | PEG 3350 | | | |
| A11 | 0,2 | M | sulfate de sodium décahydraté | | | | 6,6 | 20 | %(w/v) | PEG 3350 | | | |
| A12 | 0,2 | M | dihydrogénophosphate de potassium | | | | 4,7 | 20 | %(w/v) | PEG 3350 | | | |
| BÛ1 | | | | | | | | 0,2 | M | potassium / sodium tétrahydrate de tartrate | | | |
| B02 | 0,2 | M | acétate d'ammonium | 0,1 | M | acétate de sodium trihydraté | 4,6 | 15 | %(w/v) | PEG 4000 | | | |
| B03 | 0,2 | M | tétrahydrate d'acétate de magnésium | 0,1 | M | cacodylate de sodium | 6,5 | 10 | %(w/v) | PEG 8000 | | | |
| B04 | 0,2 | M | acétate d'ammonium | 0,1 | M | citrate trisodique dihydraté | 5,6 | 15 | %(v/v) | MPD | | | |
| B05 | | | | 0,1 | M | acétate de sodium trihydraté | 4,6 | 1 | M | formiate de sodium | | | |
| B06 | 0,05 | M | dihydrogénophosphate de potassium | | | | | 10 | %(w/v) | PEG 8000 | | | |
| B07 | 0,2 | M | fluorure de potassium | | | | 7,2 | 20 | %(w/v) | PEG 3350 | | | |
| B08 | 0,2 | M | iodure de sodium | | | | 6,9 | 20 | %(w/v) | PEG 3350 | | | |
| B09 | 0,2 | M | nitrate de potassium | | | | 6,9 | 20 | %(w/v) | PEG 3350 | | | |
| B10 | 0,2 | M | acétate de zinc dihydraté | | | | 6,3 | 20 | %(w/v) | PEG 3350 | | | |
| B11 | 0,2 | M | sulfate de potassium | | | | 6,7 | 20 | %(w/v) | PEG 3350 | | | |
| B12 | 0,2 | M | di-potassium hydrogénophosphate | | | | 9,2 | 20 | %(w/v) | PEG 3350 | | | |
| C01 | | | | | | | | 0,2 | M | dihydrogénophosphate d'ammonium | | | |
| C02 | | | | 0,1 | M | citrate trisodique dihydraté | 5,6 | 0,5 | M | dihydrogénophosphate d'ammonium | | | |
| C03 | 0,21 | M | acétate d'ammonium | 0,1 | M | TRIS HCl | 8,5 | 15 | %(v/v) | iso-propanol | | | |

| Annexe plaque « Hampton 2 | | | | | | | | | | | | | |
|---|---|---|---|---|---|---|---|---|---|---|---|---|---|
| C04 | 0,2 | M | citrate trisodique dihydraté | 0,1 | | Sel de sodium d'HEPES | 7,5 | 10 | %(v/v) | iso-propanol | | | |
| C05 | | | | 0,1 | M | Sel de sodium d'HEPES | 7,5 | 0,4 | M | dihydrogénophosphate de sodium | 0,4 | M | dihydrogénophosphate de potassium |
| C06 | | | | | | | | 15 | %(w/v) | PEG 1500 | | | |
| C07 | 0,2 | M | fluorure d'ammonium | | | | 6,2 | 20 | %(w/v) | PEG 3350 | | | |
| C08 | 0,2 | M | Iodure de potassium | | | | 6,8 | 20 | %(w/v) | PEG 3350 | | | |
| C09 | 0,2 | M | nitrate d'ammonium | | | | 6,3 | 20 | %(w/v) | PEG 3350 | | | |
| C10 | 0,2 | M | acétate de sodium trihydraté | | | | 7,9 | 20 | %(w/v) | PEG 3350 | | | |
| C11 | 0,2 | M | sulfate d'ammonium | | | | 6 | 20 | %(w/v) | PEG 3350 | | | |
| C12 | 0,2 | M | dihydrogénophosphate d'ammonium | | | | 4,6 | 20 | %(w/v) | PEG 3350 | | | |
| D01 | | | | 0,1 | M | TRIS HCl | 8,5 | 1 | M | sulfate d'ammonium | | | |
| D02 | 0,2 | M | chlorure de magnésium hexahydraté | 0,1 | M | Sel de sodium d'HEPES | 7,5 | 15 | %(v/v) | iso-propanol | | | |
| D03 | 0,2 | M | sulfate d'ammonium | 0,1 | M | acétate de sodium trihydraté | 4,6 | 12,5 | %(w/v) | PEG 4000 | | | |
| D04 | 0,2 | M | acétate de sodium trihydraté | 0,1 | M | cacodylate de sodium | 6,5 | 15 | %(w/v) | PEG 8000 | | | |
| D05 | | | | 0,1 | M | TRIS HCl | 8,5 | 4 | %(w/v) | PEG 8000 | | | |
| D06 | | | | | | | | 0,1 | M | formiate de magnésium | | | |
| D07 | 0,2 | M | chlorure de lithium anhydre | | | | 6,7 | 20 | %(w/v) | PEG 3350 | | | |
| D08 | 0,21 | M | iodure d'ammonium | | | | 6,2 | 20 | %(w/v) | PEG 3350 | | | |
| D09 | 0,2 | M | formiate de magnésium | | | | 5,9 | 20 | %(w/v) | PEG 3350 | | | |

| Annexe plaque « Hampton 2 » | | | | | | | | | | | | | |
|---|---|---|---|---|---|---|---|---|---|---|---|---|---|
| Puits | C | U | Sel | C | U | Tampon | pH | C | U | Précipitant | C | U | Additif |
| D10 | 0,2 | M | acétate de calcium hydraté | | | | 7,3 | 20 | %(w/v) | PEG 3350 | | | |
| D11 | 0,2 | M | di-sodium tartate dihydrate | | | | 7,2 | 20 | %(w/v) | PEG 3350 | | | |
| D12 | 0, | 2 M | di-ammonium hydrogen phosphate | | | | 7,9 | 20 | %(w/v) | PEG 3350 | | | |
| E01 | 0,2 | M | citrate trisodique dihydraté | 0,1 | M | Sel de sodium d'HEPES | 7,5 | 15 | %(v/v) | MPD | | | |
| E02 | 0,2 | M | citrate trisodique dihydraté | 0,1 | M | TRIS HCl | 8,5 | 15 | %(v/v) | PEG 400 | | | |
| E03 | 0,2 | M | tétrahydrate d'acétate de magnésium | 0,1 | M | cacodylate de sodium | 6,5 | 15 | %(v/v) | MPD | | | |
| E04 | | | | 0,1 | M | Sel de sodium d'HEPES | 7,5 | 0,4 | M | potassium / sodium tétrahydrate de tartrate | | | |
| E05 | | | | 0,1 | M | acétate de sodium trihydraté | 4,6 | 4 | %(w/v) | PEG 4000 | | | |
| E06 | 0,2 | M | acétate de zinc dihydraté | 0,1 | M | cacodylate de sodium | 6,5 | 9 | %(w/v) | PEG 8000 | | | |
| E07 | 0,2 | M | chlorure de magnésium hexahydraté | | | | 5,8 | 20 | %(w/v) | PEG 3350 | | | |
| E08 | 0,2 | M | thiocyanate de sodium | | | | 6,9 | 20 | %(w/v) | PEG 3350 | | | |
| E09 | 0,2 | M | formiate de sodium | | | | 7,2 | 20 | %(w/v) | PEG 3350 | | | |
| E10 | 0,2 | M | acétate de potassium | | | | 7,8 | 20 | %(w/v) | PEG 3350 | | | |
| E11 | 0,2 | M | potassium tartrate de sodium tétrahydraté | | | | 7,2 | 20 | %(w/v) | PEG 3350 | | | |
| E12 | 0,2 | M | tri-citrate de lithium tétrahydraté | | | | 8,1 | 20 | %(w/v) | PEG 3350 | | | |
| F01 | 0,2 | M | chlorure de magnésium hexahydraté 0,1 M | | M | TRIS HCl | 8,5 | 15 | %(w/v) | PEG 4000 | | | |
| F02 | 0,2 | M | chlorure de calcium dihydraté | 0,1 | M | Sel de sodium d'HEPES | 7,5 | 14 | %(v/v) | PEG 400 | | | |
| F03 | 0,2 | M | acétate de sodium trihydraté | 0,1 | M | TRIS HCl | 8,5 | 15 | %(w/v) | PEG 4000 | | | |
| F04 | 0,2 | M | sulfate d'ammonium | | | | | 15 | %(w/v) | PEG 8000 | | | |
| F05 | | | | 0,1 | M | Sel de sodium d'HEPES | 7,5 | 0,7 | M | citrate trisodique dihydraté | | | |
| F06 | 0,2 | M | acétate de calcium hydraté | 0,1 | M | cacodylate de sodium | 6,5 | 9 | %(w/v) | PEG 8000 | | | |
| F07 | 0,2 | M | sodium chloride | | | | 6,9 | 20 | %(w/v) | PEG 3350 | | | |
| F08 | 0,2 | M | potassium thiocyanate | | | | 7 | 20 | %(w/v) | PEG 3350 | | | |
| F09 | 0,2 | M | potassium formate | | | | 7,3 | 20 | %(w/v) | PEG 3350 | | | |
| F10 | 0,2 | M | acétate d'ammonium | | | | 7,1 | 20 | %(w/v) | PEG 3350 | | | |
| F11 | 0,2 | M | di-ammonium tartrate | | | | 6,6 | 20 | %(w/v) | PEG 3350 | | | |
| F12 | 0,2 | M | citrate trisodique dihydraté | | | | 8,2 | 20 | %(w/v) | PEG 3350 | | | |
| G01 | | | | 0,1 | M | cacodylate de sodium | 6,5 | 0,7 | M | acétate de sodium trihydraté | | | |
| G02 | 0,2 | M | sulfate d'ammonium | 0,1 | M | cacodylate de sodium | 6,5 | 15 | %(w/v) | PEG 8000 | | | |
| G03 | 0,2 | M | chlorure de magnésium hexahydraté | 0,1 | M | Sel de sodium d'HEPES | 7,5 | 15 | %(w/v) | PEG 400 | | | |
| G04 | 0,2 | M | sulfate d'ammonium | | | | | 15 | %(w/v) | PEG 4000 | | | |
| G05 | 2 | %(v/v) | PEG 400 | 0,1 | M | Sel de sodium d'HEPES | 7,5 | 1 | M | ammonium sulfate | | | |
| G06 | | | | 0,1 | M | acétate de sodium trihydraté | 4,6 | 1 | M | sulfate d'ammonium | | | |
| G07 | 0,2 | M | chlorure de calcium dihydraté | | | | 5,1 | 20 | %(w/v) | PEG 3350 | | | |
| G08 | 0,2 | M | nitrate de lithium | | | | 7,1 | 20 | %(w/v) | PEG 3350 | | | |
| G09 | 0,2 | M | formiate d'ammonium | | | | 6,6 | 20 | %(w/v) | PEG 3350 | | | |
| G10 | 0,2 | M | lithium monohydrate de sulfate | | | | 6,4 | 20 | %(w/v) | PEG 3350 | | | |
| G11 | 0,2 | M | dihydrogénophosphate de sodium monohydraté | | | | 4,5 | 20 | %(w/v) | PEG 3350 | | | |
| G12 | 0,2 | M | tri-citrate de potassium monohydraté | | | | 8,3 | 20 | %(w/v) | PEG 3350 | | | |
| H01 | 0,2 | M | citrate trisodique dihydraté | 0,1 | M | cacodylate de sodium | 6,5 | 15 | %(v/v) | iso-propanol | | | |
| H02 | | | | 0,1 | M | Sel de sodium d'HEPES | 7,5 | 0,75 | M | lithium monohydrate de sulfate | | | |
| H03 | 0,2 | M | chlorure de calcium dihydraté | 0,1 | M | acétate de sodium trihydraté | 4,6 | 10 | %(v/v) | iso-propanol | | | |
| H04 | | | | | | | | 1 | M | sulfate d'ammonium | | | |
| H05 | 10 | %(v/v) | iso-propanol | 0,1 | M | citrate trisodique dihydraté | 5,6 | 10 | %(w/v) | PEG 4000 | | | |
| H06 | | | | 0,1 | M | TRIS HCl | 8,5 | 1 | M | dihydrogénophosphate d'ammonium | | | |
| H07 | 0,2 | M | chlorure de potassium | | | | 6,9 | 20 | %(w/v) | PEG 3350 | | | |
| H08 | 0,2 | M | nitrate de magnésium hexahydraté | | | | 5,8 | 20 | %(w/v) | PEG 3350 | | | |
| H09 | 0,2 | M | acétate de lithium dihydraté | | | | 7,8 | 20 | %(w/v) | PEG 3350 | | | |
| H10 | 0,2 | M | sulfate de magnésium heptahydraté | | | | 5,9 | 20 | %*(*w/v*)* | PEG 3350 | | | |
| H11 | 0,2 | M | di-sodium hydrogénophosphate dihydraté | | | | 9,1 | 20 | %(w/v) | PEG 3350 | | | |
| H12 | 0,2 | M | di-ammonium citrate hydrogène | | | | 5 | 20 | %(w/v) | PEG 3350 | | | |

| ANNEXE2A | | | | | | | | | | | | | |
|---|---|---|---|---|---|---|---|---|---|---|---|---|---|
| Annexe plaque « Hampton 3 » | | | | | | | | | | | | | |
| Puit | C | U | Sel | C | U | Tampon | pH | C | U Précipitant | | C | U | Additif |
| A01 | 0,1 | M | chlorure de sodium | 0,1 | M | acétate de sodium trihydraté | 4,6 | 12 | %(v/v) | MPD | | | |
| A02 | 0,1 | M | lithium monohydrate de sulfate | 0,1 | M | acétate de sodium trihydraté | 4,6 | 1 | M | dihydrogénophosphate d'ammonium | | | |
| A03 | 0,1 | M | chlorure de sodium | 0,1 | M | citrate trisodique dihydraté | 5,6 | 12 | %(w/v) | PEG 4000 | | | |
| A04 | 0,1 | M | chlorure de magnésium hexahydraté | 0,1 | M | ADA | 6,5 | 12 | %(w/v) | PEG 6000 | | | |
| A05 | 0,1 | M | sulfate d'ammonium | 0,1 | M | Sel de sodium d'HEPES | 7,5 | 18 | %(v/v) | PEG 400 | | | |
| A06 | 0,1 | M | di-ammonium phosphate d'hydrogène | 0,1 | M | TRIS HCl | 8,5 | 0,5 | M | di-sodium hydrogénophosphate | 0,5 | M | di-potassium hydrogénophosphate |
| A07 | 0,01 | M | chlorure de magnésium hexahydraté | 0,05 | M | MES | 5,6 | 2 | M | lithium monohydrate de sulfate | | | |
| A08 | 0,1 | M | chlorure de potassium | 0,05 | M | MES | 6 | 10 | %(v/v) | PEG 400 | 0,01 | M | chlorure de magnésium hexahydraté |
| A09 | 0,2 | M | chlorure de potassium | 0,05 | M | cacodylate de sodium | 6 | 10 | %(w/v) | PEG 4000 | 0,01 | M | chlorure de calcium dihydraté |
| A10 | 0,08 | M | acétate de magnésium tetrahydrate | 0,05 | M | cacodylate de sodium | 6,5 | 30 | %(w/v) | PEG 4000 | 0 | | 0 |
| A11 | 0,2 | M | chlorure d'ammonium | 0,05 | M | Sel de sodium d'HEPES | 7 | 30,0% | %(w/v) | 1,6-hexanediol | 0,01 | M | chlorure de magnesium hexahydraté |
| A12 | 0,1 | M | chlorure de potassium | 0,05 | M | TRIS HCl | 7,5 | 10 | %(v/v) | PEG manomethyl éther 550 | 0,015 | M | chlorure de magnésium hexahydraté |
| B01 | 0,1 | M | acétate de zinc dihydraté | 0,1 | M | acétate de sodium trihydraté | 4,6 | 12 | %(w/v) | PEG 4000 | | | |
| B02 | 0,1 | M | chlorure de sodium | 0,1 | M | acétate de sodium trihydraté | 4,6 | 12 | %(w/v) | PEG 6000 | | | |
| B03 | 0,1 | M | lithium monohydrate de sulfate | 0,1 | M | citrate trisodique dihydraté | 5,6 | 12 | %(w/v) | PEG 6000 | | | |
| B04 | 0 | | 0 | 0,1 | M | ADA | 6,5 | 12 | %(v/v) | MPD | | | |
| B05 | 0,1 | M | sulfate d'ammonium | 0,1 | M | Sel de sodium d'HEPES | 7,5 | 10 | %(w/v) | PEG 4000 | | | |
| B06 | 0 | | 0 | 0,1 | M | TRIS HCl | 8,5 | 0,1 | M | acétate de sodium trihydraté | | | |
| B07 | 0,01 | M | acétate de magnésium tetrahydrate | 0,05 | M | MES | 5,6 | 2,5 | M | sulfate d'ammonium | | | |
| B08 | 0,005 | M | sulfate de magnésium | 0,05 | M | MES | 6 | 5 | %(w/v) | PEG 4000 | 0 | | 0 |
| B09 | 0,01 | M | acétate de magnésium tetrahydrate | 0,05 | M | cacodylate de sodium | 6,5 | 1,3 | M | lithium monohydrate de sulfate | 0 | | 0 |
| B10 | 0,2 | M | chlorure de potassium | 0,05 | M | cacodylate de sodium | 6,5 | 10 | %(w/v) | PEG 8000 | 0,1 | M | acétate de magnésium tetrahydrate |
| B11 | 0,1 | M | chlorure de potassium | 0,05 | M | Sel de sodium d'HEPES | 7 | 15 | %(v/v) | MPD | 0,005 | M | sulfate de magnésiumaq. |
| B12 | 0,01 | Mi | acétate de magnésium tetrahydrate | 0,05 | M | TRIS HCl | 7,5 | 5 | %(v/v) | iso-propanol | 0 | | 0 |
| C01 | 0,2 | M | sulfate d'ammonium | 0,1 | M | acétate de sodium trihydraté | 4,6 | 10 | %(w/w) | PEG 4000 | | | |
| C02 | 0,1 | M | chlorure de magnésium hexahydraté | 0,1 | M | acétate de sodium trihydraté | 4,6 | 12 | %(w/v) | PEG 6000 | | | |
| C03 | 0,1 | M | chlorure de magnésium hexahydraté | 0,1 | M | citrate trisodique dihydraté | 5,6 | 4 | %(v/v) | MPD | | | |
| C04 | 0,1 | M | lithium monohydrate de sulfate | 0,1 | M | ADA | 6,5 | 1 | M | sulfate de magnésiumhydrate | | | |

| ANNEXE 2A | | | | | | | | | | | | | |
|---|---|---|---|---|---|---|---|---|---|---|---|---|---|
| Annexe plaque « Hampton 3 » | | | | | | | | | | | | | |
| C05 | 0,1 | M | citrate trisodique dihydraté | 0,1 M | | Sel de sodium d'HEPES | 7,5 | 12 | %(v/v) | MPD | | | |
| C06 | 0 | | 0 | 0,1 | M | TRIS HCl | 8,5 | 0,1 | M | chlorure de sodium | | | |
| C07 | 0,1 | M | acétate de magnésium tetrahydrate | 0,05 | M | MES | 5,6 | 20 | %(v/v) | MPD | | | |
| C08 | 0,01 | M | chlorure de magnésium hexahydraté | 0,05 | M | cacodylate de sodium | 6 | 1 | 1M | lithium monohydrate de sulfate | 0 | | 0 |
| C09 | 0,01 | M | sulfate de magnésium | 0,05 | M | cacodylate de sodium | 6,5 | 2 | 2M | sulfate d'ammonium | 0 | | 0 |
| C10 | 0,2 | M | acétate d'ammonium | 0,05 | M | cacodylate de sodium | 6,5 | 30 | %(w/v) | PEG 8000 | 0,01 | M | acétate de magnésium tetrahydrate |
| C11 | 0,1 | M | chlorure de potassium | 0,05 | M | Sel de sodium d'HEPES | 7 | 5 | %(v/v) | PEG 400 | 0,01 | M | chlorure de magnésium hexahydraté |
| C12 | 0,05 | M | acétate d'ammonium | 0,05 | M | TRIS HCl | 7,5 | 10 | %(v/v) | MPD | 0,01 | M | chlorure de magnésium hexahydraté |
| D01 | 0,1 | M | chlorure de sodium | 0,1 | M | acétate de sodium trihydraté | 4,6 | 12 | %(v/v) | iso-propanol | | | |
| D02 | 0,1 | M | chlorure de sodium | 0,1 | M | citrate trisodique dihydraté | 5,6 | 18 | %(v/v) | PEG 400 | | | |
| D03 | 0 | | 0 | 0,1 | M | citrate trisodique dihydraté | 5,6 | 0,1 | M | chlorure de sodium | | | |
| D04 | 0,3 | M | lithium monohydrate de sulfate | 0,1 | M | ADA | 6,5 | 4 | %(v/v) | PEG 400 | | | |
| D05 | 0 | 0 | 0 | 0,1 | M | Sel de sodium d'HEPES | 7,5 | 1 | M | citrate trisodique dihydraté | | | |
| D06 | 0,1 | M | di-ammonium phosphate d'hydrogène | 0,1 | M | TRIS HCl | 8,5 | 12 | %(w/v) | PEG 6000 | | | |
| D07 | 0,2 | M | chlorure de potassium | 0,05 | M | MES | 5,6 | 10 | %(v/v) | PEG 400 | 0,01 | M | sulfate de magnésium |
| D08 | 0,01 | M | sulfate de magnésium | 0,05 | M | cacodylate de sodium | 6 | 1,8 | M | lithium monohydrate de sulfate | 0 | | 0 |
| D09 | 0,1 | M | acétate d'ammonium | 0,05 | M | cacodylate de sodium | 6,5 | 10 | %(v/v) | iso-propanol | 0,015 | M | acétate de magnésium tetrahydrate |
| Puit | C | U | Sel | C | U | Tampon | pH | C | U | Précipitant | C | U | Additif |
| D10 | 0,05 | M | sulfate de magnésium aq | 0,05 | M | Sel de sodium d'HEPES | 7 | 1,6 | M | lithium monohydrate de sulfate | 0 | | 0 |
| D11 | 0,1 | M | chlorure de potassium | 0,05 | M | Sel de sodium d'HEPES | 7 | 10 | %(v/v) | PEG 400 | 0,01 | M | chlorure de calcium dihydraté |
| D12 | 0,2 | M | chlorure de potassium | 0,05 | M | TRIS HCl | 7,5 | 10 | %(w/v) | PEG 4000 | 0,05 | M | chlorure de magnésium hexahydraté |
| E01 | 0 | | 0 | 0,1 | M | acétate de sodium trihydraté | 4,6 | 12 | %(w/v) | PEG 4000 | | | |
| E02 | 0,1 | M | lithium monohydrate de sulfate | 0,1 | M | citrate trisodique dihydraté | 5,6 | 12 | %(w/v) | PEG 4000 | | | |
| E03 | 0,1 | M | lithium monohydrate de sulfate | 0,1 | M | citrate trisodique dihydraté | 5,6 | 4 | %(v/v) | PEG 400 | | | |
| E04 | 0,1 | M | sulfate d'ammonium | 0,1 | M | Sel de sodium d'HEPES | 7,5 | 0,5 | M | di-sodium hydrogénophosphate | 0,5 | M | di-potassium hydrogénophosphate |
| E05 | 0,6 | M | sulfate de magnésiumhydrate | 0,1 | M | Sel de sodium d'HEPES | 7,5 | 4 | %(v/v) | PEG 400 | | | |
| E06 | 0,1 | M | potassium / sodium tartrate tetrahydrate | 0,1 | M | TRIS HCl | 8,5 | 0,4 | M | sulfate de magnésiumhydrate | | | |
| E07 | 0,2 | M | chlorure de potassium | 0,05 | M | MES | 5,6 | 5 | %(w/v) | PEG 8000 | 0,01 | M | chlorure de magnésium hexahydraté |
| E08 | 0,015 | M | acétate de magnésium tetrahydrate | 0,05 | M | cacodylate de sodium | 6 | 1,7 | M | sulfate d'ammonium | 0 | | 0 |
| E09 | 0,2 | M | chlorure de potassium | 0,05 | M | cacodylate de sodium | 6,5 | 10 | %(w/v) | 1,6-hexanediol | 0,005 | M | chlorure de magnésium hexahydraté |
| E10 | 0,01 | M | chlorure de magnésium hexahydraté | 0,05 | M | Sel de sodium d'HEPES | 7 | 4 | M | lithium chloride | 0 | | 0 |
| E11 | 0,2 | M | chlorure de potassium | 0,05 | M | Sel de sodium d'HEPES | 7 | 20 | %(v/v) | PEG 200 | 0,025 | M | sulfate de magnésium aq. |
| E12 | 0,025 | M | sulfate de magnésiumaq. | 0,05 | M | TRIS HCl | 8,5 | 1,8 | M | sulfate d'ammonium | 0 | | 0 |
| F01 | | | 0 | 0,1 | M | acétate de sodium trihydraté | 4,6 | 1 | M | sulfate d'ammonium | | | |
| F02 | 0,1 | M | citrate trisodique dihydraté | 0,1 | M | citrate trisodique dihydraté | 5,6 | 10 | % %(v/v) | iso-propanol | | | |
| F03 | 0 | | 0 | 0,1 | M | ADA | 6,5 | 1 | M | sulfate d'ammonium | | | |
| F04 | 0,1 | M | chlorure de sodium | 0,1 | M | Sel de sodium d'HEPES | 7,5 | 10 | %(w/v) | PEG 4000 | | | |
| F05 | 0,6 | M | sulfate de magnésiumhydrate | 0,1 | M | Sel de sodium d'HEPES | 7,51 | 4 | %(v/v) | MPD | | | |
| F06 | 0 | | 0 | 0,1 | M | TRIS HCl | 8,5 | 0,2 | M | lithium monohydrate de sulfate | | | |
| F07 | 0,1 | M | sulfate d'ammonium | 0,05 | M | MES | 5,6 | 20 | %(w/v) | PEG 8000 | 0,01 | M | chlorure de magnésium hexahydraté |
| F08 | 0,1 | M | chlorure de potassium | 0,05 | M | cacodylate de sodium | 6 | 15 | %(v/v) | iso-propanol | 0,025 | M | chlorure de magnésium hexahydraté |
| F09 | 0,08 | M | acétate de magnésium tetrahydrate | 0,05 | M | cacodylate de sodium | 6,5 | 15 | %(v/v) | PEG 400 | 0 | | 0 |
| F10 | 0,01 | M | chlorure de magnésium hexahydraté | 0,05. | M | Sel de sodium d'HEPES | 7 | 1,6 | M | sulfate d'ammonium | 0 | | 0 |
| F11 | 0,2 | M | acétate d'ammonium | 0,05 | M | Sel de sodium d'HEPES | 7 | 5 | %(w/v) | PEG 4000 | 0,151 | M | acétate de magnésium tetrahydrate |
| F12 | 0,005 | M | sulfate de magnésium aq. | 0,05 | M | TRIS HCl | 8,5 | 35 | %(w/v) | 1.6-hexanediol | 0 | | 0 |
| G01 | 0 | | 0 | 0,1 | M | acétate de sodium trihydraté | 4,6 | 1 | M | sulfate de magnésiumheptahydrate | | | |
| G02 | 0,1 | M | chlorure de sodium | 0,1 | M | citrate trisodique dihydraté | 5,6 | 12 | %(v/v) | MPD | | | |
| G03 | 0,1 | M | lithium monohydrate de sulfate | 0,1 | M | ADA | 6,5 | 12 | %(w/v) | PEG 4000 | 2 | %(v/v) | iso-propanol |
| G04 | 0,1 | M | chlorure de magnésium hexahydraté | 0,1 | M | Sel de sodium d'HEPES | 7,5 | 18 | %(v/v) PEG 400 | | | | |
| G05 | 0,1 | M | lithium monohydrate de sulfate | 0,1 | M | Sel de sodium d'HEPES | 7,5 | 0,1 | M | potassium/sodium tartrate tetrahydrate | | | |
| G06 | 0 | | 0 | 0,1 | M | TRIS HCl | 8,5 | 0,5 | M | sulfate d'ammonium | | | |
| G07 | 0,02 | M | chlorure de magnésium hexahydraté | 0,05 | M | MES | 6 | 15 | %(v/v) | iso-propanol | 0 | | 0 |
| G08 | 0,04 | M | chlorure de magnésium hexahydraté | 0,05 | M | cacodylate de sodium | 6 | 5 | %(v/v) | MPD | 0 | | 0 |
| G09 | 0,2 | M | chlorure de potassium | 0,05 | M | cacodylate de sodium | 6,5 | 10 | %(w/v) | PEG 4000 | 0,01 | M | chlorure de magnésium hexahydraté |
| G10 | 0,005 | M | chlorure de magnésium hexahydraté | 0,05 | M | Sel de sodium d'HEPES | 7 | 25 | %(v/v) | PEG monomethyl éther 550 | 0 | | 0 |
| G11 | 0,1 | M | acétate d'ammonium | 0,05 | M | Sel de sodium d'HEPES | 7 | 5 | %(w/v) | PEG 8000 | 0,02 | M | chlorure de magnésium hexahydraté |
| G12 | 0,1 | M | chlorure de potassium | 0,05 | M | TRIS HCl | 8,5 | 30 | %(v/v) | PEG 400 | 0,01 | M | chlorure de magnésium hexahydraté |
| H01 | 0,1 | M | chlorure de magnésium hexahydraté | 0,1 | M | acétate de sodium trihydraté | 4,6 | 18 | %(v/v) | PEG 400 | | | |
| H02 | 0 | | 0 | 0,1 | M | citrate trisodique dihydraté | 5,6 | 1 | M | sulfate de magnésiumheptahydrate | | | |
| H03 | 0 | | 0 | 0,1 | M | ADA | 6,5 | 1 | M | di-ammonium phosphate d'hydrogène | | | |
| H04 | 0 | | 0 | 0,1 | M | Sel de sodium d'HEPES | 7,5 | 1 | M | potassium/sodium tartrate tetrahydrate | | | |
| H05 | 0,1 | M | lithium monohydrate de sulfate | 0,1 | M | TRIS HCl | 8,5 | 12 | %(v/v) | MPD | | | |
| H06 | 0,1 | M | citrate trisodique dihydraté | 0,1 | M | TRIS HCl | 8,5 | 5 | %(v/v) | PEG 400 | | | |
| H07 | 0,1 | M | acétate d'ammonium | 0,05 | M | MES | 6 | 0,6 | M | chlorure de sodium | 0,0051 | M | sulfate de magnésium |
| H08 | 0,04 | M | acétate de magnésium tetrahydrate | 0,05 | M | cacodylate de sodium | 6 | 30 | %(v/v) | MPD | 0 | | 0 |
| H09 | 0,2 | M | acétate d'ammonium | 0,05 | M | cacodylate de sodium | 6,5 | 10 | %(w/v) | PEG 4000 | 0,01 | | chlorure de calcium dihydraté |
| H10 | 0,2 | M | chlorure de potassium | 0,05 | M | Sel de sodium d'HEPES | 7 | 20,0% | %(w/v) | 1.6-hexanediol | 0,01M | | chlorure de magnésium hexahydraté |
| H11 | 0,01 | M | chlorure de magnésium hexahydraté | 0,05 | M | TRIS HCl | 7,5 | 1,6 | M | sulfate d'ammonium | 0 | | 0 |
| H12 | 0,2 | M | chlorure d'ammonium | 0,05 | M | TRIS HCl | 8,5 | 30 | %(w/v) | PEG 4000 | 0,01 | M | chlorure de calcium dihydraté |

**Annexe 2A**

| Annexe plaque « Hampton 4 » | | | | | | | |
|---|---|---|---|---|---|---|---|
| Puit | C | U | Tampon | pH | C | U | Précipitant |
| A01 | 0,1 | M | acide citrique | 4 | 0,8 | M | sulfate d'ammonium |
| A02 | 0,1 | M | acide citrique | 5 | 0,8 | M | sulfate d'ammonium |
| A03 | 0,1 | M | MES | 6 | 0,8 | M | sulfate d'ammonium |
| A04 | 0,1 | M | HEPES | 7 | 0,8 | M | sulfate d'ammonium |
| A05 | 0,1 | M | TRIS | 8 | 0,8 | M | sulfate d'ammonium |
| A06 | 0,1 | M | bicine | 9 | 0,8 | M | sulfate d'ammonium |
| B01 | 0,1 | M | acide citrique | 4 | 1,6 | M | sulfate d'ammonium |
| B02 | 0,1 | M | acide citrique | 5 | 1,6 | M | sulfate d'ammonium |
| B03 | 0,1 | M | MES | 6 | 1,6 | M | sulfate d'ammonium |
| B04 | 0,1 | M | HEPES | 7 | 1,6 | M | sulfate d'ammonium |
| B05 | 0,1 | M | TRIS | 8 | 1,6 | M | sulfate d'ammonium |
| B06 | 0,1 | M | bicine | 9 | 1,6 | M | sulfate d'ammonium |
| C01 | 0,1 | M | acide citrique | 4 | 2,4 | M | sulfate d'ammonium |
| C02 | 0,1 | M | acide citrique | 5 | 2,4 | M | sulfate d'ammonium |
| C03 | 0,1 | M | MES | 6 | 2,4 | M | sulfate d'ammonium |
| C04 | 0,1 | M | HEPES | 7 | 2,4 | M | sulfate d'ammonium |
| C05 | 0,1 | M | TRIS | 8 | 2,4 | M | sulfate d'ammonium |
| C06 | 0,1 | M | bicine | 9 | 2,4 | M | sulfate d'ammonium |
| D01 | 0,1 | M | acide citrique | 4 | 3 | M | sulfate d'ammonium |
| D02 | 0,1 | M | acide citrique | 5 | 3 | M | sulfate d'ammonium |
| D03 | 0,1 | M | MES | 6 | 3 | M | sulfate d'ammonium |
| D04 | 0,1 | M | HEPES | 7 | 3 | M | sulfate d'ammonium |
| D05 | 0,1 | M | TRIS | 8 | 3 | M | sulfate d'ammonium |
| D06 | 0,1 | M | bicine | 9 | 3 | M | sulfate d'ammonium |
| E01 | | | | 4 | 1 | M | malonate |
| E02 | | | | 4 | 1,5 | M | malonate |
| E03 | | | | 4 | 1,9 | M | malonate |
| E04 | | | | 4 | 2,4 | M | malonate |
| E05 | | | | 4 | 2,9 | M | malonate |
| E06 | | | | 4 | 3,4 | M | malonate |
| F01 | | | | 5 | 1 | M | malonate |
| F02 | | | | 5 | 1,5 | M | malonate |
| F03 | | | | 5 | 1,9 | M | malonate |
| F04 | | | | 5 | 2,4 | M | malonate |
| F05 | | | | 5 | 2,9 | M | malonate |
| F06 | | | | 5 | 3,4 | M | malonate |
| G01 | | | | 6 | 1 | M | malonate |
| G02 | | | | 6 | 1,5 | M | malonate |
| G03 | | | | 6 | 1,9 | M | malonate |
| G04 | | | | 6 | 2,4 | M | malonate |
| G05 | | | | 6 | 2,9 | M | malonate |
| G06 | | | | 6 | 3,4 | M | malonate |
| H01 | | | | 7 | 1 | M | malonate |
| H02 | | | | 7 | 1,5 | M | malonate |
| H03 | | | | 7 | 1,9 | M | malonate |

**Annexe 2A**

| Annexe plaque « Hampton 4 » | | | | | | | | | | |
|---|---|---|---|---|---|---|---|---|---|---|
| Puit | C | U | Tampon | pH | C | U | Précipitant | C | U | Additif |
| H04 | | | | 7 | 2,4 | M | malonate | | | |
| H05 | | | | 7 | 2,9 | M | malonate | | | |
| H06 | | | | 7 | 3,4 | M | malonate | | | |
| A07 | 0,8 | M | sodium / phosphate de potassium | 5 | 0,78 | M | dihydrogénophosphate de sodium monohydraté | 0,016 | M | di-potassium hydrogénophosphate |
| A08 | 0,8 | M | sodium / phosphate de potassium | 5,6 | 0,72 | M | dihydrogénophosphate de sodium monohydraté | 0,08 | M | di-potassium hydrogénophosphate |
| A09 | 0,8 | M | sodium / phosphate de potassium | 6,3 | 0,52 | M | dihydrogénophosphate de sodium monohydraté | 0,28 | M | di-potassium hydrogénophosphate |
| A10 | 0,8 | M | sodium / phosphate de potassium | 6,9 | 0,28 | M | dihydrogénophosphate de sodium monohydraté | 0,52 | M | di-potassium hydrogénophosphate |
| A11 | 0,8 | M | sodium / phosphate de potassium | 7,5 | 0,12 | M | dihydrogénophosphate de sodium monohydraté | 0,68 | M | di-potassium hydrogénophosphate |
| A12 | 0,8 | M | sodium / phosphate de potassium | 8,2 | 0,032 | M | dihydrogénophosphate de sodium monohydraté | 0,768 | M | di-potassium hydrogénophosphate |
| B07 | 1 | M | sodium / phosphate de potassium | 5 | 0,98 | M | dihydrogénophosphate de sodium monohydraté | 0,02 | M | di-potassium hydrogénophosphate |
| B08 | 1 | M | sodium / phosphate de potassium | 5,6 | 0,9 | M | dihydrogénophosphate de sodium monohydraté | 0,1 | M | di-potassium hydrogénophosphate |
| B09 | 1 | M | sodium / phosphate de potassium | 6,3 | 0,65 | M | dihydrogénophosphate de sodium monohydraté | 0,35 | M | di-potassium hydrogénophosphate |
| B10 | 1 | M | sodium / phosphate de potassium | 6,9 | 0,35 | M | dihydrogénophosphate de sodium monohydraté | 0,65 | M | di-potassium hydrogénophosphate |
| B11 | 1 | M | sodium / phosphate de potassium | 7,5 | 0,15 | M | dihydrogénophosphate de sodium monohydraté | 0,85 | M | di-potassium hydrogénophosphate |
| B12 | 1 | M | sodium / phosphate de potassium | 8,2 | 0,04 | M | dihydrogénophosphate de sodium monohydraté | 0,96 | M | di-potassium hydrogénophosphate |
| C07 | 1,4 | M | sodium / phosphate de potassium | 5 | 1,372 | M | dihydrogénophosphate de sodium monohydraté | 0,028 | M | di-potassium hydrogénophosphate |
| C08 | 1,4 | M | sodium 1 phosphate de potassium | 5,6 | 1,26 | M | dihydrogénophosphate de sodium monohydraté | 0,14 | M | di-potassium hydrogénophosphate |
| C09 | 1,4 | M | sodium / phosphate de potassium | 6,3 | 0,91 | M | dihydrogénophosphate de sodium monohydraté | 0,49 | M | di-potassium hydrogénophosphate |
| C10 | 1,4 | M | sodium / phosphate de potassium | 6,9 | 0,49 | M | dihydrogénophosphate de sodium monohydraté | 0,91 | M | di-potassium hydrogénophosphate |
| C11 | 1,4 | M | sodium / phosphate de potassium | 7,5. | 0,21 | M | dihydrogénophosphate de sodium monohydraté | 1,19 | M | di-potassium hydrogénophosphate |
| C12 | 1,4 | M | sodium / phosphate de potassium | 8,2 | 0,056 | M | dihydrogénophosphate de sodium monohydraté | 1,344 | M | di-potassium hydrogénophosphate |
| D07 | 1,8 | M | sodium / phosphate de potassium | 5 | 1,764 | M | dihydrogénophosphate de sodium monohydraté | 0,036 | M | di-potassium hydrogénophosphate |
| D08 | 1,8 | M | sodium / phosphate de potassium | 5,6 | 1,62 | M | dihydrogénophosphate de sodium monohydraté | 0,18 | M | di-potassium hydrogénophosphate |
| D09 | 1,8 | M | sodium / phosphate de potassium | 6,3 | 1,17 | M | dihydrogénopnosphate de sodium monohydraté | 0,631 | M | di-potassium hydrogénophosphate |
| D10 | 1,8 | M | sodium / phosphate de potassium | 6,9 | 0,63 | M | dihydrogénophosphate de sodium monohydraté | 1,171 | M | di-potassium hydrogénophosphate |
| D11 | 1,8 | M | sodium / phosphate de potassium | 7,5 | 0,27 | M | dihydrogénophosphate de sodium monohydraté | 1,531 | M | di-potassium hydrogénophosphate |
| D12 | 1,8 | M | sodium / phosphate de potassium | 8,2 | 0,072 | M | dihydrogénophosphate de sodium monohydraté | 1,728 | M | di-potassium hydrogénophosphate |
| E07 | 0,1 | M | acide citrique | 4 | 0,8 | M | formiate de sodium pH4 | | | |
| E08 | 0,1 | M | acide citrique | 5 | 0,8 | M | formiate de sodium pH5 | | | |
| E09 | 0,1 | | MES | 6 | 0,8 | M | formiate de sodium pH6 | | | |
| E10 | 0,1 | M | HEPES | 7 | 0,8 | M | formiate de sodium pH7 | | | |
| E11 | 0,1 | M | TRIS | 8 | 0,8 | M | formiate de sodium pH8 | | | |
| E12 | 0,1 | M | bicine | 9 | 0,8 | M | formiate de sodium pH9 | | | |
| F07 | 0,1 | M | acide citrique | 4 | 1,6 | M | formiate de sodium pH4 | | | |
| F08 | 0,1 | M | acide citrique | 5 | 1,6 | M | formiate de sodium pH5 | | | |
| F09 | 0,1 | M | MES | 6 | 1,6 | M | formiate de sodium pH6 | | | |
| F10 | 0,1 | M | HEPES | 7 | 1,6 | M | formiate de sodium pH7 | | | |
| Fil | 0,1 | M | TRIS | 8 | 1,6 | M | formiate de sodium pH8 | | | |
| F12 | 0,1 | M | bicine | 9 | 1,6 | M | formiate de sodium pH9 | | | |
| G07 | 0,1 | M | acide citrique | 4 | 2,4 | M | formiate de sodium pH4 | | | |
| G08 | 0,1 | M | acide citrique | 5 | 2,4 | M | formiate de sodium pH5 | | | |
| G09 | 0,1 | M | MES | 6 | 2,4 | M | formiate de sodium pH6 | | | |
| G10 | 0,1 | M | HEPES | 7 | 2,4 | M | formiate de sodium pH7 | | | |
| C11 | 0,1 | M | TRIS | 8 | 2,4 | M. | formiate de sodium pH8 | | | |
| G12 | 0,1 | M | bicine | 9 | 2,4 | M | formiate de sodium pH9 | | | |
| H07 | 0,1 | M | acide citrique | 4 | 3,2 | M | formiate de sodium pH4 | | | |
| H08 | 0,1 | M | acide citrique | 5 | 3,2 | M | formiate de sodium pH5 | | | |
| H09 | 0,1 | M | MES | 6 | 3,2 | M | formiate de sodium pH6 | | | |
| H10 | 0,1 | M | HEPES | 7 | 3,2 | M | formiate de sodium pH7 | | | |
| H11 | 0,1 | M | TRIS | 8 | 3,2 | M | formiate de sodium pH8 | | | |
| H12 | 0,1 | M | bicine | 9 | 3,2 | M | formiate de sodium pH9 | | | |

| Annexe plaque « Hampton 5 » | | | | | | | | | | |
|---|---|---|---|---|---|---|---|---|---|---|
| Well | C | U | Sel | C | U | Tampon | pH | C | U | Précipitant |
| A01 | | | | 0,1 | M | acide citrique | 4 | 5 | %(w/v) | PEG 6000 |
| A02 | | | | 0,1 | M | acide citrique | 5 | 5 | %(w/v) | PEG 6000 |
| A03 | | | | 0,1 | M | MES | 6 | 5 | %(w/v) | PEG 6000 |
| A04 | | | | 0,1 | M | HEPES | 7 | 5 | %(w/v) | PEG 6000 |
| A05 | | | | 0,1 | M | TRIS | 8 | 5 | %(w/v) | PEG 6000 |
| A06 | | | | 0,1 | M | bicine | 9 | 5 | %(w/v) | PEG 6000 |
| B01 | | | | 0,1 | M | acide citrique | 4 | 10 | %(w/v) | PEG 6000 |
| B02 | | | | 0,1 | M | acide citrique | 5 | 10 | %(w/v) | PEG 6000 |
| B03 | | | | 0,1 | M | MES | 6 | 10 | %(w/v) | PEG 6000 |
| B04 | | | | 0,1 | M | HEPES | 7 | 10 | %(w/v) | PEG 6000 |
| B05 | | | | 0,1 | M | TRIS | 8 | 10 | %(w/v) | PEG 6000 |
| B06 | | | | 0,1 | M | bicine | 9 | 10 | %(w/v) | PEG 6000 |
| C01 | | | | 0,1 | M | acide citrique | 4 | 20 | %(w/v) | PEG 6000 |
| C02 | | | | 0,1 | M | acide citrique | 5 | 20 | %(w/v) | PEG 6000 |
| C03 | | | | 0,1 | M | MES | 6 | 20 | %(w/v) | PEG 6000 |
| C04 | | | | 0,1 | M | HEPES | 7 | 20 | %(w/v) | PEG 6000 |
| C05 | | | | 0,1 | M | TRIS | 8 | 20 | %(w/v) | PEG 6000 |
| C06 | | | | 0,1 | M | bicine | 9 | 20 | %(w/v) | PEG 6000 |
| D01 | | | | 0,1 | M | acide citrique | 4 | 30 | %(w/v) | PEG 6000 |
| D02 | | | | 0,1 | M | acide citrique | 5 | 30 | %(w/v) | PEG 6000 |
| D03 | | | | 0,1 | M | MES | 6 | 30 | %(w/v) | PEG 6000 |
| D04 | | | | 0,1 | M | HEPES | 7 | 30 | %(w/v) | PEG 6000 |
| D05 | | | | 0,1 | M | TRIS | 8 | 30 | %(w/v) | PEG 6000 |
| D06 | | | | 0,1 | M | bicine | 9 | 30 | %(w/v) | PEG 6000 |
| E01 | | | | 0,1 | M | acide citrique | 4 | 10 | %(v/v) | 2-Methyl-2.4-penta nediol |
| E02 | | | | 0,1 | M | acétate de sodium trihydraté | 5 | 10 | %(v/v) | 2-Methyl-2.4-pentanediol |
| E03 | | | | 0,1 | M | MES | 6 | 10 | %(v/v) | 2-Methyl-2.4-pentanediol |
| E04 | | | | 0,1 | M | HEPES | 7 | 10 | %(v/v) | 2-Methyl-2.4-pentanediol |
| E05 | | | | 0,1 | M | TRIS | 8 | 10 | %(v/v) | 2-Methyl-2.4-pentanediol |
| E06 | | | | 0,1 | M | bicine | 9 | 10 | %(v/v) | 2-Methyl-2.4-pentanediol |
| F01 | | | | 0,1 | M | acide citrique | 4 | 20 | %(v/v) | 2-Methyl-2.4-pentanediol |
| F02 | | | | 0,1 | M | acétate de sodium trihydraté | 5 | 20 | %(v/v) | 2-Methl-2.4-pentanediol |
| F03 | | | | 0,1 | M | MES | 6 | 20 | %(v/v) | 2-Methyl-2.4-pentanediol |
| F04 | | | | 0,1 | M | HEPES | 7 | 20 | %(v/v) | 2-Methyl-2.4-pentanediol |
| F05 | | | | 0,1 | M | TRIS | 8 | 20 | %(v/v) | 2-Methyl-2.4-pentanediol |
| F06 | | | | 0,1 | M | bicine | 9 | 20 | %(v/v) | 2-Methyl-2.4-pentanediol |
| G01 | | | | 0,1 | M | acide citrique | 4 | 40 | %(v/v) | 2-Methyl-2.4-pentanediol |
| G02 | | | | 0,1 | M | acétate de sodium trihydraté | 5 | 40 | %(v/v) | 2-Methyl-2.4-pentanediol |
| G03 | | | | 0,1 | M | MES | 6 | 40 | %(v/v) | 2-Methyl-2.4-pentanediol |
| G04 | | | | 0,1 | M | HEPES | 7 | 40 | %(v/v) | 2-Methyl-2.4-pentanediol |
| G05 | | | | 0,1 | M | TRIS | 8 | 40 | %(v/v) | 2-Methyl-2.4-pentanediol |
| G06 | | | | 0,1 | M | bicine | 9 | 40 | %(v/v) | 2-Methyl-2.4-pentanediol |
| H01 | | | | 0,1 | M | acide citrique | 4 | 65 | %(v/v) | 2-Methyl-2.4-pentanediol |
| H02 | | | | 0,1 | M | acétate de sodium trihydraté | 5 | 65 | %(v/v) | 2-Methyl-2.4-pentanediol |
| H03 | | | | 0,1 | M | MES | 6 | 65 | %(v/v) | 2-Methyl-2.4-pentanediol |
| Well | C | U | Sel | C | U | Tampon | pH | C | U | Précipitant |
| H04 | | | | 0,1 | M | HEPES | 7 | 65 | %(v/v) | 2-Methyl-2,4-pentanediol |
| H05 | | | | 0,1 | M | TRIS | 8 | 65 | %(v/v) | 2-Methyl-2,4-pentanediol |
| H06 | | | | 0,1 | M | bicine | 9 | 65 | %(v/v) | 2-Methyl-2,4-pentanediol |
| A07 | 1 | M | chlorure de lithium | 0,1 | M | acide citrique | 4 | | | |
| A08 | 1 | M | chlorure de lithium | 0,1 | M | acide citrique | 5 | | | |
| A09 | 1 | M | chlorure de lithium | 0,1 | M | MES | 6 | | | |
| A10 | 1 | M | chlorure de lithium | 0,1 | M | HEPES | 7 | | | |
| A11 | 1 | M | chlorure de lithium | 0,1 | M | TRIS | 8 | | | |
| A12 | 1 | M | chlorure de lithium | 0,1 | M | bicine | 9 | | | |
| B07 | 1 | M | chlorure de lithium | 0,1 | M | acide citrique | 4 | 10 | %(w/v) | PEG 6000 |
| B08 | 1 | M | chlorure de lithium | 0,1 | M | acide citrique | 5 | 10 | %(w/v) | PEG 6000 |
| 809 | 1 | M | chlorure de lithium | 0,1 | M | MES | 6 | 10 | %(w/v) | PEG 6000 |
| B10 | 1 | M | chlorure de lithium | 0,1 | M | HEPES | 7 | 10 | %(w/v) | PEG 6000 |
| B11 | 1 | M | chlorure de lithium | 0,1 | M | TRIS | 8 | 10 | %(w/v) | PEG 6000 |
| B12 | 1 | M | chlorure de lithium | 0,1 | M | bicine | 9 | 10 | %(w/v) | PEG 6000 |
| C07 | 1 | M | chlorure de lithium | 0,1 | M | acide citrique | 4 | 20 | %(w/v) | PEG 6000 |
| C08 | 1 | M | chlorure de lithium | 0,1 | M | acide citrique | 5 | 20 | %(w/v) | PEG 6000 |
| C09 | 1 | M | chlorure de lithium | 0,1 | M | MES | 6 | 20 | %(w/v) | PEG 6000 |
| C10 | 1 | M | chlorure de lithium | 0,1 | M | HEPES | 7 | 20 | %(w/v) | PEG 6000 |
| C11 | 1 | M | chlorure de lithium | 0,1 | M | TRIS | 8 | 20 | %(w/v) | PEG 6000 |
| C12 | 1 | M | chlorure de lithium | 0,1 | M | bicine | 9 | 20 | %(w/v) | PEG 6000 |
| D07 | 1 | M | chlorure de lithium | 0,1 | M | acide citrique | 4 | 30 | %(w/v) | PEG 6000 |
| D08 | 1 | M | chlorure de lithium | 0,1 | M | acide citrique | 5 | 30 | %(w/v) | PEG 6000 |
| D09 | 1 | M | chlorure de lithium | 0,1 | M | MES | 6 | 30 | %(w/v) | PEG 6000 |
| D10 | 1 | M | chlorure de lithium | 0,1 | M | HEPES | 7 | 30 | %(w/v) | PEG 6000 |
| D11 | 1 | M | chlorure de lithium | 0,1 | M | TRIS | 8 | 30 | %(w/v) | PEG 6000 |
| D12 | 1 | M | chlorure de lithium | 0,1 | M | bicine | 9 | 30 | %(w/v) | PEG 6000 |
| E07 | | | | 0,1 | M | acide citrique | 4 | 5 | %(v/v) | PEG MME 5000 |
| E08 | | | | 0,1 | M | acide citrique | 5 | 5 | %(v/v) | PEG MME 5000 |
| E09 | | | | 0,1 | M | MES | 6 | 5 | %(v/v) | PEG MME 5000 |
| E10 | | | | 0,1 | M | HEPES | 7 | 5 | %(v/v) | PEG MME 5000 |
| E11 | | | | 0,1 | M | TRIS | 8 | 5 | %(v/v) | PEG MME 5000 |
| E12 | | | | 0,1 | M | bicine | 9 | 5 | %(v/v) | PEG MME 5000 |
| F07 | | | | 0,1 | M | acide citrique | 4 | 10 | %(v/v) | PEG MME 5000 |
| F08 | | | | 0,1 | M | acide citrique | 5 | 10 | %(v/v) | PEG MME 5000 |
| F09 | | | | 0,1 | M | MES | 6 | 10 | %(v(v) | PEG MME 5000 |
| F10 | | | | 0,1 | M | HEPES | 7 | 10 | %(v/v) | PEG MME 5000 |
| F11 | | | | 0,1 | M | TRIS | 8 | 10 | %(v/v) | PEG MME 5000 |
| F12 | | | | 0,1 | M | bicine | 9 | 10 | %(v/v) | PEG MME 5000 |
| G07 | | | | 0,1 | M | acide citrique | 4 | 15 | %(v/v) | PEG MME 5000 |
| G08 | | | | 0,1 | M | acide citrique | 5 | 15 | %(v/v) | PEG MME 5000 |
| G09 | | | | 0,1 | M | MES | 6 | 15 | %(v/v) | PEG MME 5000 |
| G10 | | | | 0,1 | M | HEPES | 7 | 15 | %(v/v) | PEG MME 5000 |
| G11 | | | | 0,1 | M | TRIS | 8 | 15 | %(v/v) | PEG MME 5000 |
| G12 | | | | 0,1 | M | bicine | 9 | 15 | %(v/v) | PEG MME 5000 |
| H07 | | | | 0,1 | M | acide citrique | 4 | 20 | %(v/v) | PEG MME 5000 |
| H08 | | | | 0,1 | M | acide citrique | 5 | 20 | %(v/v) | PEG MME 5000 |
| H09 | | | | 0,1 | M | MES | 6 | 20 | %(v/v) | PEG MME 5000 |
| H10 | | | | 0,1 | M | HEPES | 7 | 20 | %(v/v) | PEG MME 5000 |
| H11 | | | | 0,1 | M | TRIS | 8 | 20 | %(v/v) | PEG MME 5000 |
| H12 | | | | 0,1 | M | bicine | 9 | 20 | %(v/v) | PEG MME 5000 |

**Annexe 2A**

| Annexe plaque « Hampton 6 » | | | | | | | | | | | | | | | | | | | |
|---|---|---|---|---|---|---|---|---|---|---|---|---|---|---|---|---|---|---|---|
| Puit | C | U | Sel | C | U | Tampon | pH | C | U | Précipitant | | | | | | | | | |
| A01 | | | | 0,1 | M | acide citrique | 3,5 | 2 | M | sulfate d'ammonium | | | | | | | | | |
| A02 | | | | 0,1 | M | bis-tris | 5,5 | 3 | M | chlorure de sodium | | | | | | | | | |
| A03 | | | | | | | 5,6 | 1,4 | M | phosphate de potassium /sodium | | | | | | | | | |
| A04 | | | | | | | 7 | 3,5 | M | sodium formate pH 7.0 | | | | | | | | | |
| A05 | 1,1 | M | malonate de sodium pH 7.0 | 0,1 | M | HEPES | 7 | 0,5 | %(v/v) | jeffamine ED-2001 reagent pH 7.0 | | | | | | | | | |
| A06 | | | | 0,1 | M | acétate de sodium trihydraté | 4,5 | 25 | %(w/v) | PEG 3350 | | | | | | | | | |
| A07 | 0,2 | M | chlorure de calcium | 0,1 | M | bis-tris | 6,5 | 45 | %(v/v) | 2-methyl-2.4-pentanediol | | | | | | | | | |
| A08 | 0,05 | M | sulfate d'ammonium | 0,1 | M | bis-tris | 6,5 | 30 | %(v/v) | pentaerythritol ethoxylate (15/4 EO/OH) | | | | | | | | | |
| A09 | 0,1 | M | acétate d'ammonium | 0,1 | M | bis-tris | 5,5 | 17 | %(w/v) | PEG 10.000 | | | | | | | | | |
| A10 | 0,2 | M | chlorure de sodium | 0,1 | M | TRIS | 8,5 | 25 | %(w/v) | PEG 3350 | | | | | | | | | |
| A11 | 0,2 | M | acétate d'ammonium | 0,1 | M | TRIS | 8,5 | 25 | %(w/v) | PEG 3350 | | | | | | | | | |
| A12 | 0,1 | M | acide succinique pH 7.0 | | | | | 15 | %(w/v) | PEG 3350 | | | | | | | | | |
| B01 | | | | 0,1 | M | acétate de sodium trihydraté | 4,5 | 2 | M | sulfate d'ammonium | | | | | | | | | |
| B02 | | | | 0,1 | M | bis-tris | 6,5 | 3 | M | chlorure de sodium | | | | | | | | | |
| B03 | | | | | | | 6,9 | 1,4 | M | phosphate de potassium /sodium | | | | | | | | | |
| B04 | | | | | | | 7 | 1,1 | M | di-ammonium tartrate pH 7.0 | | | | | | | | | |
| B05 | 1 | M | acide succinique pH 7.0 | 0,1 | M | HEPES | 7 | 1 | %(w/v) | PEG MME 2000 | | | | | | | | | |
| B06 | | | | 0,1 | M | bis-tris | 5,5 | 25 | %(w/v) | PEG 3350 | | | | | | | | | |
| B07 | 0,2 | M | acétate d'ammonium | 0,1 | M | bis-tris | 5,5 | 45 | %(v/v) | 2-methyl-2.4-pentanediol | | | | | | | | | |
| B08 | | | | 0,1 | M | bis-tris | 6,5 | 45 | %(v/v) | polypropylene glycol P 400 | | | | | | | | | |
| B09 | 0,2 | M | sulfate d'ammonium | 0,1 | M | bis-tris | 5,5 | 25 | %(w/v) | PEG 3350 | | | | | | | | | |
| B10 | 0,2 | M | sulfate de lithium | 0,1 | M | bis-tris | 5,5 | 25 | %(w/v) | PEG 3350 | | | | | | | | | |
| B11 | 0,2 | M | chlorure de maqnesium | 0,1 | M | bis-tris | 5,5 | 25 | %(w/v) | PEG 3350 | | | | | | | | | |
| B12 | 0,2 | M | sodium formate | | | | | 20 | %(w/v) | PEG 3350 | | | | | | | | | |
| C01 | | | | 0,1 | M | bis-tris | 5,5 | 2 | M | sulfate d'ammonium | | | | | | | | | |
| C02 | | | | 0,1 | M | HEPES | 7,5 | 3 | M | chlorure de sodium | | | | | | | | | |
| C03 | | | | | | | 8,2 | 1,4 | M | phosphate de potassium /sodium | | | | | | | | | |
| C04 | | | | | | | 7 | 2,4 | M | malonate de sodium pH 7.0 | | | | | | | | | |
| C05 | 1 | M | sulfate d'ammonium | 0,1 | M | HEPES | 7 | 0,5 | %(w/v) | PEG 8000 | | | | | | | | | |
| C06 | | | | 0,1 | M | bis-tris | 6,5 | 25 | %(w/v) | PEG 3350 | | | | | | | | | |
| C07 | 0,2 | M | acétate d'ammonium | 0,1 | M | bis-tris | 6,5 5 | 45 | %(v/v) | 2-methyl-2,4-pentanediol | | | | | | | | | |
| C08 | 0,02 | M | chlorure de maqnesium | 0,1 | M | HEPES | 7,5 | 22 | %(w/v) | polyacrylic acid 5100 sodium salt | | | | | | | | | |
| C09 | 0,2 | M | sulfate d'ammonium | 0,1 | M | bis-tris | 6,5 | 25 | %(w/v) | PEG 3350 | | | | | | | | | |
| C10 | 0,2 | M | sulfate de lithium | 0,1 | M | bis-tris | 6,5 | 25 | %(w/v) | PEG 3350 | | | | | | | | | |
| C11 | 0,2 | M | chlorure de magnesium | 0,1 | M | bis-tris | 6,5 | 25 | %(w /v) | PEG 3350 | | | | | | | | | |
| C12 | 0,15 | M | DL-malic acid pH 7.0 | | | | | 20 | %(w/v) | PEG 3350 | | | | | | | | | |
| D01 | | | | 0,1 | M | bis-tris | 6,5 | 2 | M | sulfate d'ammonium | | | | | | | | | |
| D02 | | | | 0,1 | M | TRIS | 8,5 | 3 | M | chlorure de sodium | | | | | | | | | |
| D03 | | | | 0,1 | M | HEPES | 7,5 | 1,4 | M | tri-sodium citrate dihydrate | | | | | | | | | |
| D04 | | | | | | | 7 | 35 | %(v/v) | tacsimate pH 7.0 | | | | | | | | | |
| D05 | 15 | %(w/v) | tacsimate pH 7.0 | 0,1 | M | HEPES | 7 | 2 | %(w/v) | PEG 3350 | | | | | | | | | |
| D06 | D06 | | | 0,1 | M | HEPES | 7,5 | 25 | %(w/v) | PEG 3350 | | | | | | | | | |
| D07 | 0,2 | M | acétate d'ammonium | 0,1 | M | HEPES | 7,5 | 45 | %(v/v) | 2-methyl-2,4-pentanediol | | | | | | | | | |
| D08 | 0,1 | M | chlorure de cobalt | 0,1 | M | TRIS | 8,5 | 20 | %(w/v) | polyvinylpyrrolidone K15 | | | | | | | | | |
| D09 | 0,2 | M | sulfate d'ammonium | 0,1 | M | HEPES | 7,5 | 25 | %(w/v) | PEG 3350 | | | | | | | | | |

**Annexe 2A**

| Annexe plaque « Hampton 6 » | | | | | | | | | | | | | | | | | | | |
|---|---|---|---|---|---|---|---|---|---|---|---|---|---|---|---|---|---|---|---|
| Puit | C | U | Sel | C | U | Tampon | pH | C | U | Précipitant | C | U | Additif | C | U Additif_ 2 | | C | U | Additif_2 |
| D10 | 0,2 | M | sulfate de lithium | 0,1 | M | HEPES | 7,5 | 25 | %(w/v) | PEG 3350 | | | | | | | | | |
| D11 | 0,2 | M | chlorure de maqnesium | 0,1 | M | HEPES | 7,5 | 25 | %(w/v) | PEG 3350 | | | | | | | | | |
| D12 | 0,1 | M | formiate de magnesium | | | | | 15 | %(w/v) | PEG 3350 | | | | | | | | | |
| E01 | | | | 0,1 | M | HEPES | 7,5 | 2 | M | sulfate d'ammonium | | | | | | | | | |
| E02 | | | | 0,1 | M | bis-tris | 5,5 | 0,3 | M | formiate de magnésium | | | | | | | | | |
| E03 | | | | | | | 7 | 1,8 | M | citrate tri-ammonium pH 7.0 | | | | | | | | | |
| E04 | | | | | | | 7 | 60 | %(v/v) | tacsimate pH 7.0 | | | | | | | | | |
| E05 | | | | | | | | 25 | %(w/v) | PEG 1500 | | | | | | | | | |
| E06 | | | | 0,1 | M | TRIS | 8,5 | 25 | %(w/v) | PEG 3350 | | | | | | | | | |
| E07 | 0,2 | M | acétate d'ammonium | 0,1 | M | TRIS | 8,5 | 45 | %(v/v) | 2-methyl-2,4-pentanediol | | | | | | | | | |
| E08 | 0,2 | M | proline | 0,1 | M | HEPES | 7,5 | 10 | %(w/v) | PEG 3350 | | | | | | | | | |
| E09 | 0,2 | M | sulfate d'ammonium | 0,1 | M | TRIS | 8,5 | 25 | %(w/v) | PEG 3350 | | | | | | | | | |
| E10 | 0,2 | M | sulfate de lithium | 0,1 | M | TRIS | 8,5 | 25 | %(w/v) | PEG 3350 | | | | | | | | | |
| E11 | 0,2 | M | chlorure de maqnesium | 0,1 | M | TRIS | 8,5 | 25 | %(w/v) | PEG 3350 | | | | | | | | | |
| E12 | 0,05 | M | acétate de zinc | | | | | 20 | %(w/v) | PEG 3350 | | | | | | | | | |
| F01 | | | | 0,1 | M | TRIS | 8,5 | 2 | M | sulfate d'ammonium | | | | | | | | | |
| F02 | | | | 0,1 | M | bis-tris | 6,5 | 0,5 | M | formiate de magnesium | | | | | | | | | |
| F03 | | | | | | | 7 | 0,8 | M | acide succinique pH 7.0 | | | | | | | | | |
| F04 | 0,1 | M | chlorure de sodium | 0,1 | M | bis-tris | 6,5 | 1,5 | M | sulfate d'ammonium | | | | | | | | | |
| F05 | | | | 0,1 | M | HEPES | 7 | 30 | %(v/v) | jeffamine M-600 reagent pH 7.0 | | | | | | | | | |
| F06 | | | | 0,1 | M | bis-tris | 6,5 | 20 | %(w/v) | PEG MME 5000 | | | | | | | | | |
| F07 | 0,05 | M | chlorure de calcium | 0,1 | M | bis-tris | 6,5 | 30 | %(v/v) | PEG MME 550 | | | | | | | | | |
| F08 | 0,2 | M | trimethylamine n-oxide | 0,1 | M | TRIS | 8,5 | 20 | %(w/v) | PEG MME 2000 | | | | | | | | | |
| F09 | 0,2 | M | chlorure de sodium | 0,1 | M | bis-tris | 5,5 | 25 | %(w/v) | PEG 3350 | | | | | | | | | |
| F10 | 0,2 | M | acétate d'ammonium | 0,1 | M | bis-tris | 5,5 | 25 | %(w/v) | PEG 3350 | | | | | | | | | |
| F11 | 0,2 | M | potassium / tartrate de sodium | | | | | 20 | %(w/v) | PEG 3350 | | | | | | | | | |
| F12 | 0,2 | M | tri-sodium citrate | | | | | 20 | %(w/v) | PEG 3350 | | | | | | | | | |
| G01 | | | | 0,1 | M | acide citrique | 3,5 | 3 | M | chlorure de sodium | | | | | | | | | |
| G02 | | | | 0,1 | M | HEPES | 7,5 | 0,5 | M | formiate de magnesium | | | | | | | | | |
| G03 | | | | | | | 7 | 2,1 | M | DL-malic acid pH 7.0 | | | | | | | | | |
| G04 | 0,8 | M | potassium / tartrate de sodium | 0,1 | M | TRIS | 8,5 | 0,5 | %(w/v) | PEG MME 5000 | | | | | | | | | |
| G05 | | | | 0,1 | M | HEPES | 7 | 30 | %(v/v) | jeffamine ED-2001 reagent pH 7.0 | | | | | | | | | |
| G06 | | | | 0,1 | M | bis-tris | 6,5 | 28 | %(w/v) | PEG MME 2000 | | | | | | | | | |
| G07 | 0,05 | M | chlorure de magnesium | 0,1 | M | HEPES | 7,5 | 30 | %(v/v) | PEG MME 550 | | | | | | | | | |
| G08 | 5 | %(w /v) | tacsimate pH 7.0 | 0,1 | M | HEPES | 7 | 10 | %(w/v) | PEG MME 5000 | | | | | | | | | |
| G09 | 0,2 | M | chlorure de sodium | 0,1 | M | bis-tris | 6,5 | 25 | %(w/v) | PEG 3350 | | | | | | | | | |
| G10 | 0,2 | M | acétate d'ammonium | 0,1 | M | bis-tris | 6,5 | 25 | %(w/v) | PEG 3350 | | | | | | | | | |
| G11 | 0,2 | M | malonate de sodium pH 7.0 | | | | | 20 | %(w/v) | PEG 3350 | | | | | | | | | |
| G12 | 0,1 | M | thiocyanate de potassium | | | | | 30 | %(w/v) | PEG MME 2000 | | | | | | | | | |
| H01 | | | | 0,1 | M | acétate de sodium trihydrat é | 4,5 | 3 | M | chlorure de sodium | | | | | | | | | |
| H02 | | | | 0,1 | M | TRIS | 8,5 | 0,3 | M | formiate de magnésium | | | | | | | | | |
| H03 | | | | | | | 7 | 2,8 | M | acétate de sodium trihydraté pH 7.0 | | | | | | | | | |
| H04 | 1 | M | sulfate d'ammonium | 0,1 | M | bis-tris | 5,5 | 1 | %(w/v) | PEG 3350 | | | | | | | | | |
| H05 | | | | 0,1 | M | acide citrique | 3,5 | 25 | %(w/v) | PEG 3350 | | | | | | | | | |
| H06 | 0,2 | M | chlorure de calcium | 0,1 | M | bis-tris | 5,5 | 45 | %(v/v) | 2-methyl-2,4-pentanediol | | | | | | | | | |
| H07 | 0,2 | M | chlorure de potassium | 0,05 | M | HEPES | 7,5 | 30 | %(v/v) | pentaerythritol propoxylate (5/4 P0/0H) | | | | | | | | | |
| H08 | 0,005 | M | chlorure de magnesium | 0,1 | M | HEPES | 7,5 | 12 | %(w/v) | PEG 3350 | 0,005 | M | nickel (II) chloride | 0,005 | M | cadmium chloride | 0,005 | M | chlorure de cobalt |
| H09 | 0,2 | M | chlorure de sodium | 0,1 | M | HEPES | 7,5 | 25 | %(w/v) | PEG 3350 | | | | | | | | | |
| H10 | 0,2 | M | acétate d'ammonium | 0,1 | M | HEPES | 7,5 | 25 | %(w/v) | PEG 3350 | | | | | | | | | |
| H11 | 0,2 | M | citrate tri-ammonium pH 7,0 | | | | | 20 | %(w/v) | PEG 3350 | | | | | | | | | |
| H12 | 0,15 | M | bromure de potassium | | | | | 30 | %(w/v) | PEG MME 2000 | | | | | | | | | |

**ANNEXE 2B**

| Annexe plaque : « The JCSG » | | | | | | | | | | | | | | | | | | | |
|---|---|---|---|---|---|---|---|---|---|---|---|---|---|---|---|---|---|---|---|
| Puits | C | U | sel | C | U | Tampon | pH | C | U | Précipitant | C | U | Précipitant 2 | | | | | | |
| D10 | 0,2 | M | acétate de calcium | 0,1 | M | cacodylate de sodium | 6,5 | 40 | %(v/v) | PEG 300 | | | | | | | | | |
| D11 | 0,14 | M | chlorure de calcium | 0,07 | M | acétate de sodium | 4,6 | 14 | %(v/v) | isopropanol | 30 | %(v/v) | glycérol | | | | | | |
| D12 | 0,04 | M | phosphate de potassium | | | | | 16 | %(w/v) | PEG 8000 | 20 | %(v/v) | glycérol | | | | | | |
| E01 | 1 | M | citrate trisodique | 0,1 | M | cacodylate de sodium | 6,5 | | | 0 | | | | | | | | | |
| E02 | 0,2 | M | chlorure de sodium | 0,1 | M | cacodylate de sodium | 6,5 | 2 | M | sulfate d'ammonium | | | | | | | | | |
| E03 | 0,2 | M | chlorure de sodium | 0,1 | M | HEPES | 7,5 | 10 | %(v/v) | isopropanol | | | | | | | | | |
| E04 | 0,2 | M | sulfate de lithium | 0,1 | M | TRIS | 8,5 | 1,26 | M | sulfate d'ammonium | | | | | | | | | |
| E05 | | | 0 | 0,1 | M | CAPS | 10,5 | 40 | %(v/v) | MPD | | | | | | | | | |
| E06 | 0,2 | M | acétate de zinc | 0,1 | M | imidazole | 8 | 20 | %(w/v) | PEG 3000 | | | | | | | | | |
| E07 | 0,2 | M | acétate de zinc | 0,1 | M | cacodylate de sodium | 6,5 | 10 | %(v/v) isopropanol | | | | | | | | | | |
| E08 | 1 | M | di-phosphate d'ammonium | 0,1 | M | acétate de sodium | 4,5 | | | 0 | | | | | | | | | |
| E09 | 1,6 | M | sulfate de magnésium | 0,1 | M | MES | 6,5 | | | 0 | | | | | | | | | |
| E10 | | | 0 | 0,1 | M | bicine | 9 | 10 | %(w/v) | PEG 6000 | | | | | | | | | |
| E11 | 0,16 | M | acétate de calcium | 0,08 | M | cacodylate de sodium | 6,5 | 14,4 | %(w/v) | PEG 8000 | 20 | %(v/v) | glycérol | | | | | | |
| E12 | | | 0 | 0,1 | M | imidazole | 8 | 10 | %(w/v) | PEG 8000 | | | | | | | | | |
| F01 | 0,05 | M | cesium chloride | 0,1 | M | MES | 6,5 | 30 | %(w/v) | jeffamine M-600 | | | | | | | | | |
| F02 | 3,15 | M | sulfate d'ammonium | 0,1 | M | citrate trisodique | 5 | | | 0 | | | | | | | | | |
| F03 | | | 0 | 0,1 | M | TRIS | 8 | 20 | %(v/v) | MPD | | | | | | | | | |
| F04 | | | 0 | 0,1 | M | HEPES | 6,5 *20* | | %(w/v) | jeffamine M-600 | | | | | | | | | |
| F05 | 0,2 | M | chlorure de magnesium | 0,1 | M | TRIS | 8,5 50 | | %(v/v) | éthylène glycol | | | | | | | | | |
| F06 | | | 0 | 0,1 | M | bicine | 9 | 10 | %(v/v) | MPD | | | | | | | | | |
| F07 | 0,8 | M | acide succiniquepH 7.0 | | | | | | | 0 | | | | | | | | | |
| F08 | 2,1 | M | Acide DL-maliquepH 7.0 | | | | | | | 0 | | | | | | | | | |
| F09 | 2,4 | M | malonate de sodiumpH 7.0 | | | | | | | 0 | | | | | | | | | |
| F10 | 1,1 | M | malonate de sodium | 0,1 | M | HEPES | 7 | 0,5 | %(v/v) | jeffamine ED-2001 | | | | | | | | | |
| F11 | 1 | M | acide succinique | 0,1 | M | HEPES | 7 | 1 | %(w/v) | PEG MME 2000 | | | | | | | | | |
| F12 | | | 0 | 0,1 | M | HEPES | 7 | 30 | %(v/v) | jeffamine M-600 pH 7.0 | | | | | | | | | |
| G01 | | | 0 | 0,1 | M | HEPES | 7 | 30 | %(v/v) | jeffamine ED-2001 pH 7.0 | | | | | | | | | |
| G02 | 0,02 | M | chlorure de magnesium | 0,1 | M | HEPES | 7,5 | 22 | %(w/v) | sel de l'acide polyacrylique 5100 de sodium | | | | | | | | | |

| An nexe p laque : « The JC SG » | | | | | | | | | | | | | | | | | | | |
|---|---|---|---|---|---|---|---|---|---|---|---|---|---|---|---|---|---|---|---|
| G03 | 0,1 | M' | chlorure de cobalt | 0,1 | M | TRIS | 8,5 | 20 | %(w/v) | polyvinylpyrrolidone K15 | | | | | | | | | |
| G04 | 0,2 | M | trimethylamine N-oxide | 0,1 | M | TRIS | 8,5 | 20 | %(w/v) | PEG MME 2000 | | | | | | | | | |
| G05 | 0,005 | M | chlorure de cobalt | 0,1 | M | HEPES | 7,5 | 12 | %(w/v) | PEG 3350 | 0,005 | M | chlorure de cadmium | | | | | | |
| G06 | 0,24 | M | malonate de sodium pH 7.0 | | | | | 20 | %(w/v) | PEG 3350 | | | | | | | | | |
| 607 | 0,1 | M | acide succinique pH 7.0 | | | | | 15 | %(w/v) | PEG 3350 | | | | | | | | | |
| G08 | 0,15 | M | Acide DL-maliquepH 7.0 | | | | | 20 | %(w/v) | PEG 3350 | | | | | | | | | |
| G09 | 0,1 | M | thiocyanate de potassium | | | | | 30 | %w/v | PEG MME 2000 | | | | | | | | | |
| G10 | 0,15 | M | bromure de potassium | | | | | 30 | %(w/v) | PEG MME 2000 | | | | | | | | | |
| G11 | 2 | M | sulfate d'ammonium | 0,1 | M | bis-tris | 5,5 | | | 0 | | | | | | | | | |
| G12 | 3 | M | chlorure de sodium | 0,1 | M | bis-tris | 5,5 | | | 0 | | | | | | | | | |
| H01 | 0,3 | M | formiate de magnésium | 0,1 | M | bis-tris | 5,5 | | | 0 | | | | | | | | | |
| H02 | 1 | M | sulfate d'ammonium | 0,1 | M | bis-tris | 5,5 | 1 | %(w/v) | PEG 3350 | | | | | | | | | |
| H03 | | | 0 | 0,1 | M | bis-tris | 5,5 | 25 | %(w/v) | PEG 3350 | | | | | | | | | |
| H04 | 0,2 | M | chlorure de calcium | 0,1 | M | bis-tris | 5,5 | 45 | %(v/v) | MPD | | | | | | | | | |
| H05 | 0,2 | M | acétate d'ammonium | 0,1 | M | bis-tris | 5,5 | 45 | %(v/v) | MPD | | | | | | | | | |
| H06 | 0,1 | M | acétate d'ammonium | 0,1 | M | bis-tris | 5,5 | 17 | %(w/v) | PEG 10.000 | | | | | | | | | |
| H07 | 0,2 | M | sulfate d'ammonium | 0,1 | M | bis-tris | 5,5 | 25 | %(w/v) | PEG 3350 | | | | | | | | | |
| H08 | 0,2 | M | chlorure de sodium | 0,1 | M | bis-tris | 5,5 | 25 | %(w/v) | PEG 3350 | | | | | | | | | |
| H09 | 0,2 | M | sulfate de lithium | 0,1 | M | bis-tris | 5,5 | 25 | %(w/v) | PEG 3350 | | | | | | | | | |
| H10 | 0,2 | M | acétate d'ammonium | 0,1 | M | bis-tris | 5,5 | 25 | %(w/v) | PEG 3350 | | | | | | | | | |
| H11 | 0,2 | M | chlorure de magnésium | 0,1 | M | bis-tris | 5,5 | 25 | %(w/v) | PEG 3350 | | | | | | | | | |
| H12 | 0,2 | M | acétate d'ammonium | 0,1 | M | HEPES | 7,5 | 45 | %(v/v) | MPD | | | | | | | | | |

**ANNEXE 2B**

| Annexe : Plaque « The PACT » | | | | | | | | | | |
|---|---|---|---|---|---|---|---|---|---|---|
| Puits | C | U | Sel | C | U | Tampon | pH | C | U | Précipitant |
| A01 | | | | 0,1 | | tampon SPG | 4 | 25 | %(w/v) | PEG 1500 |
| A02 | | | | 0,1 | | tampon SPG | 5 | 25 | %(w/v) | PEG 1500 |
| A03 | | | | 0,1 | | tampon SPG | 6 | 25 | %(w/v) | PEG 1500 |
| A04 | | | | 0,1 | | tampon SPG | 7 | 25 | %(w/v) | PEG 1500 |
| A05 | | | | 0,1 | | tampon SPG | 8 | 25 | %(w/v) | PEG 1500 |
| A06 | | | | 0,1 | | tampon SPG | 9 | 25 | %(w/v) | PEG 1500 |
| A07 | 0,2 | | chlorure de sodium | 0,1 | | acétate de sodium | 5 | 20 | %(w/v) | PEG 6000 |
| A08 | 0,2 | | chlorure d'ammonium | 0,1 | | acétate de sodium | 5 | 20 | %(w/v) | PEG 6000 |
| A09 | 0,2 | | chlorure de lithium | 0,1 | | acétate de sodium | 5 | 20 | %(w/v) | PEG 6000 |
| A10 | 0,2 | | chlorure de magnesium | 0,1 | | acétate de sodium | 5 | 20 | %(w/v) | PEG 6000 |
| A11 | 0,2 | | chlorure de calcium | 0,1 | | acétate de sodium | 5 | 20 | %(w/v) | PEG 6000 |
| A12 | 0,01 | | chlorure de zinc | 0,1 | | acétate de sodium | 5 | 20 | %(w/v) | PEG 6000 |
| B01 | | | | 0,1 | | tampon MIB | 4 | 25 | %(w/v) | PEG 1500 |
| B02 | | | | 0,1 | | tampon MIB | 5 | 25 | %(w/v) | PEG 1500 |
| B03 | | | | 0,1 | | tampon MIB | 6 | 25 | %(w/v) | PEG 1500 |
| B04 | | | | 0,1 | | tampon MIB | 7 | 25 | %(w/v) | PEG 1500 |
| B05 | | | | 0,1 | | tampon MIB | 8 | 25 | %(w/v) | PEG 1500 |
| B06 | | | | 0,1 | | tampon MIB | 9 | 25 | %(w/v) | PEG 1500 |
| 807 | 0,2 | | chlorure de sodium | 0,1 | | MES | 6 | 20 | %(w/v) | PEG 6000 |
| B08 | 0,2 | | chlorure d'ammonium | 0,1 | | MES | 6 | 20 | %(w/v) | PEG 6000 |
| B09 | 0,2 | | chlorure de lithium | 0,1 | | MES | 6 | 20 | %(w/v) | PEG 6000 |
| B10 | 0,2 | | chlorure de magnesium | 0,1 | | MES | 6 | 20 | %(w/v) | PEG 6000 |
| B11 | 0,2 | | chlorure de calcium | 0,1 | | MES | 6 | 20 | %(w/v) | PEG 6000 |
| B12 | 0,01 | | chlorure de zinc | 0,1 | | MES | 6 | 20 | %(w/v) | PEG 6000 |
| C01 | | | | 0,1 | | tampon PCB | 4 | 25 | %(w/v) | PEG 1500 |
| C02 | | | | 0,1 | | tampon PCB | 5 | 25 | %(w/v) | PEG 1500 |
| C03 | | | | 0,1 | | tampon PCB | 6 | 25 | %(w/v) | PEG 1500 |
| C04 | | | | 0,1 | | tampon PCB | 7 | 25 | %(w/v) | PEG 1500 |
| C05 | | | | 0,1 | | tampon PCB | 8 | 25 | %(w/v) | PEG 1500 |
| C06 | | | | 0,1 | | tampon PCB | 9 | 25 | %(w/v) | PEG 1500 |
| C07 | 0,2 | | chlorure de sodium | 0,1 | | HEPES | 7 | 20 | %(w/v) | PEG 6000 |
| C08 | 0,2 | | chlorure d'ammonium | 0,1 | | HEPES | 7 | 20 | %(w/v) | PEG 6000 |
| C09 | 0,2 | | chlorure de lithium | 0,1 | | HEPES | 7 | 20 | %(w/v) | PEG 6000 |
| C10 | 0,2 | | chlorure de maqnesium | 0,1 | | HEPES | 7 | 20 | %(w/v) | PEG 6000 |
| C11 | 0,2 | | chlorure de calcium | 0,1 | | HEPES | 7 | 20 | %(w/v) | PEG 6000 |
| C12 | 0,01 | | chlorure de zinc | 0,1 | | HEPES | 7 | 20 | %(w/v) | PEG 6000 |
| D01 | | | | 0,1 | | tampon MMT | 4 | 25 | %(w/v) | PEG 1500 |
| D02 | | | | 0,1 | | tampon MMT | 5 | 25 | %(w/v) | PEG 1500 |
| D03 | | | | 0,1 | | tampon MMT | 6 | 25 | %(w/v) | PEG 1500 |
| D04 | | | | 0,1 | | tampon MMT | 7 | 25 | %(w/v) | PEG 1500 |
| D05 | | | | 0,1 | | tampon MMT | 8 | 25 | %(w/v) | PEG 1500 |
| D06 | | | | 0,1 | | tampon MMT | 9 | 25 | %(w/v) | PEG 1500 |
| D07 | 0,2 | | chlorure de sodium | 0,1 | | TRIS | 8 | 20 | %(w/v) | PEG 6000 |
| D08 | 0,2 | | chlorure d'ammonium | 0,1 | | TRIS | 8 | 20 | %(w/v) | PEG 6000 |
| D09 | 0,2 | | chlorure de lithium | 0,1 | | TRIS | 8 | 20 | %(w/v) | PEG 6000 |
| F Puits ( | C | U | Sel C | C | U | Tampon | pH | C | U | Précipitant |
| D10 | 0,2 | | chlorure de magnésium | 0,1 | | TRIS | 8 | 20 | %(w/v) | PEG 6000 |
| D11 | 0,2 | | chlorure de calcium | 0,1 | | TRIS | 8 | 20 | %(w/v) | PEG 6000 |
| D12 | 0,01 | | chlorure de zinc | 0,1 | | TRIS | 8 | 20 | %(w/v) | PEG 6000 |
| E01 | 0,2 | | fluorure de sodium | | | | | 20 | %(w/v) | PEG 3350 |
| E02 | 0,2 | | bromure de sodium | | | | | 20 | %(w/v) | PEG 3350 |
| E03 | 0,2 | | iodure de sodium | | | | | 20 | %(w/v) | PEG 3350 |
| E04 | 0,2 | | thiocyanate de potassium | | | | | 20 | %(w/v) | PEG 3350 |
| E05 | 0,2 | | nitrate de sodium | | | | | 20 | %(w/v) | PEG 3350 |
| E06 | 0,2 | | formiate de sodium | | | | | 20 | %(w/v) | PEG 3350 |
| E07 | 0,2 | | acétate de sodium | | | | | 20 | %(w/v) | PEG 3350 |
| E08 | 0,2 | | sulfate de sodium | | | | | 20 | %(w/v) | PEG 3350 |
| E09 | 0,2 | | potassium / tartrate de sodium | | | | | 20 | %(w/v) | PEG 3350 |
| E10 | 0,2 | | sodium / phosphate de potassium | | | | | 20 | %(w/v) | PEG 3350 |
| E11 | 0,2 | | citrate de sodium | | | | | 20 | %(w/v) | PEG 3350 |
| E12 | 0,2 | | sodium malonate | | | | | 20 | %(w/v) | PEG 3350 |
| F01 | 0,2 | | fluorure de sodium | 0,1 | | bis-tris propane | 6,5 | 20 | %(w/v) | PEG 3350 |
| F02 | 0,2 | | bromure de sodium | 0,1 | | bis-tris propane | 6,5 | 20 | %(w/v) | PEG 3350 |
| F03 | 0,2 | | iodure de sodium | 0,1 | | bis-tris propane | 6,5 | 20 | %(w/v) | PEG 3350 |
| F04 | 0,2 | | thiocyanate de potassium | 0,1 | | bis-tris propane | 6,5 | 20 | %(w/v) | PEG 3350 |
| F05 | 0,2 | | nitrate de sodium | 0,1 | | bis-tris propane | 6,5 | 20 | %(w/v) | PEG 3350 |
| F06 | 0,2 | | formiate de sodium | 0,1 | | bis-tris propane | 6,5 | 20 | %(w/v) | PEG 3350 |
| F07 | 0,2 | | acétate de sodium | 0,1 | | bis-tris propane | 6,5 | 20 | %(w/v) | PEG 3350 |
| F08 | 0,2 | | sulfate de sodium | 0.1 | | bis-tris propane | 6,5 | 20 | %(w/v) | PEG 3350 |
| F09 | 0,2 | | potassium / tartrate de sodium | 0,1 | | bis-tris propane | 6,5 | 20 | %(w/v) | PEG 3350 |
| F10 | 0,2 | | sodium / phosphate de potassium | 0,1 | | bis-tris propane | 6,5 | 20 | %(w/v) | PEG 3350 |
| F11 | 0,2 | | citrate de sodium | 0,1 | | bis-tris propane | 6,5 | 20 | %(w/v) | PEG 3350 |
| F12 | 0,2 | | malonate de sodium | 0,1 | | bis-tris propane | 6,5 | 20 | %(w/v) | PEG 3350 |
| G01 | 0,2 | | fluorure de sodium | 0,1 | | bis-tris propane | 7,5 | 20 | %(w/v) | PEG 3350 |
| G02 | 0,2 | | bromure de sodium | 0,1 | | bis-tris propane | 7,5 | 20 | %(w/v) | PEG 3350 |
| G03 | 0,2 | | iodure de sodium | 0,1 | | bis-tris propane | 7,5 | 20 | %(w/v) | PEG 3350 |
| G04 | 0,2 | | thiocyanate de potassium | 0,1 | | bis-tris propane | 7,5 | 20 | %(wlv) | PEG 3350 |
| G05 | 0,2 | | nitrate de sodium | 0,1 | | bis-tris propane | 7,5 | 20 | %(w/v) | PEG 3350 |
| G06 | 0,2 | | formiate de sodium | 0,1 | | bis-tris propane | 7,5 | 20 | %(w/v) | PEG 3350 |
| G07 | 0,2 | | acétate de sodium | 0,1 | | bis-tris propane | 7,5 | 20 | %(w/v) | PEG 3350 |
| G08 | 0,2 | | sulfate de sodium | 0,1 | | bis-tris propane | 7,5 | 20 | %(w/v) | PEG 3350 |
| G09 | 0,2 | | potassium/sodium tartarte | 0,1 | | bis-tris propane | 7,5 | 20 | %(w/v) | PEG 3350 |
| G10 | 0,2 | | sodium / phosphate de potassium | 0,1 | | bis-tris propane | 7,5 | 20 | %(w/v) | PEG 3350 |
| G11 | 0,2 | | citrate de sodium | 0,1 | | bis-tris propane | 7,5 | 20 | %(w/v) | PEG 3350 |
| G12 | 0,2 | | malonate de sodium | 0,1 | | bis-tris propane | 7,5 | 20 | %(w/v) | PEG 3350 |
| H01 | 0,2 | | fluorure de sodium | 0,1 | | bis-tris propane | 8,5 | 20 | %(w/v) | PEG 3350 |
| H02 | 0,2 | | bromure de sodium | 0,1 | | bis-tris propane | 8,5 | 20 | %(w/v) | PEG 3350 |
| H03 | 0,2 | | iodure de sodium | 0,1 | | bis-tris propane | 8,5 | 20 | %(w/v) | PEG 3350 |
| H04 | 0,2 | | thiocyanate de potassium | 0,1 | | bis-tris propane | 8,5 | 20 | %(w/v) | PEG 3350 |
| H05 | 0,2 | | nitrate de sodium | 0,1 | | bis-tris propane | 8,5 | 20 | %(w/v) | PEG 3350 |
| H06 | 0,2 | | formiate de sodium | 0,1 | | bis-tris propane | 8,5 | 20 | %(w/v) | PEG 3350 |
| H07 | 0,2 | | acétate de sodium | 0,1 | | bis-tris propane | 8,5 | 20 | %(w/v) | PEG 3350 |
| H08 | 0,2 | | sulfate de sodium | 0,1 | | bis-tris propane | 8,5 | 20 | %(w/v) | PEG 3350 |
| H09 | 0,2 | | potassium / tartrate de sodium | 0,1 | | bis-tris propane | 8,5 | 20 | %(w/v) | PEG 3350 |
| H10 | 0,2 | | sodium / phosphate de potassium | 0,1 | | bis-tris propane | 8,5 | 20 | %(w/v) | PEG 3350 |
| H11 | 0,2 | | citrate de sodium | 0,1 | | bis-tris propane | 8,5 | 20 | %(w/v) | PEG 3350 |
| H12 | 0,2 | | sodium malonate | 0,1 | | bis-tris propane | 8,5 | 20 | %(w/v) | PEG 3350 |

**ANNEXE 2B**

| Annexe : Plaque « Classic suite » Quiagen | | | | | | | | | | | | | |
|---|---|---|---|---|---|---|---|---|---|---|---|---|---|
| Puits | C | U | Sel | C | U | Tampon | pH | C | U | Précipitant | C | U | Additif |
| A03 | 0,2 | M | chlorure de magnésium | 0,1 | M | TRIS | 8,5 | 3,4 | M | 1,6-hexanediol | | | |
| A02 | | | | 0,1 | M | citrate trisodique | 5,6 | 2,5 | M | 1,6-hexanediol | | | |
| A01 | 0,01 | M | chlorure de cobalt | 0,1 | M | acétate de sodium | 4,6 | 1 | M | 1.6-hexanediol | | | |
| C04 | | | | 0,1 | M | HEPES | 7,5 | 2 | M | ammonium formate | | | |
| C03 | | | | 0,1 | M | TRIS HCl | 8,5 | 2 | M | phosphate d'ammonium | | | |
| C02 | | | | 0,1 | M | citrate trisodique | 5,6 | 1 | M | phosphate d'ammonium | | | |
| C01 | | | | | | | | 0,4 | M | phosphate d'ammonium | | | |
| C08 | 0,1 | M | chlorure de sodium | 0,1 | M | HEPES | 7,5 | 1,6 | M | sulfate d'ammonium | | | |
| C09 | 0,01 | M | chlorure de cobalt | 0,1 | M | MES | 6,5 | 1,8 M | M | sulfate d'ammonium | | | |
| C05 | | | | 0,1 | M | acétate de sodium | 4,6 | 2 | M | sulfate d'ammonium | | | |
| C06 | | | | 0,1 | M | TRIS HCl | 8,6 | 2 | M | sulfate d'ammonium | | | |
| C10 | 0,2 | M | potassium / tartrate de sodium | 0,1 | M | citrate trisodique | 5,6 | 2 | M | sulfate d'ammonium | | | |
| C07 | | | | | | | | 2 | M | sulfate d'ammonium | | | |
| E06 | 0,5 | M | chlorure de sodium | | | | | 0,01 | M | CTAB | 0,01 | M | chlorure de magnesium |
| E02 | | | | 0,1 | M | MES | 6,5 | 10 | %(v/v) | dioxane | 1,6 | M | sulfate d'ammonium |
| E03 | | | | | | | | 35 | %(v/v) | dioxane | | | |
| E01 | | | | 0,1 | M | bicine | 9 | 2 | %(w/v) | dioxane | 10 | %(w/v) | PEG 20000 |
| A12 | | | | | | | | 10 | % (v/v) | ethanol | 1,5 | M | chlorure de sodium |
| B01 | | | | 0,1 | M | TRIS | 8,5 | 20 | %(v/v) | ethanol | | | |
| B02 | | | | | | | | 25 | %(v/v) | éthylène glycol | | | |
| E04 | 0,5 | M | chlorure de sodium | 0,1 | M | citrate trisodique | 5,6 | 2 | %(v/v) | polymère d'éthylène-imine | | | |
| E05 | | | | 0,1 | M | TRIS | 8,5 | 12 | %(v/v) | glycérol | 1,5 | M | sulfate d'ammonium |
| C11 | | | | | | | | 1 | M | imidazole pH 7.0 | | | |
| A10 | 0,2 | M | chlorure de magnesium | 0,1 | M | Sel de sodium d'HEPES | 7,5 | 30 | %(v/v) | isopropanol | | | |
| A08 | 0,2 | M | citrate trisodique | 0,1 | M | Sel de sodium d'HEPES | 7,5 | 20 | %(v/v) | isopropanol | | | |
| A05 | | | | 0,1 | M | Sel de sodium d'HEPES | 7,5 | 10 | %(v/v) | isopropanol | 20 | %(w/v) | PEG 4000 |
| A06 | 0,2 | M | chlorure de calcium | 0,1 | M | acétate de sodium | 4,6 | 20 | %(v/v) | isopropanol | | | |
| A09 | 0,2 | M | citrate trisodique | 0,1 | | cacodylate de sodium | 6,5 | 30 | %(v/v) | isopropanol | | | |
| A11 | 0,2 | M | acétate d'ammonium | 0,1 | M | TRIS HCl | 8,5 | 30 | %(v/v) | isopropanol | | | |
| A07 | | | | 0,1 | M | citrate trisodique | 5,6 | 20 | %(v/v) | isopropanol | 20 | %(w/v) | PEG 4000 |
| A04 | | | | | | | | 5 | %(v/v) | isopropanol | 2 | M | sulfate d'ammonium |
| E08 | | | | 0,1 | M | HEPES | 7,5 | 20 | %(v/v) | jeffamine M-600 | | | |
| E07 | 0,01 | M | chlorure ferrique | 0,1 | M | citrate trisodique | 5,6 | 10 | %(v/v) | jeffamine M-600 | | | |
| D01 | | | | 0,1 | M | Sel de sodium d'HEPES | 7,5 | 0,8 | M | potassium / tartrate de sodium | | | |
| C12 | | | | | | | | 0,4 | M | potassium / tartrate de sodium | | | |
| E11 | | | | 0,1 | M | Sel de sodium d'HEPES | 7,5 | 1,5 | M | sulfate de lithium | | | |
| E10 | 0,01 | M | chlorure de nickel | 0,1 | M | TRIS | 8,5 | 1 | M | sulfate de lithium | | | |
| E09 | 0,5 | M | sulfate d'ammonium | 0,1 | M | citrate trisodique | 5,6 | 1 | M | sulfate de lithium | | | |
| E12 | | | | 0,1 | M | bicine | 9 | 2 | M | chlorure de magnesium | | | |
| F01 | | | | | | | | 0,2 | M | formiate de magnésium | | | |
| F02 | | | | 0,1 | M | MES | 6,5 | 1,6 | M | sulfate de magnésium | | | |
| B10 | | | | 0,1 | M | HEPES | 7,5 | 70 | %(v/v) | MPD | | | |
| B07 | 0,2 | M | citrate trisodique | 0,1 | M | Sel de sodium d'HEPES | 7,5 | 30 | %(v/v) | MPD | | | |
| B03 | 0,02 | M | chlorure de calcium | 0,1 | M | acétate de sodium | 4,6 | 30 | %(v/v) | MPD | | | |
| B04 | 0,2 | M | chlorure de sodium | 0,1 | M | acétate de sodium | 4,6 | 30 | %(v/v) | MPD | | | |
| Puits | C | U | Sel | C | U | Tampon | pH | C | U | Précipitant | C | U | Additif |
| B09 | 0,2 | M | phosphate d'ammonium | 0,1 | M | TRIS | 8,5 | 50 | %(v/v) | MPD | | | |
| B05 | 0,2 | M | acétate d'ammonium | 0,1 | M | citrate trisodique | 5,6 | 30 | %(v/v) | MPD | | | |
| B08 | 0,5 | M | sulfate d'ammonium | 0,1 | M | HEPES | 7,5 | 30 | %(v/v) | MPD | | | |
| B06 | 0,2 | M | acétate de magnésium | 0,1 | M | cacodylate de sodium | 6,5 | 30 | %(v/v) | MPD | | | |
| G08 | | | | | | | | 10 | %(w/v) | PEG 1000 | 10 | %(w/v) | PEG 8000 |
| H11 | | | | 0,1 | M | HEPES | 7,5 | 20 | %(w/v) | PEG 10000 | 8 | %(v/v) | ethylene glycol |
| G09 | | | | | | | | 30 | %(w/v) | PEG 1500 | | | |
| G11 | 0,2 | M | sulfate d'ammonium | 0,1 | M | acétate de sodium | 4,6 | 30 | %(w/v) | PEG 2000 MME | | | |
| G10 | 0,01 | M | chlorure de nickel | 0,1 | M | TRIS | 8,5 | 20 | %(w/v) | PEG 2000 MME | | | |
| H12 | | | | 0,1 | M | MES | 6,5 | 12 | %(w/v) | PEG 20000 | | | |
| G02 | 0,2 | M | chlorure de calcium | 0,1 | M | Sel de sodium d'HEPES | 7,5 | 28 | %(v/v) | PEG 400 | | | |
| G04 | 0,2 | M | chlorure de magnesium | 0,1 | M | Sel de sodium d'HEPES | 7,5 | 30 | %(v/v) | PEG 400 | | | |
| G05 | 0,2 | M | citrate trisodique | 0,1 | M | TRIS HCl | 8,5 | 30 | %(v/v) | PEG 400 | | | |
| G01 | | | | 0,1 | M | Sel de sodium d'HEPES | 7,5 | 2 | %(v/v) | PEG 400 | 2 | M | Sulfate d'ammonium |
| G03 | 0,1 | M | chlorure de cadmium | 0,1 | M | acétate de sodium | 4,6 | 30 | %(v/v) | PEG 400 | | | |
| H02 | 0,2 | M. | acétate d'ammonium | 0,1 | M | acétate de sodium | 4,6 | 30 | %(w/v) | PEG 4000 | | | |
| H01 | 0,2 | M | sulfate d'ammonium | 0,1 | M | acétate de sodium | 4,6 | 25 | %(w/v) | PEG 4000 | | | |
| H05 | 0,2 | M | sulfate de lithium | 0,1 | M | TRIS HCl | 8,5 | 30 | %(w/v) | PEG 4000 | | | |
| H06 | 0,2 | M | acétate de sodium | 0,1 | M | TRIS HCl | 8,5 | 30 | %(w/v) | PEG 4000 | | | |
| H03 | 0,2 | M | acétate d'ammonium | 0,1 | M | citrate trisodique | 5,6 | 30 | %(w/v) | PEG 4000 | | | |
| H07 | 0,2 | M | sulfate d'ammonium | | | | | 30 | %(w/v) | PEG 4000 | | | |
| G12 | | | | 0,1 | M | acétate de sodium | 4,6 | 8 | %(w/v) | PEG 4000 | | | |
| H04 | 0,2 | M | chlorure de magnesium | 0,1 | M | TRIS HCl | 8,5 | 30 | %(w/v) | PEG 4000 | | | |
| H08 | 0,2 | M | sulfate d'ammonium | 0,1 | M | MES | 6,5 | 30 | %(w/v) | PEG 5000 MME | | | |
| G06 | 0,1 | M | chlorure de sodium | 0,1 | M | bicine | 9 | 20 | %(w/v) | PEG 550 MME | | | |
| G07 | 0,01 | M | sulfate de zinc | 0,1 | M | MES | 6,5 | 25 | %(w/v) | PEG 550 MME | | | |
| H09 | | | | 0,1 | M | HEPES | 7,5 | 10 | %(w/v) | PEG 6000 | 5 | %(v/v) | MPD |
| H10 | | | | | | | | 10 | %(w/v) | PEG 6000 | 2 | M | chlorure de sodium |
| F04 | | | | 0,1 | M | HEPES | 7,5 | 10 | %(w/v) | PEG 8000 | | | |
| F06 | 0,2 | M | zinc acetate | 0,1 | M | cacodylate de sodium | 6,5 | 18 | %(w/v) | PEG 8000 | | | |
| F10 | 0,2 | M | sulfate d'ammonium | 0,1 | M | cacodylate de sodium | 6,5 | 30 | %(w/v) | PEG 8000 | | | |
| F07 | 0,2 | M | calcium acetate | 0,1 | M | cacodylate de sodium | 6,5 | 18 | %(w/v) | PEG 8000 | | | |
| F08 | 0,2 | M | acétate de magnésium | 0,1 | M | cacodylate de sodium | 6,5 | 20 | %(w/v) | PEG 8000 | | | |
| F11 | 0,2 | | acétate de sodium | 0,1 | M | cacodylate de sodium | 6,5 | 30 | %(w/v) | PEG 8000 | | | |
| F12 | 0,2 | M | sulfate d'ammonium | | | | | 30 | %(w/v) | PEG 8000 | | | |
| F05 | 0,5 | M | sulfate de lithium | | | | | 15 | %(w/v) | PEG 8000 | | | |
| F09 | 0,05 | M | phosphate de potassium | | | | | 20 | %(w/v) | PEG 8000 | | | |
| F03 | | | | 0,1 | M | TRIS HCl | 8,5 | 8 | %(w/v) | PEG 8000 | | | |
| D03 | 0,05 | M | sulfate de cadmium | 0,1 | M | HEPES | 7,5 | 1 | M | acétate de sodium | | | |
| D02 | | | | 0,1 | M | imidazole | 6,5 | 1 | M | acétate de sodium | | | |
| D04 | | | | 0,1 | M | cacodylate de sodium | 6,5 | 1,4 | M | acétate de sodium | | | |
| D07 | | | | 0,1 | M | HEPES | 7,5 | 4,3 | M | chlorure de sodium | | | |
| D06 | 0,1 | M | phosphate de sodium | 0,1 | M | MES | 6,5¹ | 2 | M | chlorure de sodium | 0,1 | M | phosphate de potassium |
| D05 | | | | 0,1 | M | acétate de sodium | 4,6 | 2 | M | chlorure de sodium | | | |
| D11 | | | | 0,1 | M | acétate de sodium | 4,6 | 2 | M | sodium formate | | | |
| D12 | | | | | | | | 4 | M | sodium formate | | | |
| D10 | | | | 0,1 | M | Sel de sodium d'HEPES | 7,5 | 0,8 | M | phosphate de sodium | 0,8 | M | phosphate de potassium |
| B11 | | | | 0,1 | M | TRIS | 8,5 | 25 | %(v/v) | tert-butanol | | | |
| B12 | | | | 0,1 | M | citrate trisodique | 5,6 | 35 | %(v/v) | tert-butanol | | | |
| D08 | | | | 0,1 | M | Sel de sodium d'HEPES | 7,5 | 1,4 | M | citrate trisodique | | | |
| D09 | | | | | | | | 1,6 | M | citrate trisodique pH 6.5 | | | |

**ANNEXE 2B**

| Annexe plaque : « The PEGs » | | | | | | | | | | |
|---|---|---|---|---|---|---|---|---|---|---|
| Puits | C | U | Sel | C | U | Tampon | pH | C | U | Précipitant |
| A01 | 0 | | 0 | 0,1 | M | acétate de sodium | 4,6 | 40 | %(v/v) | PEG 200 |
| A02 | 0 | | 0 | 0,1 | M | acétate de sodium | 4,6 | 30 | %(v/v) | PEG 300 |
| A03 | 0 | | 0 | 0,1 | M | acétate de sodium | 4,6 | 30 | %(v/v) | PEG 400 |
| A04 | 0 | | 0 | 0,1 | M | acétate de sodium | 4,6 | 25 | %(v/v) | PEG 550 MME |
| A05 | 0 | | 0 | 0,1 | M | acétate de sodium | 4,6 | 25 | %(w/v) | PEG 1000 |
| A06 | 0 | | 0 | 0,1 | M | acétate de sodium | 4,6 | 25 | %(w/v) | PEG 2000 MME |
| A07 | 0 | | 0 | 0,1 | M | MES | 6,5 | 40 | %(v/v) | PEG 200 |
| A08 | 0 | | 0 | 0,1 | M | MES | 6,5 | 30 | %(v/v) | PEG 300 |
| A09 | 0 | | 0 | 0,1 | M | MES | 6,5 | 30 | %(v/v) | PEG 400 |
| A10 | 0 | | 0 | 0,1 | M | MES | 6,5 | 25 | %(v/v) | PEG 550 MME |
| A11 | 0 | | 0 | 0,1 | M | MES | 6,5 | 25 | %(w/v) | PEG 1000 |
| A12 | 0 | | 0 | 0,1 | M | MES | 6,5 | 25 | %(w/v) | PEG 2000 MME |
| B01 | 0 | | 0 | 0,1 | M | Sel de sodium d'HEPES | 7,5 | 40 | %(v/v) | PEG 200 |
| B02 | 0 | | 0 | 0,1 | M | Sel de sodium d'HEPES | 7,5 | 30 | %(v/v) | PEG 300 |
| B03 | 0 | | 0 | 0,1 | M | Sel de sodium d'HEPES | 7,5 | 30 | %(v/v) | PEG 400 |
| B04 | 0 | | 0 | 0,1 | M | Sel de sodium d'HEPES | 7,5 | 25 | %(v/v) | PEG 550 MME |
| B05 | 0 | | 0 | 0,1 | M | Sel de sodium d'HEPES | 7,5 | 25 | %(w/v) | PEG 1000 |
| B06 | 0 | | 0 | 0,1 | M | Sel de sodium d'HEPES | 7,5 | 25 | %(w/v) | PEG 2000 MME |
| B07 | 0 | | 0 | 0,1 | M | TRIS HCl | 8,5 | 40 | %(v/v) | PEG 200 |
| B08 | 0 | | 0 | 0,1 | M | TRIS HCl | 8,5 | 30 | %(v/v) | PEG 300 |
| B09 | 0 | | 0 | 0,1 | M | TRIS HCl | 8,5 | 30 | %(v/v) | PEG 400 |
| B10 | 0 | | 0 | 0,1 | M | TRIS HCl | 8,5 | 25 | %(v/v) | PEG 550 MME |
| B11 | 0 | | 0 | 0,1 | M | TRIS HCl | 8,5 | 25 | %(w/v) | PEG 1000 |
| B12 | 0 | | 0 | 0,1 | M | TRIS HCl | 8,5 | 25 | %(w/v) | PEG 2000 MME |
| C01 | 0 | | 0 | 0,1 | M | acétate de sodium | 4,6 | 25 | %(w/v) | PEG 3000 |
| C02 | 0 | | 0 | 0,1 | M | acétate de sodium | 4,6 | 25 | %(w/v) | PEG 4000 |
| C03 | 0 | | 0 | 0,1 | M | acétate de sodium | 4,6 | 25 | %(w/v) | PEG 6000 |
| C04 | 0 | | 0 | 0,1 | M | acétate de sodium | 4,6 | 25 | %(w/v) | PEG 8000 |
| C05 | 0 | | 0 | 0,1 | M | acétate de sodium | 4,6 | 20 | %(w/v) | PEG 10000 |
| C06 | 0 | | 0 | 0,1 | M | acétate de sodium | 4,6 | 15 | %(w/v) | PEG 20000 |
| C07 | 0 | | 0 | 0,1 | M | MES | 6,5 | 25 | %(w/v) | PEG 3000 |
| C08 | 0 | | 0 | 0,1 | M | MES | 6,5 | 25 | %(w/v) | PEG 4000 |
| C09 | 0 | | 0 | 0,1 | M | MES | 6,5 | 25 | %(w/v) | PEG 6000 |
| C10 | 0 | | 0 | 0,1 | M | MES | 6,5 | 25 | %(w/v) | PEG 8000 |
| C11 | 0 | | 0 | 0,1 | M | MES | 6,5 | 20 | %(w/v) | PEG 10000 |
| C12 | 0 | | 0 | 0,1 | M | MES | 6,5 | 15 | %(w/v) | PEG 20000 |
| D01 | 0 | | 0 | 0,1 | M | Sel de sodium d'HEPES | 7,5 | 25 | %(w/v) | PEG 3000 |
| D02 | 0 | | 0 | 0,1 | M | Sel de sodium d'HEPES | 7,5 | 25 | %(w/v) | PEG 4000 |
| D03 | 0 | | 0 | 0,1 | M | Sel de sodium d'HEPES | 7,5 | 25 | %(w/v) | PEG 6000 |
| D04 | 0 | 0 | 0 | 0,1 | M | Sel de sodium d'HEPES | 7,5 | 25 | %(w/v) | PEG 8000 |
| D05 | 0 | | 0 | 0,1 | M | Sel de sodium d'HEPES | 7,5 | 20 | %(w/v) | PEG 10000 |
| D06 | 0 | | 0 | 0,1 | M | Sel de sodium d'HEPES | 7,5 | 15 | %(w/v) | PEG 20000 |
| D07 | 0 | | 0 | 0,1 | M | TRIS HCl | 8,5 | 25 | %(w/v) | PEG 3000 |
| D08 | 0 | | 0 | 0,1 | M | TRIS HCl | 8,5 | 25 | %(w/v) | PEG 4000 |
| D09 | 0 | | 0 | 0,1 | M | TRIS HCl | 8,5 | 25 | %(w/v) | PEG 6000 |
| Puits | C | U | Sel | C | U | Tampon | pH | C | U | Précipitant |
| D10 | 0 | | 0 | 0,1 | M | TRIS HCl | 8,5 | 25 | %(w/v) | PEG 8000 |
| D11 | 0 | | 0 | 0,1 | M | TRIS HCl | 8,5 | 20 | %(w/v) | PEG 10000 |
| D12 | 0 | | 0 | 0,1 | M | TRIS HCl | 8,5 | 15 | %(w/v) | PEG 20000 |
| E01 | 0,2 | M | fluorure de sodium | | | | | 20 | %(w/v) | PEG 3350 |
| E02 | 0,2 | M | fluorure de potassium | | | | | 20 | %(w/v) | PEG 3350 |
| E03 | 0,2 | M | fluorure d'ammonium | | | | | 20 | %(w/v) | PEG 3350 |
| E04 | 0,2 | M | chlorure de lithium | | | | | 20 | %(w/v) | PEG 3350 |
| E05 | 0,2 | M | chlorure de magnesium | | | | | 20 | %(w/v) | PEG 3350 |
| E06 | 0,2 | M | chlorure de sodium | | | | | 20 | %(w/v) | PEG 3350 |
| E07 | 0,2 | M | chlorure de calcium | | | | | 20 | %(w/v) | PEG 3350 |
| E08 | 0,2 | M | chlorure de potassium | | | | | 20 | %(w/v) | PEG 3350 |
| E09 | 0,2 | M | chlorure d'ammonium | | | | | 20 | %(w/v) | PEG 3350 |
| E10 | 0,2 | M | iodure de sodium | | | | | 20 | %(w/v) | PEG 3350 |
| E11 | 0,2 | M | Iodure de potassium | | | | | 20 | %(w/v) | PEG 3350 |
| E12 | 0,2 | M | iodure d'ammonium | | | | | 20 | %(w/v) | PEG 3350 |
| F01 | 0,2 | M | thiocyanate de sodium | | | | | 20 | %(w/v) | PEG 3350 |
| F02 | 0,2 | M | thiocyanate de potassium | | | | | 20 | %(w/v) | PEG 3350 |
| F03 | 0,2 | M | nitrate de lithium | | | | | 20 | %(w/v) | PEG 3350 |
| F04 | 0,2 | M | nitrate de magnésium | | | | | 20 | %(w/v) | PEG 3350 |
| F05 | 0,2 | M | nitrate de sodium | | | | | 20 | %(w/v) | PEG 3350 |
| F06 | 0,2 | M | nitrate de potassium | | | | | 20 | %(w/v) | PEG 3350 |
| F07 | 0,2 | M | nitrate d'ammonium | | | | | 20 | %(w/v) | PEG 3350 |
| F08 | 0,2 | M | formiate de magnésium | | | | | 20 | %(w/v) | PEG 3350 |
| F09 | 0,2 | M | formiate de sodium | | | | | 20 | %(w/v) | PEG 3350 |
| F10 | 0,2 | M | formiate de potassium | | | | | 20 | %(w/v) | PEG 3350 |
| F11 | 0,2 | M | formiate d'ammonium | | | | | 20 | %(w/v) | PEG 3350 |
| F12 | 0,2 | M | acétate de lithium | | | | | 20 | %(w/v) | PEG 3350 |
| G01 | 0,2 | M | maqnesium acetate | | | | | 20 | %(w/v) | PEG 3350 |
| G02 | 0,2 | M | zinc acetate | | | | | 20 | %(w/v) | PEG 3350 |
| G03 | 0,2 | M | acétate de sodium | | | | | 20 | %(w/v) | PEG 3350 |
| G04 | 0,2 | M | acétate de calcium | | | | | 20 | %(w/v) | PEG 3350 |
| G05 | 0,2 | M | acétate de potassium | | | | | 20 | %(w/v) | PEG 3350 |
| G06 | 0,2 | M | acétate d'ammonium | | | | | 20 | %(w/v) | PEG 3350 |
| G07 | 0,2 | M | Sulfate de lithium | | | | | 20 | %(w/v) | PEG 3350 |
| G08 | 0,2 | M | Sulfate de magnésium | | | | | 20 | %(w/v) | PEG 3350 |
| G09 | 0,2 | M | sulfate de sodium | | | | | 20 | %(w/v) | PEG 3350 |
| G10 | 0,2 | M | sulfate de potassium | | | | | 20 | %(w/v) | PEG 3350 |
| G11 | 0,2 | M | sulfate d'ammonium | | | | | 20 | %(w/v) | PEG 3350 |
| G12 | 0,2 | M | tartrate de di-sodium | | | | | 20 | %(w/v) | PEG 3350 |
| H01 | 0,2 | M | tartrate de potassium/sodium | | | | | 20 | %(w/v) | PEG 3350 |
| H02 | 0,2 | M | di-tartrate d'ammonium | | | | | 20 | %(w/v) | PEG 3350 |
| H03 | 0,2 | M | phosphate de sodium | | | | | 20 | %(w/v) | PEG 3350 |
| H04 | 0,2 | M | phosphate disodique | | | | | 20 | %(w/v) | PEG 3350 |
| H05 | 0,2 | M | phosphate de potassium | | | | | 20 | %(w/v) | PEG 3350 |
| H06 | 0,2 | M | phosphate di-potassium | | | | | 20 | %(w/v) | PEG 3350 |
| H07 | 0,2 | M | phosphate d'ammonium | | | | | 20 | %(w/v) | PEG 3350 |
| H08 | 0,2 | M | phosphate di-ammonium | | | | | 20 | %(w/v) | PEG 3350 |
| H09 | 0,2 | M | tri-citrate de lithium | | | | | 20 | %(w/v) | PEG 3350 |
| H10 | 0,2 | M | citrate trisodique | | | | | 20 | %(w/v) | PEG 3350 |
| H11 | 0,2 | M | citrate de tri-potassium | | | | | 20 | %(w/v) | PEG 3350 |
| H12 | 0,18 | M | citrate tri-ammonium | | | | | 20 | %(w/v) | PEG 3350 |

**ANNEXE 2B**

| Annexe plaque « Wizard I et II de Rigaku » | | | | | | | | | | | | | |
|---|---|---|---|---|---|---|---|---|---|---|---|---|---|
| Well | C | U | Sel | C | U | Tampon | pH | C | U | Précipitant | C | U | Additif |
| D10 | 0,2 | M | acétate de calcium | 0,1 | M | imidazole/HCl | 8 | 10 | %(w/v) | PEG-8000 | | | |
| D11 | 0,2 | M | sulfate de lithium | 0,1 | | Tris base/HCl | 8,5 | 1,26 | M | sulfate d'ammonium | | | |
| D12 | 0,2 | M | acétate de zinc | 0,1 | M | acétate de sodium / acide citrique | 4,5 | 20 | %(w/v) | PEG-1000 | | | |
| E01 | 0,2 | M | acétate de zinc | 0,1 | M | acétate de sodium / acide citrique | 4,5 | 10 | %(w/v) | PEG-3000 | | | |
| E02 | 0,2 | M | sulfate de lithium | 0,1 | M | MES/NaOH | 6 | 35 | %(v/v) | 2-methyl-2.4-pentanediol | | | |
| E03 | 0,2 | M | chlorure de magnesium | 0,1 | M | Tris base/HCl | 8,5 | 20 | %(w/v) | PEG-8000 | | | |
| E04 | 0,2 | M | chlorure de sodium | 0,1 | M | cacodylate de sodium/HCl | 6,5 | 2 | M | sulfate d'ammonium | | | |
| E05 | 0,2 | M | chlorure de sodium | 0,1 | M | HEPES/NaOH | 7,5 | 20 | %(v/v) | 1,4-bubnediol | | | |
| E06 | 0,2 | M | sulfate de lithium | 0,1 | M | phosphate disodique / acide citrique | 4,2 | 10 | %(v/v) | 2-propanol | | | |
| E07 | 0,2 | M | chlorure de sodium | 0,1 | M | Tris base/HCl | 7 | 30 | %(w/v) | PEG-3000 | | | |
| EG8 | 0,2 | M | chlorure de sodium | 0,1 | M | phosphate de potassium monobasique / dibasique de sodium | 6,2 | 10 | %(w/v) | PEG-8000 | | | |
| E09 | | | | 0,1 | M | phosphate disodique / acide citrique | 4,2 | 2 | M | sulfate d'ammonium | | | |
| E10 | | | | 0,1 | M | Tris base/HCl | 8,5 | 1 | M | phosphate d'ammonium dibasique | | | |
| E11 | 0,2 | M | acétate de zinc | 0,1 | M | cacodylate de sodium/HCl | 6,5 | 10 | %(v /v) | 2-propanol | | | |
| E12 | 0,2 | M | sulfate de lithium | 0,1 | M | cacodylate de sodium/HCl | 6,5 | 30 | %(v/v) | PEG-400 | | | |
| FOI | 0,2 | M | sulfate de lithium | 0,1 | M | citrate de sodium / acide citrique | 5,5 | 15 | %(v/v) | alcool réactif* | | | |
| F02 | 0,2 | M | chlorure de sodium | 0,1 | M | phosphate de potassium monobasique / dibasique de sodium | 6,2 | 20 | %(w/v) | PEG-1000 | | | |
| F03 | | | | 0,1 | M | HEPES/NaOH | 7,5 | 1,26 | M | sulfate d'ammonium | | | |
| F04 | | | | 0,1 | M | CHES/NaOH | 9,5 | 1 | M | citrate trisodique | | | |
| FOS | 0,2 | M | chlorure de magnesium | 0,1 | M | Tris base/HCl | 7 | 2,5 | M | chlorure de sodium | | | |
| F06 | 0,2 | M | acétate de calcium | 0,1 | M | Tris base/HCl | 7 | 20 | %(w/v) | PEG-3000 | | | |
| F07 | | | | 0,1 | M | phosphate disodique / acide citrique | 4,2 | 1,6 | M | phosphate de sodium monobasique | 0,4 | M | phosphate dibasique de potassium |
| F08 | 0,2 | M | acétate de zinc | 0,1 | M | MES/NaOH | 6 | 15 | %(v/v) | alcool réactif* | | | |
| F09 | | | | 0,1 | M | acétate de sodium / acide citrique | 4,5 | 35 | %(v/v) | 2-methyl-2,4-pentanediol | | | |
| F1O | | | | 0,1 | M | imidazole/HCl | 8 | 10 | %(v/v) | 2-propanol | | | |
| F11 | 0,2 | M | chlorure de magnesium | 0,1 | M | HEPES/NaOH | 7,5 | 15 | %(v/v) | alcool réactif* | | | |
| F12 | 0,2 | M | chlorure de sodium | 0,1 | M | imidazole/HCl | 8 | 30 | %(w/v) | PEG-8000 | | | |
| G01 | 0,2 | M | chlorure de sodium | 0,1 | M | HEPES/NaOH | 7,5 | 35 | %(v/v) | 2-methyl-2,4-pentanediol | | | |
| G02 | | | | 0,1 | M | CHES/NaOH | 9,5 | 30 | %(v/v) | PEG-400 | | | |
| G03 | 0,2 | M | chlorure de magnesium | 0,1 | M | cacodylate de sodium/HCl | 6,5 | 10 | %(w/v) | PEG-3000 | | | |
| G04 | 0,2 | M | acétate de calcium | 0,1 | M | MES/NaOH | 6 | 20 | %(w/v) | PEG-8000 | | | |
| G05 | 0,2 | M | chlorure de sodium | 0,1 | M | CHES/NaOH | 9,5 | 1,26 | M | sulfate d'ammonium | | | |
| G0s | 0,2 | M | acétate de zinc | 0,1 | M | imidazole/HCl | 8 | 20 | %(v/v) | 1.4-butanediol | | | |
| G07 | 0,2 | M | chlorure de sodium | 0,1 | M | Tris base/HCl | 7 | 1 | M | citrate trisodique | | | |
| G08 | | | | 0,1 | M | Tris base/HCl | 8,5 | 20 | %(w/v) | PEG-1000 | | | |
| G09 | 0,2 | M | chlorure de sodium | 0,1 | M | citrate de sodium / acide citrique | 5,5 | 1 | M | phosphate d'ammonium dibasique | | | |
| G10 | | | | 0,1 | M | imidazole/HCl | 8 | 10 | %(w/v) | PEG-8000 | | | |
| G11 | | | | 0,1 | M | acétate de sodium / acide citrique | 4,5 | 0,8 | M | phosphate de sodium monobasique | 1.2 M | | phosphate dibasique de potassium |
| G12 | 0,2 | M | chlorure de sodium | 0,1 | M | phosphate disodique / acide citrique | 4,2 | 10 | %(w/v) | PEG-3000 | | | |
| H01 | 0,2 | M | sulfate de lithium | 0,1 | M | Tris base/HCl | 7 | 1 | M | potassium / tartrate de sodium | | | |
| H02 | 0,2 | M | sulfate de lithium | 0,1 | M | acétate de sodium / acide citrique | 4,5 | 2,5 | M | chlorure de sodium | | | |
| H03 | 0,2 | M | chlorure de sodium | 0,1 | M | CAPS/NaOH | 10,5 | 20 | %(w/v) | PEG-8000 | | | |
| H04 | 0,2 | M | acétate de zinc | 0,1 | M | imidazole/HCl | 8 | 20 | %(w/v) | PEG-3000 | | | |
| H05 | 0,2 | M | sulfate de lithium | 0,1 | M | Tris base/HCl | 7 | 2 | M | sulfate d'ammonium | | | |
| H06 | 0,2 | M | chlorure de sodium | 0,1 | M | HEPES/NaOH | 7,5. | 30 | %(v/v) | PEG-400 | | | |
| H07 | 0,2 | M | chlorure de magnesium | 0,1 | M | Tris base/HCl | 7 | 10 | %(w/v) | PEG-8000 | | | |
| HO8 | 0,2 | M | chlorure de magnesium | 0,1 | M | cacodylate de sodium/HCl | 6,5 | 20 | %(w/v) . | PEG-1000 | | | |
| H09 | | | | 0,1 | M | MES/NaOH | 6 | 1,26 | M | sulfate d'ammonium | | | |
| H10 | 0,2 | M | chlorure de sodium | 0,1 | M | imidazole/HCl | 8 | 1 | M | phosphate d'ammonium dibasique | | | |
| H11 | 0,2 | M | acétate de zinc | 0,1 | M | imidazole/HCl | 8 | 2,5 | M | chlorure de sodium | | | |
| H12 | | | | 0,1 | M | MES/NaOH | 6 | 1 | M | potassium / tartrate de sodium | | | |
| Well | C | U | Sel | C | U | Tampon | pH | C | U | Précipitant | C | U | Additif |
| A01 | | | | 0,1 | M | CHES/NaOH | 9,5 | 20 | %(w/v) | PEG-8000 | | | |
| A02 | 0,2 | M | chlorure de sodium | 0,1 | M | HEPES/NaOH | 7,5 | 10 | %(v/v) | 2-propanol | | | |
| A03 | | | | 0,1 | M | CHES/NaOH | 9,5 | 15 | %(v/v) | alcool réactif* | | | |
| A04 | 0,2 | M | chlorure de magnesium | 0,1 | M | imidazole/HCl | 8 | 35 | %(v/v) | 2-methyl-2,4-pentanediol | | | |
| A05 | | | | 0,1 | M | CAPS/NaOH | 10,5 | 30 | %(v/v) | PEG-400 | | | |
| A06 | | | | 0,1 | M | citrate de sodium / acide citrique | 5,5 | 20 | %(w/v) | PEG-3000 | | | |
| A07 | 0,2 | M | acétate de zinc | 0,1 | M | MES/NaOH | 6 | 10 | %(w/v) | PEG-8000 | | | |
| A08 | | | | 0,1 | M | citrate de sodium / acide citrique | 5,5 | 2 | M | sulfate d'ammonium | | | |
| A09 | | | | 0,1 | M | acétate de sodium / acide citrique | 4,5 | 1 | M | phosphate d'ammonium dibasique | | | |
| A10 | | | | 0,1 | M | Tris base/HCl | 7 | 20 | %(w/v) | PEG-2000 MME | | | |
| A11 | 0,2 | M | sulfate de lithium | 0,1 | M | MES/NaOH | 6 | 20 | %(v/v) | 1,4-butanediol | | | |
| A12 | 0,2 | M | acétate de calcit/ffl | 0,1 | M | imidazole/HCl | B | 20 | %(w/v) | PEG-1000 | | | |
| B01 | | | | 0,1 | M | cacodylate de sodium/HCl | 6,5 | 1,26 | M | sulfate d'ammonium | | | |
| B02 | | | | 0,1 | M | cacodylate de sodium/HCl | 6,5 | 1 | M | citrate trisodique | | | |
| B03 | 0,2 | M | sulfate de lithium | 0,1 | M | imidazole/HCl | 8 | 10 | %(w/v) | PEG-3000 | | | |
| B04 | | | | 0,1 | M | phosphate de potassium monobasique/ dibasique de sodium | 6,2 | 2,5 | M | chlorure de sodium | | | |
| B05 | 0,2 | M | sulfate de lithium | 0,1 | M | acétate de sodium / acide citrique | 4,5 | 30 | %(w/v) | PEG-8000 | | | |
| B06 | 0,2 | M | chlorure de sodium | 0,1 | M | imidazole/HCl | 8 | 1 | M | potassium / tartrate de sodium | | | |
| B07 | | | | 0,1 | M | Tris base/HCl | 7 | 20 | %(w/v) | PEG-1000 | | | |
| B08 | 0,2. | | chlorure de sodium | 0,1 | M | imidazole/HCl | 8 | 0,4 | M | phosphate de sodium monobasique | 1.6 | M | phosphate dibasique de potassium |
| B09 | | | | 0,1 | M | HEPES/NaOH | 7,5 | 20 | %(w/v) | PEG-8000 | | | |
| B1c | | | | 0,1 | M | Tris base/HCl | 8,5 | 10 | %(v/v) | 2-propanol | | | |
| B11 | 0,2 | M | chlorure magnesium | 0,1 | M | imidazole/HCl | 8 | 15 | %(v/v) | alcool réactif* | | | |
| B12 | 0,21 | M | chlorure de sodium | 0,1 | M | Tris base/HCl | 7 | 35 | %(v/v) | 2-methyl-2,4-pentanediol | | | |
| C01 | 0,2 | M | chlorure de magnesium | 0,11 | M | Tris base/HCl | 8,5 | 30 | %(v/v) | PEG-400 | | | |
| C02 | | | | 0,11 | M | CHES/NaOH | 9,5 | 10 | %(w/v) | PEG-3000 | | | |
| C03 | 0,21 | M | sulfate de lithium | 0,1 | M | CAPS/NaOH | 10,5 | 1,2 | M | phosphate de sodium monobasique | 0.8 | M | phosphate dibasique de potassium |
| C04 | 0,2 | M | chlorure de sodium | 0,1 | M | HEPES/NaOH | 7,5 | 20 | %(w/v) | PEG-3000 | | | |
| C05 | 0,2 | M | chlorure de sodium | 0,1 | M | CHES/NaOH | 9,5 | 10 | %(w/v) | PEG-8000 | | | |
| C06 | 0,21 | M | chlorure de sodium | 0,1 | M | acétate de sodium / acide citrique | 4,5 | 1,26 | M | sulfate d'ammonium | | | |

| Annexe plaque « Wizard I et 11 de Rigaku » | | | | | | | | | | | | | |
|---|---|---|---|---|---|---|---|---|---|---|---|---|---|
| C07 | 0,2 | 2 M | chlorure de sodium | 0,1 | M | phosphate disodique / acide citrique | 4,2 | 20 | %(w/v) | PEG-8000 | | | |
| C08 | | | | 0,1 | M | phosphate de potassium monobasique/ dibasique de sodium | 6,2 | 10 | %(w/v) | PEG-3000 | | | |
| C09 | 0,2 | M | sulfate de lithium | 0,1 | M | CAPS/NaOH | 10,5 | 2 | M | sulfate d'ammonium | | | |
| C10 | | | | 0,1 | M | imidazole/HCl | 8 | 1 | M | phosphate d'ammonium dibasique | | | |
| C11 | | | | 0,1 | M | acétate de sodium / acide citrique | 4,5 | 20 | %(v/v) | 1,4-butanediol | | | |
| C12 | | | | 0,1 | M | imidazole/HCl | 8 | 1 | M | citrate trisodique | | | |
| D01 | | | | 0,1 | M | imidazole/HCl | 8 | 2,5 | M | chlorure de sodium | | | |
| D02 | 0,2 | M | sulfate de lithium | 0,1 | M | CHES/NaOH | 9,5 | 1 | M | potassium / tartrate de sodium | | | |
| D03 | 0,2 | M | sulfate de lithium | 0,1 | M | phosphate disodique / adde citrique | 4,2 | 20 | %(w/v) | PEG-1000 | | | |
| D04 | 0,2_{"} | M | acétate de calcium | 0,1 | M | MES/NaOH | 6 | 10 | %(v/v) | 2-propanol | | | |
| D05 | | | | 0,1 | M | CHES/NaOH | 9,5 | 30 | %(w/v) | PEG-3000 | | | |
| D06 | | | | 0,1 | M | Tris base/HCl | 7 | 15 | %(v/v) | alcool réactif* | | | |
| D07 | | | | 0,1 | M | phosphate de potassium monobasique / dibasique de sodium | 6,2 | 35 | %(v/v) | 2-methyl-2,4-pentanediol | | | |
| D08 | 0,2! | M | acétate de calcium | 0,1 | M | acétate de sodium / acide citrique | 4,5 | 30 | %(v/v) | PEG-400 | | | |
| D09 | | | | 0,1 | M | acétate de sodium / acide citrique | 4,5 | 20 | %(w/v) | PEG-3000 | | | |

**ANNEXE 2B**

| Annexe : Plaque « Salt grid Hampton » | | | | | | | | | | | |
|---|---|---|---|---|---|---|---|---|---|---|---|
| Puits | C | U | Tampon | pH | C | U | Précipitant | C | U | précipitant 2 | pH |
| A 01 | 0,1 | M | acide citrique | 4 | 0,8 | M | sulfate d'ammonium | | | | |
| A02 | 0,1 | M | acide citrique | 5 | 0,8 | M | sulfate d'ammonium | | | | |
| A03 | 0,1 | M | MES | 6 | 0,8 | M | sulfate d'ammonium | | | | |
| A04 | 0,1 | M | HEPES | 7 | 0,8 | M | sulfate d'ammonium | | | | |
| A05 | 0,1 | M | TRIS | 8 | 0,8 | M | sulfate d'ammonium | | | | |
| A06 | 0,1 | M | bicine | 9 | 0,8 | M | sulfate d'ammonium | | | | |
| B01 | 0,1 | M | acide citrique | 4 | 1,6 | M | sulfate d'ammonium | | | | |
| B02 | 0,1 | M | acide citrique | 5 | 1,6 | M | sulfate d'ammonium | | | | |
| B03 | 0,1 | M | MES | 6 | 1,6 | M | sulfate d'ammonium | | | | |
| B04 | 0,1 | M | HEPES | 7 | 1,6 | M | sulfate d'ammonium | | | | |
| B05 | 0,1 | M | TRIS | 8 | 1,6 | M | sulfate d'ammonium | | | | |
| B06 | 0,1 | M | bicine | 9 | 1,6 | M | sulfate d'ammonium | | | | |
| C01 | 0,1 | M | acide citrique | 4 | 2,4 | M | sulfate d'ammonium | | | | |
| C02 | 0,1 | M | acide citrique | 5 | 2,4 | M | sulfate d'ammonium | | | | |
| C03 | 0,1 | M | MES | 6 | 2,4 | M | sulfate d'ammonium | | | | |
| C04 | 0,1 | M | HEPES | 7 | 2,4 | M | sulfate d'ammonium | | | | |
| C05 | 0,1 | M | TRIS | 8 | 2,4 | M | sulfate d'ammonium | | | | |
| C06 | 0,1 | M | bicine | 9 | 2,4 | M | sulfate d'ammonium | | | | |
| D01 | 0,1 | M | acide citrique | 4 | 3 | M | sulfate d'ammonium | | | | |
| D02 | 0,1 | M | acide citrique | 5 | 3 | M | sulfate d'ammonium | | | | |
| D03 | 0,1 | M | MES | 6 | 3 | M | sulfate d'ammonium | | | | |
| D04 | 0,1 | M | HEPES | 7 | 3 | M | sulfate d'ammonium | | | | |
| D05 | 0,1 M | | TRIS | 8 | 3 | M | sulfate d'ammonium | | | | |
| D06 | 0,1 | M | bicine | 9 | 3 | M | sulfate d'ammonium | | | | |
| E01 | | | | 4 | 1 | M | malonate | | | | |
| E02 | | | | 4 | 1,5 | M | malonate | | | | |
| E03 | | | | 4 | 1,9 | M | malonate | | | | |
| E04 | | | | 4 | 2,4 | M | malonate | | | | |
| E05 | | | | 4 | 2,9 | M | malonate | | | | |
| E06 | | | | 4 | 3,4 | M | malonate | | | | |
| F01 | | | | 5 | 1 | M | malonate | | | | |
| F02 | | | | 5 | 1,5 | M | malonate | | | | |
| F03 | | | | 5 | 1,9 | M | malonate | | | | |
| F04 | | | | 5 | 2,4 | M | malonate | | | | |
| F05 | | | | 5 | 2,9 | M | malonate | | | | |
| F06 | | | | 5 | 3,4 | M | malonate | | | | |
| G01 | | | | 6 | 1 | M | malonate | | | | |
| G02 | | | | 6 | 1,5 | M | malonate | | | | |
| G03 | | | | 6 | 1,9 | M | malonate | | | | |
| G04 | | | | 6 | 6 2,4 | M | malonate | | | | |
| G05 | | | | 6 | 6 2,9 | M | malonate | | | | |
| G06 | | | | 6 | 3,4 | M | malonate | | | | |
| H01 | | | | 7 | 1 | M | malonate | | | | |
| H02 | | | | 7 | 1,5 | M | malonate | | | | |
| H03 | | | | 7 | 1,9 | M | malonate | | | | |
| H04 | | | | 7 | 2,4 | M | malonate | | | | |
| H05 | | | | 7 | 2,9 | M | malonate | | | | |
| H06 | | | | 7 | 3,4 | M | malonate | | | | |
| A07 | | | | | 0,78 | M | dihydrogénophosphate de sodium monohydraté | 0,016 | M | di-potassium hydrogénophosphate | 5 |
| A08 | | | | | 0,72 | M | dihydrogénophosphate de sodium monohydraté | 0,08 | M | di-potassium hydrogénophosphate | 5,6 |
| A09 | | | | | 0,52 | M | dihydrogénophosphate de sodium monohydraté | 0,28 | M | di-potassium hydrogénophosphate | 6,3 |
| A10 | | | | | 0,28 | M | dihydrogénophosphate de sodium monohydraté | 0,52 | M | di-potassium hydrogénophosphate | 6,9 |
| A11 | | | | | 0,12 | M | dihydrogénophosphate de sodium monohydraté | 0,68 | M | di-potassium hydrogénophosphate | 7,5 |
| A12 | | | | | 0,032 | M | dihydrogénophosphate de sodium monohydraté | 0,768 | M | di-potassium hydrogénophosphate | 8,2 |
| B07 | | | | | 0,98 | M | dihydrogénophosphate de sodium monohydraté | 0,02 | M | di-potassium hydrogénophosphate | 5 |
| B08 | | | | | 0,9 | M | dihydrogénophosphate de sodium monohydraté | 0,1 | M | di-potassium hydrogénophosphate | 5,6 |
| B09 | | | | | 0,65 | M | dihydrogénophosphate de sodium monohydraté | 0,35 | M | di-potassium hydrogénophosphate | 6,3 |
| B10 | | | | | 0,35 | M | dihydrogénophosphate de sodium monohydraté | 0,65 | M | di-potassium hydrogénophosphate | 6,9 |
| B11 | | | | | 0,15 | M | dihydrogénophosphate de sodium monohydraté | 0,85 | M | di-potassium hydrogénophosphate | 7,5 |
| B12 | | | | | 0,04 | M | dihydrogénophosphate de sodium monohydraté | 0,96 | M | di-potassium hydrogénophosphate | 8,2 |
| C07 | | | | | 1,372 | M | dihydrogénophosphate de sodium monohydraté | 0,028 | M | di-potassium hydrogénophosphate | 5 |
| C08 | | | | | 1,26 | M | dihydrogénophosphate de sodium monohydraté | 0,14 | M | di-potassium hydrogénophosphate | 5,6 |
| C09 | | | | | 0,91 | M | dihydrogénophosphate de sodium monohydraté | 0,49 | M | di-potassium hydrogénophosphate | 6,3 |
| C10 | | | | | 0,49 | M | dihydrogénophosphate de sodium monohydraté | 0,91 | M | di-potassium hydrogénophosphate | 6,9 |
| C11 | | | | | 0,21 | M | dihydrogénophosphate de sodium monohydraté | 1,19 | M | di-potassium hydrogénophosphate | 7,5 |
| C12 | | | | | 0,056 | M | dihydrogénophosphate de sodium monohydraté | 1,344 | M | di-potassium hydrogénophosphate | 8,2 |
| D07 | | | | | 1,764 | M | dihydrogénophosphate de sodium monohydraté | 0,036 | M | di-potassium hydrogénophosphate | 5 |
| D08 | | | | | 1,62 | M | dihydrogénophosphate de sodium monohydraté | 0,18 | M | di-potassium hydrogénophosphate | 5,6 |
| D09 | | | | | 1,17 | M | dihydrogénophosphate de sodium monohydraté | 0,63 | M | di-potassium hydrogénophosphate | 6,3 |
| D10 | | | | | 0,63 | M | dihydrogénophosphate de sodium monohydraté | 1,17 | M | di-potassium hydrogénophosphate | 6,9 |
| D11 | | | | | 0,27 | M | dihydrogénophosphate de sodium monohydraté | 1,53 | M | di-potassium hydrogénophosphate | 7,5 |
| D12 | | | | | 0,072 | M | dihydrogénophosphate de sodium monohydraté | 1,728 | M | di-potassium hydrogénophosphate | 8,2 |
| E07 | 0,1 | M | acide citrique | 4 | 0,8 | M | formiate de sodium pH4 | | | | |
| E08 | 0,1 | M | acide citrique | 5 | 0,8 | M | formiate de sodium pH5 | | | | |
| E09 | 0,1 | | MES | 6 | 0,8 | M | formiate de sodium pH6 | | | | |
| E10 | 0,1 | M | HEPES | 7 | 0,8 | M | formiate de sodium pH7 | | | | |
| E11 | 0,1 | M | TRIS | 8 | 0,8 | M | formiate de sodium pH8 | | | | |
| E12 | 0,1 | M | bicine | 9 | 0,8 | M | formiate de sodium pH9 | | | | |
| F07 | 0,1 | M | acide citrique | 4 | 1,6 | M | formiate de sodium pH4 | | | | |
| F08 | 0,1 | M | acide citrique | 5 | 1,6 | M | formiate de sodium pH5 | | | | |
| F09 | 0,1 | M | MES | 6 | 1,6 | M | formiate de sodium pH6 | | | | |
| F10 | 0,1 | M | HEPES | 7 | 1,6 | M | formiate de sodium pH7 | | | | |
| F11 | 0,1 | M | TRIS | 8 | 1,6 | M | formiate de sodium pH8 | | | | |
| F12 | 0,1 | M | bicine | 9 | 1,6 | M | formiate de sodium pH9 | | | | |
| G07 | 0,1 | M | acide citrique | 4 | 2,4 | M | formiate de sodium pH4 | | | | |
| G08 | 0,1 | M | acide citrique | 5 | 2,4 | M | formiate de sodium pH5 | | | | |
| G09 | 0,1 | M | MES | 6 | 2,4 | M | formiate de sodium pH6 | | | | |
| G10 | 0,1 | | HEPES | 7 | 2,4 | M | formiate de sodium pH7 | | | | |
| G11 | 0,1 | M | TRIS | 8 | 2,4 | M | formiate de sodium pH8 | | | | |
| G12 | 0,1 | M | bicine | 9 | 2,4 | M | formiate de sodium pH9 | | | | |
| H07 | 0,1 | M | acide citrique | 4 | 3,2 | M | formiate de sodium pH4 | | | | |
| H08 | 0,1 | M | acide citrique | 5 | 3,2 | M | formiate de sodium pH5 | | | | |
| H09 | 0,1 | M | MES | 6 | 3,2 | M | formiate de sodium pH6 | | | | |
| H10 | 0,1 | M | HEPES | 7 | 3,2 | M | formiate de sodium pH7 | | | | |
| H11 | 0,1 | M | TRIS | 8 | 3,2 | M | formiate de sodium pH8 | | | | |
| H12 | 0,1 | M | bicine | 9 | 3,2 | M | formiate de sodium pH9 | | | | |

## Revendications

1. Complexes cationiques formés d'un ion lanthanide Ln³⁺ et d'un ligand de formule : 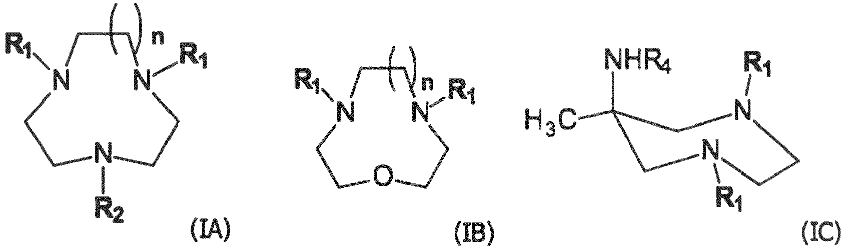 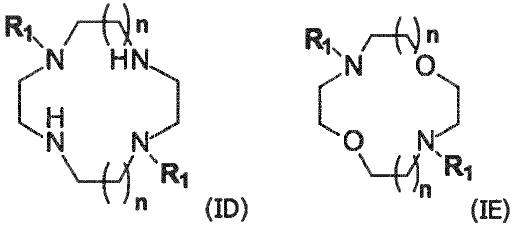 dans laquelle :
∘ **n** est égal à 1, 2 ou 3 ;
∘ **R₂** est un atome d'hydrogène ou un groupe méthyle ou -CH₂**R₅**, **R₅** représentant un groupe phényle, pyridinyle ou picolinyle ;
∘ **R₄** représente -H, -CH₃, -CH₂**R₆** ; **R₆** représentant un groupe phényle ou pyridinyle ;
- **R₁** représente : 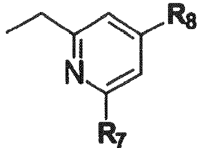 avec :
∘ **R₇** qui représente -COO⁻, -CONH₂, -CONH**R₉**, -P**R₉**OO⁻, ou un groupe choisi 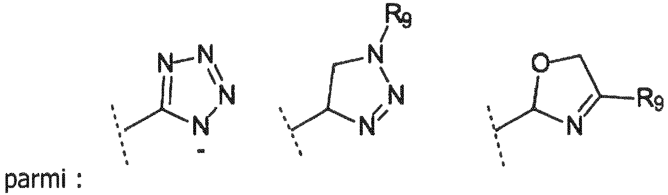 avec **R₉** qui représente un atome d'hydrogène, un groupe méthyle, éthyle ou phényle ; et
∘ **R₈** qui représente un atome d'hydrogène, de fluor, chlore, brome ou iode, un groupe -OH ou -NH₂ ; et leurs sels avec un anion, leurs solvats et hydrates ;
à l'exception des complexes cationiques formés d'un ion lanthanide Ln³⁺ et d'un ligand répondant à l'une des formule (I.1) ou (I.4) suivantes, et de leurs sels avec un anion, leurs solvats et hydrates :

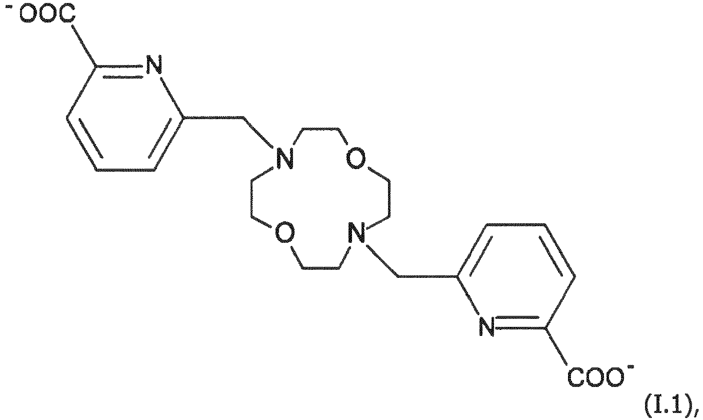

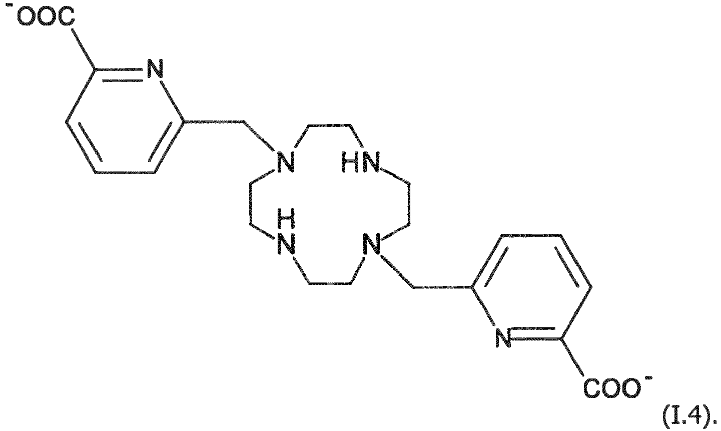

2. Complexes selon la revendication 1 sous la forme d'un sel avec un anion choisi parmi : Cl⁻, Br⁻, I⁻, OH⁻, NO₃⁻, triflate, PF₆⁻, SbF₆⁻, B(Ph)₄⁻, BF₄⁻, les sulfates, carbonates, phosphates et carboxylates.

3. Complexes selon la revendication 1 ou 2 formés avec un ligand répondant à la formule (IA) : 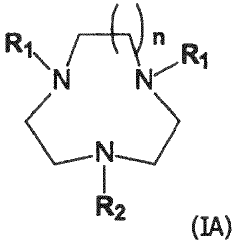 dans laquelle **n**=1 et **R₂**=H, et **R₁** est tel que défini à la revendication 1, ainsi que leurs sels avec un anion, leurs solvats et hydrates.

4. Complexes selon l'une des revendications 1 à 3 **caractérisé en ce que R₁** représente : 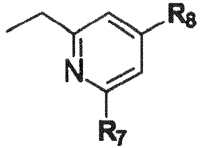 avec :
- **R₇** qui représente -COO⁻ ou -P**R₉**OO⁻ avec **R₉** qui représente un groupe méthyle ou éthyle ; ou bien **R₇** qui représente un groupe: 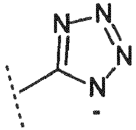
- **R₈** qui représente un atome d'hydrogène, de fluor, chlore, brome ou iode; ainsi que leurs sels avec un anion, leurs solvats et hydrates.

5. Complexes selon la revendication 1 choisis parmi les complexes de formule : 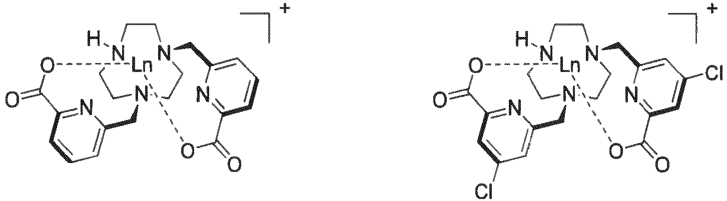 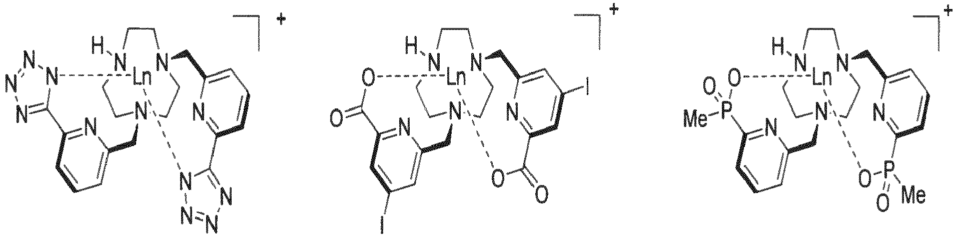 ainsi que leurs sels avec un anion, en particulier leur sel chlorhydrate, leurs solvats et hydrates.

6. Complexes selon l'une des revendications 1 à 5, avec un ion lanthanide Ln³⁺, Ln étant Sm, Eu, Gd, Tb, Dy, Ho, Er, Tm, Yb ou Lu, avec Eu, Tb, Yb et Lu qui sont préférés.

7. Utilisation d'un complexe tel que défini à l'une quelconque des revendications 1 à 6, ou d'un complexe cationique formé d'un ion lanthanide Ln³⁺ et d'un ligand de formule (I.1) ou (I.4) telle que définie à la revendication 1, ou d'un complexe cationique formé d'un ion lanthanide Ln³⁺ et d'un ligand de formule : 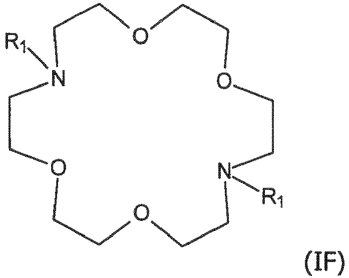 avec R₁ tel que défini à la revendication 1 ou à la revendication 4, ou d'un de leurs sels avec un anion, leurs solvats ou hydrates, en tant qu'aide à la cristallisation d'une macromolécule biologique choisie parmi les peptides et les protéines.

8. Utilisation selon la revendication 7 **caractérisée en ce que** le complexe est utilisé en tant qu'agent nucléant et/ou en tant qu'agent cristallisant lors de la cristallisation d'une macromolécule biologique choisie parmi les peptides et les protéines.

9. Utilisation selon la revendication 7 ou 8 **caractérisée en ce que**, dans le complexe, Ln =Eu ou Tb et le complexe est également utilisé en tant qu'agent luminescent pour la détection de cristaux.

10. Utilisation d'un complexe tel que défini à l'une quelconque des revendications 1 à 6, ou d'un complexe cationique formé d'un ion lanthanide Ln³⁺ et d'un ligand de formule (I.1) ou (I.4) telle que définie à la revendication 1, ou d'un complexe cationique formé d'un ion lanthanide Ln³⁺ et d'un ligand de formule : 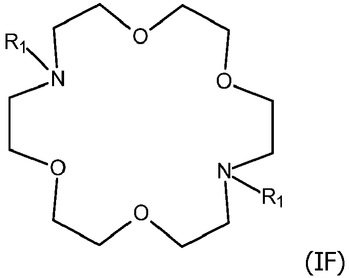 avec R₁ tel que défini à la revendication 1 ou à la revendication 4,
ou d'un de leurs sels avec un anion, leurs solvats ou hydrates, en tant qu'aide à l'obtention de données structurales d'une macromolécule biologique choisie parmi les peptides et les protéines.

11. Utilisation selon la revendication 10 **caractérisée en ce que** le complexe est utilisé en tant qu'agent phasant lors de la détermination structurale par diffraction des rayons X.

12. Utilisation selon la revendication 10 ou 11 **caractérisée en ce que**, dans le complexe, Ln =Eu ou Tb et le complexe est également utilisé en tant qu'aide au positionnement du cristal dans un faisceau de rayons X.

13. Cristal d'une macromolécule biologique choisie parmi les peptides et les protéines comprenant un complexe selon l'une des revendications 1 à 6 ou un complexe cationique formé d'un ion lanthanide Ln³⁺ et d'un ligand de formule (I.1) ou (I.4) telle que définie à la revendication 1, ou d'un complexe cationique formé d'un ion lanthanide Ln³⁺ et d'un ligand de formule : 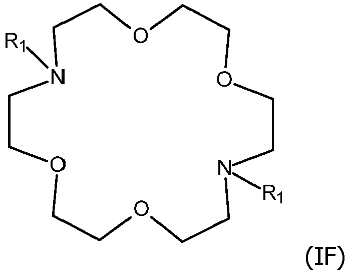 avec R₁ tel que défini à la revendication 1 ou à la revendication 4, ou un de ses sels avec un anion, solvat ou hydrate.

## Patentansprüche

1. Kationische Komplexe, die aus einem Lanthanidion Ln³⁺ und einem Liganden der Formel: 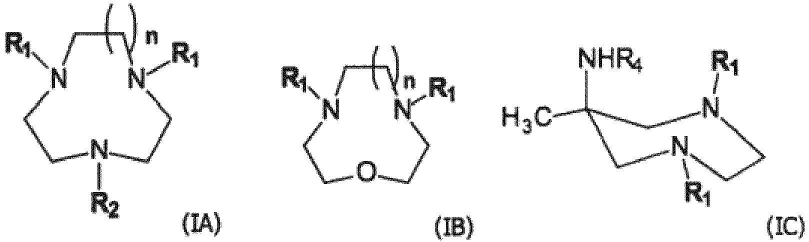 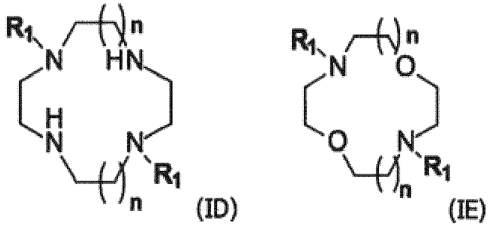 gebildet sind, wobei:
∘ n gleich 1, 2 oder 3 ist,
∘ R₂ ein Wasserstoffatom oder eine Methylgruppe oder -CH₂R₅ ist, wobei R₅ eine Phenyl-, Pyridinyl- oder Picolinylgruppe darstellt,
∘ R₄ -H, -CH₃, -CH₂R₆ darstellt, wobei R₆ eine Phenyl- oder Pyridinylgruppe darstellt,
- R₁: 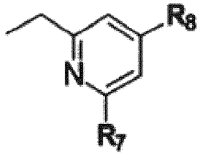 darstellt,
wobei:
∘ R₇ -COO⁻, -CONH₂, -CONHR₉, -PR₉OO⁻ oder eine Gruppe darstellt, die ausgewählt ist aus: 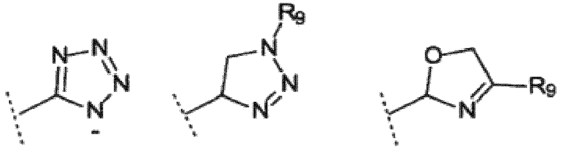 wobei R₉ ein Wasserstoffatom, eine Methyl-, Ethyl- oder Phenylgruppe darstellt, und
∘ R₈ ein Wasserstoff-, Fluor-, Chlor-, Brom- oder lodatom, eine OH- oder NH₂-Gruppe darstellt,
und ihre Salze mit einem Anion, ihren Solvaten und Hydraten,
mit der Ausnahme von kationischen Komplexen, die aus einem Lanthanidion Ln³⁺ und einem Liganden mit einer der folgenden Formeln (I.1) oder (I.4) gebildet sind, und ihren Salzen mit einem Anion, ihren Solvaten und Hydraten:

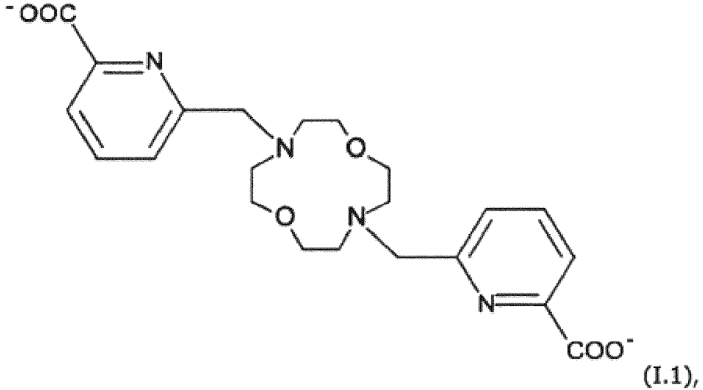

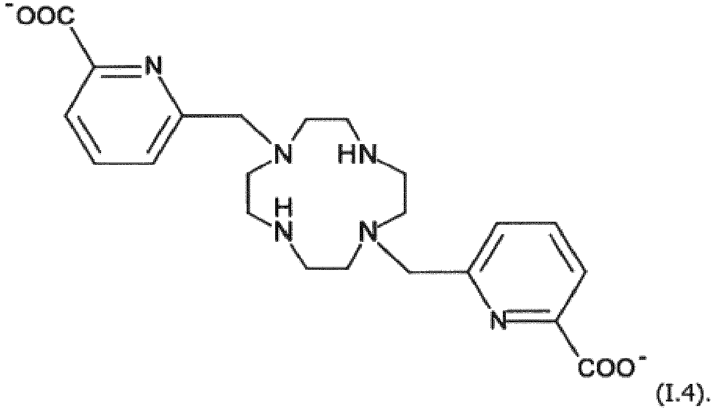

2. Komplexe nach Anspruch 1 in der Form eines Salzes mit einem Anion ausgewählt aus: Cl⁻, Br⁻, I⁻, OH⁻, NO₃⁻, Triflat, PF₆⁻, SbF₆⁻, B(Ph)₄⁻, BF₄⁻, den Sulfaten, Carbonaten, Phosphaten und Carboxylaten.

3. Komplexe nach Anspruch 1 oder 2, die mit einem Liganden mit der Formel (IA) gebildet sind: 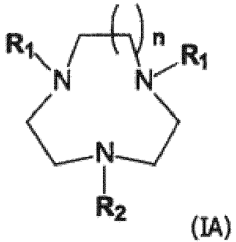 wobei n=1 und R₂=H, und R₁ wie in Anspruch 1 definiert ist,
sowie ihre Salze mit einem Anion, ihren Solvaten und Hydraten.

4. Komplexe nach einem der Ansprüche 1 bis 3, **dadurch gekennzeichnet, dass** R₁: 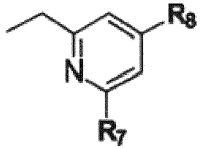 darstellt,
wobei:
- R₇ -COO⁻ oder -PR₉OO⁻ darstellt, wobei Rg eine Methyl- oder Ethylgruppe darstellt, oder R₇ eine Gruppe darstellt: 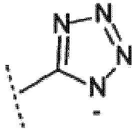
- R₈ ein Wasserstoff-, Fluor-, Chlor-, Brom- oder lodatom darstellt, sowie ihre Salze mit einem Anion, ihren Solvaten und Hydraten.

5. Komplexe nach Anspruch 1, ausgewählt aus den Komplexen der Formel: 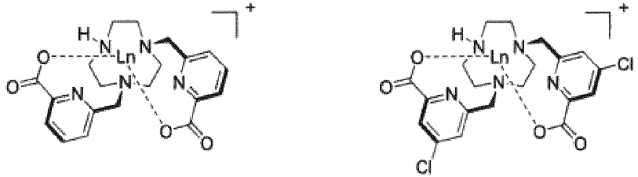 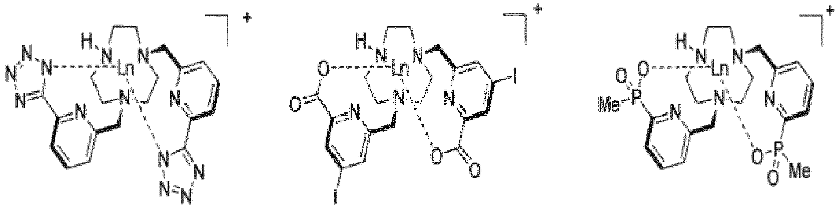 sowie ihren Salzen mit einem Anion, insbesondere ihrem Chlorhydratsalz, ihren Solvaten und Hydraten.

6. Komplexe nach einem der Ansprüche 1 bis 5, mit einem Lanthanidion Ln³⁺, wobei Ln Sm, Eu, Gd, Tb, Dy, Ho, Er, Tm, Yb oder Lu ist, wobei Eu, Tb, Yb und Lu bevorzugt sind.

7. Verwendung eines Komplexes wie in einem der Ansprüche 1 bis 6 definiert, oder eines kationischen Komplexes, der aus einem Lanthanidion Ln³⁺ und einem Liganden der Formel (I.1) oder (I.4), wie in Anspruch 1 definiert, gebildet ist, oder eines kationischen Komplexes, der aus einem Lanthanidion Ln³⁺ und einem Liganden der Formel: 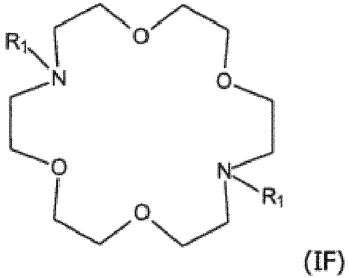 gebildet ist,
wobei R₁ wie in Anspruch 1 oder Anspruch 4 definiert ist,
oder einem ihrer Salze mit einem Anion, ihren Solvaten oder Hydraten, als Hilfe bei der Kristallisierung eines biologischen Makromoleküls, das aus Peptiden und Proteinen ausgewählt ist.

8. Verwendung nach Anspruch 7, **dadurch gekennzeichnet, dass** der Komplex als Nukleierungsmittel und/oder als Kristallisierungsmittel bei der Kristallisierung eines biologischen Makromoleküls verwendet wird, das aus Peptiden und Proteinen ausgewählt ist.

9. Verwendung nach Anspruch 7 oder 8, **dadurch gekennzeichnet, dass** in dem Komplex Ln=Eu oder Tb und der Komplex auch als lumineszierendes Mittel für die Detektion von Kristallen verwendet wird.

10. Verwendung eines Komplexes wie in einem der Ansprüche 1 bis 6 definiert, oder eines kationischen Komplexes, der aus einem Lanthanidion Ln³⁺ und einem Liganden der Formel (I.1) oder (I.4), wie in Anspruch 1 definiert, gebildet ist, oder eines kationischen Komplexes, der aus einem Lanthanidion Ln³⁺ und einem Liganden der Formel: 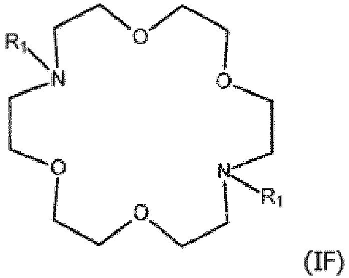 gebildet ist,
wobei R₁ wie in Anspruch 1 oder Anspruch 4 definiert ist,
oder einem ihrer Salze mit einem Anion, ihren Solvaten oder Hydraten, als Hilfe beim Erhalt von strukturellen Daten eines biologischen Makromoleküls, das aus Peptiden und Proteinen ausgewählt ist.

11. Verwendung nach Anspruch 10, **dadurch gekennzeichnet, dass** der Komplex als Phasenmittel bei der strukturellen Bestimmung durch Röntgendiffraktion verwendet wird.

12. Verwendung nach Anspruch 10 oder 11, **dadurch gekennzeichnet, dass** in dem Komplex Ln=Eu oder Tb und der Komplex auch als Positionierungshilfe des Kristalls in einem Röntgenstrahl verwendet wird.

13. Kristall eines biologischen Makromoleküls, das aus Peptiden und Proteinen ausgewählt ist und einen Komplex nach einem der Ansprüche 1 bis 6 oder einen kationischen Komplex, der aus einem Lanthanidion Ln³⁺ und einem Liganden der Formel (I.1) oder (I.4), wie in Anspruch 1 definiert, gebildet ist, oder einen kationischen Komplex umfasst, der aus einem Lanthanidion Ln³⁺ und einem Liganden der Formel: 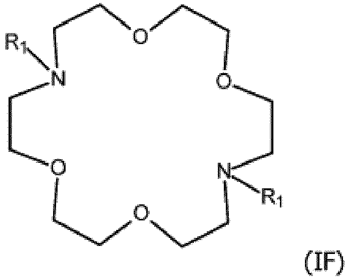 gebildet ist, wobei R₁ wie in Anspruch 1 oder in Anspruch 4 definiert ist, oder einem seiner Salze mit einem Anion, einem Solvat oder Hydrat.

## Claims

1. Cationic complexes formed of a lanthanide ion Ln³⁺ and a ligand of formula : 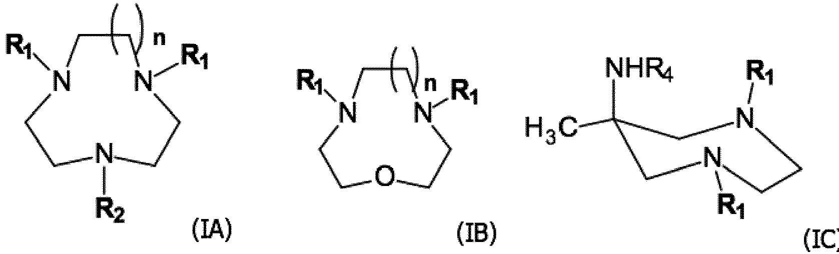 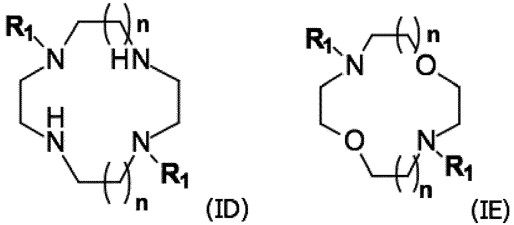 in which:
∘**n** is equal to 1, 2 or 3;
∘**R₂** is a hydrogen atom or a methyl group or -CH₂**R₅**, where **R₅** is a phenyl,
pyridinyl or picolinyl group;
∘**R₄** represents -H, -CH₃, -CH₂**R₆** ; **R₆** represents a phenyl or pyridinyl group;
- **R₁** represents : 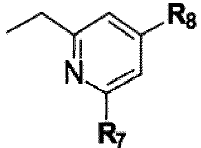 with :
∘**R₇** which represents -COO⁻, -CONH₂, -CONH**R₉**, -P**R₉**OO⁻, or a group selected n among : 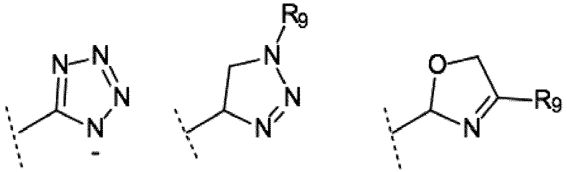 with **R₉** which represents a hydrogen atom, a methyl, ethyl or phenyl group; and
∘**R₈** which represents a hydrogen, fluorine, chlorine, bromine or iodine atom, a group -OH or -NH₂ ;
and their salts with an anion, their solvates and hydrates;
with the exception of cationic complexes consisting of a lanthanide ion Ln³⁺ and a ligand having one of the following formula (I.1) or (I.4) and salts thereof with anion, solvates and hydrates thereof: :

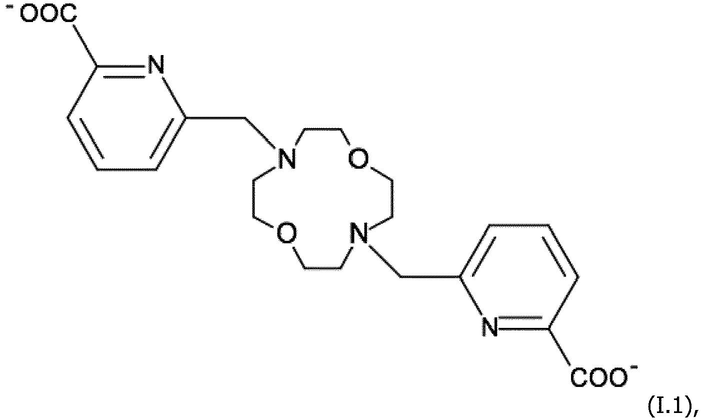

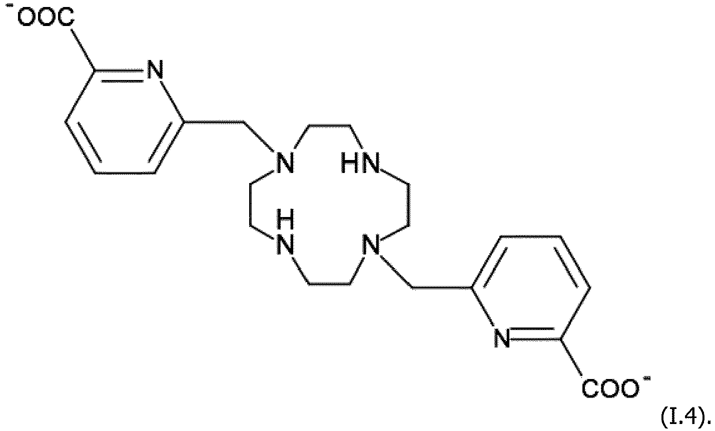

2. Complexes according to claim 1 in the form of a salt with an anion chosen from: Cl⁻, Br⁻, I⁻, OH⁻, NO₃⁻, triflate, PF₆⁻, SbF₆⁻, B(Ph)₄⁻, BF₄⁻, sulphates, carbonates, phosphates and carboxylates.

3. Complexes according to claim 1 or 2 formed with a ligand corresponding to the formula (IA) : 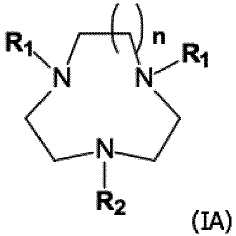 wherein **n**=1 and **R₂**=H, and **R₁** is as defined in claim 1, as well as their salts with an anion, their solvates and hydrates.

4. Complexes according to any of claims 1 to 3 **characterized in that R₁** represents: 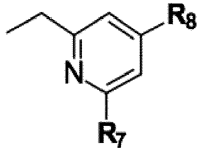 with:
- **R₇** which represents -COO⁻ or -P**R₉**OO⁻ with **R₉** which represents a group methyl or ethyl; or **R₇** which represents a group: 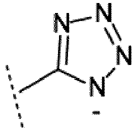
- **R₈** which represents a hydrogen, fluorine, chlorine, bromine or iodine atom ; as well as their salts with an anion, their solvates and hydrates.

5. Complexes according to claim 1, chosen from among complexes of formula: 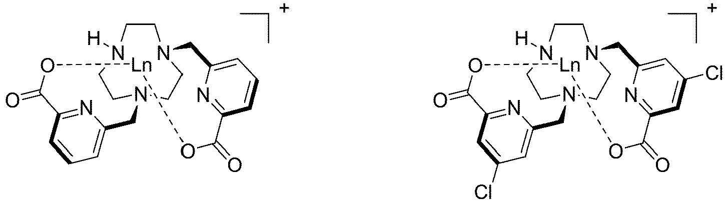 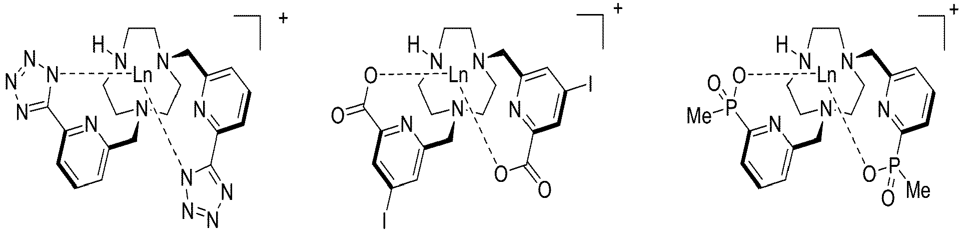 as well as their salts with an anion, in particular their hydrochloride salt, their solvates and hydrates.

6. Complexes according to any of claims 1 to 5, a with a lanthanide ion Ln³⁺, Ln being Sm, Eu, Gd, Tb, Dy, Ho, Er, Tm, Yb or Lu, with Eu, Tb, Yb and Lu being prefered.

7. Use of a complex as defined in anyone of claims 1 to 6, or of a cationic complex formed of a lanthanide ion Ln³⁺ and a ligand of formula (I.1) or (1.4) as defined in claim 1, or a cationic complex formed of a lanthanide ion Ln³⁺ and a ligand of formula: 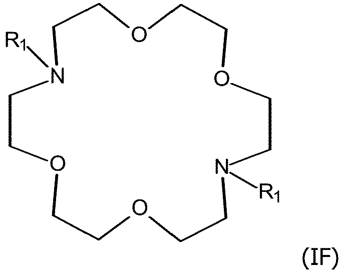 with R₁ as defined in claim 1 or claim 4,
or a salt thereof with an anion, their solvates or hydrates, as an aid to the crystallization of a biological macromolecule chosen among peptides and proteins.

8. Use according to claim 7 **characterized in that** the complex is used as a nucleating agent and / or as a crystallizing agent during the crystallization of a biological macromolecule chosen among peptides and proteins.

9. Use according to claim 7 or 8 **characterized in that**, in the complex, Ln =Eu or Tb and the complex is also used as a luminescent agent for the detection of crystals.

10. Use of a complex according to any of claims 1 to 6, or of a cationic complex formed of a lanthanide ion Ln³⁺ and a ligand of formula (I.1) or (I.4) as defined in claim 1, or a cationic complex formed of a lanthanide ion Ln³⁺ and a ligand of formula: 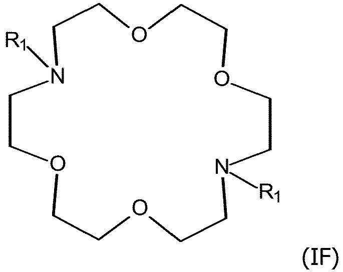 with R₁ as defined in claim 1 or claim 4,
or a salt thereof with an anion, their solvates or hydrates, as an aid in obtaining structural data of a biological macromolecule chosen among peptides and proteins.

11. Use according to claim 10 **characterized in that** the complex is used as a phasing agent during the structural determination by X-ray diffraction.

12. Use according to claim 10 or 11 **characterized in that**, in the complex, Ln = Eu or Tb and the complex is also used as a positioning aid for the crystal in an X-ray beam.

13. Crystal of a biological macromolecule chosen among peptides and proteins comprising a complex according to any of claims 1 to 6 or a cationic complex formed of a lanthanide ion Ln³⁺ and a ligand of formula (I.1) or (I.4) as defined in claim 1, or a cationic complex formed of a lanthanide ion Ln³⁺ and a ligand of formula: 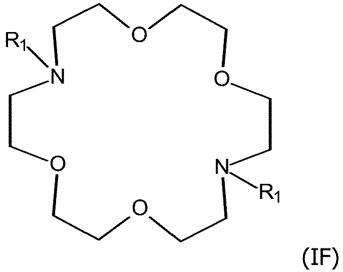 with R₁ as defined in claim 1 or claim 4,
or a salt thereof with an anion, solvate or hydrate.
